# EUROPEAN PATENT APPLICATION

(11) **EP 2 283 833 A2**
(43) Date of publication of application: **16.02.2011**
(21) Application number: 10011094.9
(22) Date of filing: 25.02.2005
(51) Int. Cl.: A61K 31/135, A61K 31/137, A61K 31/165, A61K 31/40, A61P 29/00, A61P 19/02, A61P 37/00, A61P 25/00, A61P 1/00, A61P 1/16, A61P 25/28, A61P 3/00, A61P 11/00, A61K 31/44

(54) **Amines and amides for the treatment of diseases**

(30) Priority: 25.02.2004 US 547997 P; 06.05.2004 US 569017 P; 13.08.2004 US 601221 P; 15.10.2004 US 619159 P
(62) Divisional of application: 05723671.3
(71) Applicant: LA JOLLA PHARMACEUTICAL CO., San Diego, CA 92121 (US)
(72) Inventor: Salter-Cid, Luisa Maria, San Diego CA 92122 (US); Wang, Eric Y., San Diego CA 92130 (US); MacDonald, Mary T., San Diego CA 92109 (US); Zhao, Jingjing, San Diego CA 92122 (US)
(74) Representative: Sexton, Jane Helen

(57) **Abstract**

Compositions and methods are disclosed for inhibiting semicarbazide-sensitive amine oxidase (SSAO), also known as vascular adhesion protein-1 (VAP-1). The compounds disclosed are amine-containing and amide-containing compounds. The compounds and compositions are useful for treatment of diseases, including inflammation, inflammatory diseases and autoimmune disorders.

## Description

### CROSS REFERENCE TO RELATED APPLICATIONS

This application claims the priority benefit of United States Provisional Patent Application No. 60/547,997, filed February 25, 2004, of United States Provisional Patent Application No. 60/569,017, filed May 6, 2004, of United States Provisional Patent Application No. 60/601,221, filed August 13, 2004, and of United States Provisional Patent Application No. 60/619,159, filed October 15, 2004. The entire contents of those applications are hereby incorporated by reference herein.

### TECHNICAL FIELD

This application relates to compositions and methods for inhibiting semicarbazide-sensitive amine oxidase (SSAO), also known as vascular adhesion protein-1 (VAP-1), for treatment of inflammation, inflammatory diseases and autoimmune disorders.

### BACKGROUND

Human vascular adhesion protein-1 (VAP-1) is a type 2,180 kD homodimeric endothelial cell adhesion molecule. Cloning and sequencing of VAP-1 revealed that the VAP-1 cDNA sequence is identical to that of the previously known protein semicarbazide-sensitive amine oxidase (SSAO), a copper-containing amine oxidase. The precise difference (if any) between the membrane-bound VAP-1 adhesion protein and the soluble SSAO enzyme has not yet been determined; one hypothesis indicates that proteolytic cleavage of the membrane-bound VAP-1 molecule results in the soluble SSAO enzyme. Both the membrane-bound VAP-1 protein and the soluble SSAO enzyme have amine oxidase enzymatic activity. Thus membrane-bound VAP-1 can function both as an amine oxidase and a cell adhesion molecule.

Semicarbazide-sensitive amine oxidase is a member of a group of enzymes; that group is referred to generically as semicarbazide-sensitive amine oxidases (SSAOs). SSAOs are mostly soluble enzymes that catalyze oxidative deamination of primary amines. The reaction results in the formation of the corresponding aldehyde and release of H₂O₂ and ammonium. These enzymes are different from monoamine oxidases A and B (MAO-A and MAO-B, respectively), in terms of their substrates, inhibitors, cofactors, subcellular localization and function. To date, no physiological function has been definitively associated with SSAOs, and even the nature of the physiological substrates is not firmly established (reviewed in Buffoni F. and Ignesti G. (2000) Mol. Genetics Metabl. 71:559-564). However, they have been implicated in the metabolism of exogenous and endogenous amines and in the regulation of glucose transport.

SSAO molecules are highly conserved across species; the closest homologue to the human protein is the bovine serum amine oxidase (about 85% identity). Substrate specificity and tissue distribution vary considerably among different species. In humans, SSAO specific activity has been detected in most tissues but with marked differences (highest in aorta and lung). Human and rodent plasma have very low SSAO activity compared with ruminants. Depletion studies suggest that SSAO/VAP-1 accounts for ∼90% of cell and serum SSAO activity (Jaakkola K. et al.(1999) Am. J. Pathol. 155:1953).

Membrane-bound VAP-1 is primarily expressed in high endothelial cells (ECs) of lymphatic organs, sinusoidal ECs of the liver and small caliber venules of many other tissues. Moreover, SSAO/VAP-1 is also found in dendritic cells of germinal centers and is abundantly present in adipocytes, pericytes and smooth muscle cells. However, it is absent from capillaries, ECs of large blood vessels, epithelial cells, fibroblasts and leukocytes (Salmi M. et al. (2001) Trends Immunol. 22:211). Studies in clinical samples revealed that SSAO/VAP-1 is upregulated on vasculature at many sites of inflammation, such as synovitis, allergic and other skin inflammations, and inflammatory bowel disease (IBD). However, expression appears to be controlled by additional mechanisms. Animal studies indicate that the luminal SSAO/VAP-1 is induced only upon elicitation of inflammation. Thus, in ECs, SSAO/VAP-1 is stored in intracellular granules and is translocated onto the luminal surface only at sites of inflammation.

In the serum of healthy adults a soluble form of SSAO/VAP-1 is found at a concentration of 80 ng/ml. Soluble SSAONAP-1 levels increase in certain liver diseases and in diabetes, but remain normal in many other inflammatory conditions. Soluble SSAO/VAP-1 has an N-terminal amino acid sequence identical to the proximal extracellular sequence of the membrane bound form of SSAO/VAP-1. In addition, there is good evidence that at least a significant portion of the soluble molecule is produced in the liver by proteolytic cleavage of sinusoidal VAP-1 (Kurkijarvi R. et al. (2000) Gastroenterology 119:1096).

SSAO/VAP-1 regulates leukocyte-subtype-specific adhesion to ECs. Studies show that SSAO/VAP-1 is involved in the adhesion cascade at sites where induction/activation of selectins, chemokines, immunoglobulin superfamily molecules, and integrins takes place. In the appropriate context, nevertheless, inactivation of SSAO/VAP-1 function has an independent and significant effect on the overall extravasion process. A recent study shows that both the direct adhesive and enzymatic functions of SSAO/VAP-1 are involved in the adhesion cascade (Salmi M. et al. (2001) Immunity 14:265). In this study, it was proposed that the SSAO activity of VAP-1 is directly involved in the pathway of leukocyte adhesion to endothelial cells by a novel mechanism involving direct interaction with an amine substrate presented on a VAP-1 ligand expressed on the surface of a leukocyte. Under physiological laminar shear, it seems that SSAO/VAP-1 first comes into play after tethering (which takes place via binding of selectins to their ligands) when lymphocytes start to roll on ECs. Accordingly, anti-VAP-1 monoclonal antibodies inhibit ∼50% of lymphocyte rolling and significantly reduce the number of firmly bound cells. In addition, inhibition of VAP-1 enzymatic activity by SSAO inhibitors, also results in a > 40% reduction in the number of rolling and firmly bound lymphocytes. Thus, inhibitors of SSAO/VAP-1 enzymatic activity could reduce leukocyte adhesion in areas of inflammation and thereby reduce leukocyte trafficking into the inflamed region and, consequently, reduce the inflammatory process itself.

Increased SSAO activity has been found in the plasma and islets of Type I and Type II diabetes patients and animal models, as well as after congestive heart failure, and in an artherosclerosis mouse model (Salmi M, et al. (2002) Am. J. Pathol. 161:2255; Bono P. et al (1999) Am. J. Pathol. 155:1613; Boomsma F. et al (1999) Diabetologia 42:233; Gronvall-Nordquist J. et al (2001) J. Diabetes Complications 15:250; Ferre I. et al. (2002) Neurosci. Lett. 15; 321: 21; Conklin D. J. et al. (1998) Toxicological Sciences 46: 386; Yu P.H. and Deng Y.L. (1998) Atherosclerosis 140:357; Vidrio H. et al. (2002) General Pharmacology 35:195; Conklin D.J. (1999) Toxicology 138: 137). In addition to upregulation of expression of VAP-1 in the inflamed joints of rheumatoid arthritis (RA) patients and in the venules from lamina propria and Peyer's patches of IBD patients, increased synthesis of VAP-1 was also found in chronic skin inflammation and liver disease (Lalor P.F. et al. (2002) J. Immunol. 169:983; Jaakkola K. et al. (2000) Am. J. Pathol. 157:463; Salmi M. and Jalkanen S. (2001) J. Immunol. 166:4650; Lalr P.F. et al. (2002) Immunol Cell Biol 80:52; Salmi M et al. (1997) J. Cin. Invest. 99:2165; Kurkijarvi R. et al. (1998) J. Immunol. 1611549).

In summary, SSAO/VAP-1 is an inducible endothelial enzyme that regulates leukocyte-subtype-specific adhesion and mediates the interaction between lymphocytes and inflamed vessels. The fact that SSAO/VAP-1 has both enzymatic and adhesion activities together with the strong correlation between its upregulation in many inflammatory conditions, makes it a potential therapeutic target for all the above-mentioned disease conditions.

### DISCLOSURE OF THE INVENTION

SSAO inhibitors can block inflammation and autoimmune processes, as well as other pathological conditions associated with an increased level of the circulating amine substrates and/or products of SSAO. In one embodiment, the invention relates to a method of inhibiting an inflammatory response by administration of compounds to inhibit SSAO enzyme activity (where the enzyme activity is due either to soluble SSAO enzyme or membrane-bound VAP-1 protein, or due to both) and/or inhibit binding to VAP-1 protein. In another embodiment, the inflammatory response is an acute inflammatory response. In another embodiment, the invention relates to treating diseases mediated at least in part by SSAO or VAP-1, as generally indicated by one or more of abnormal levels of SSAO and/or VAP-1 or abnormal activity of SSAO and/or VAP-1 (where the abnormal activity of VAP-1 may affect its binding function, its amine oxidase function, or both), by administering a therapeutically effective amount of an SSAO inhibitor, or administering a therapeutically effective combination of SSAO inhibitors. In another embodiment, the invention relates to a method of treating immune disorders, by administering a therapeutically effective amount of an SSAO inhibitor, or administering a therapeutically effective combination of SSAO inhibitors. In another embodiment, the invention relates to a method of treating multiple sclerosis (including chronic multiple sclerosis), by administering a therapeutically effective amount of an SSAO inhibitor, or administering a therapeutically effective combination of SSAO inhibitors. In another embodiment, the invention relates to a method of treating ischemic diseases (for example, stroke) and/or the sequelae thereof (for example, an inflammatory response), by administering a therapeutically effective amount of an SSAO inhibitor, or administering a therapeutically effective combination of SSAO inhibitors. The SSAO inhibitors administered can inhibit the SSAO activity of soluble SSAO, the SSAO activity of membrane-bound VAP-1, binding to membrane-bound VAP-1, or any two of those activities, or all three of those activities. In another embodiment, the invention relates to a method of inhibiting SSAO activity or inhibiting binding to VAP-1 *in vitro* using the compounds provided herein. In another embodiment, the invention relates to a method of inhibiting SSAO activity or inhibiting binding to VAP-1 *in vivo,* that is, in a living organism, such as a vertebrate, mammal, or human, using the compounds provided herein.

In another embodiment, the present invention relates to various compounds which are useful for inhibiting SSAO enzyme activity (where the enzyme activity is due either to soluble SSAO enzyme or membrane-bound VAP-1 protein, or due to both) and/or inhibition of binding to membrane-bound VAP-1 protein. In another embodiment, the present invention relates to methods of using various compounds to inhibit SSAO enzyme activity (where the enzyme activity is due either to soluble SSAO enzyme or membrane-bound VAP-1 protein, or due to both) and/or inhibit binding to VAP-1 protein. In another embodiment, the present invention relates to methods of using irreversible inhibitors of SSAO enzyme activity (where the enzyme activity is due either to soluble SSAO enzyme or membrane-bound VAP-1 protein, or due to both) and/or irreversible inhibitors of binding to VAP-1 protein.

In another embodiment, the present invention relates to methods of treating inflammation, by administering an SSAO inhibitor which has a specificity for inhibition of SSAO as compared to MAO-A and/or MAO-B of about 10, about 100, or about 500, or a specificity for inhibition of SSAO as compared to MAO-A and/or MAO-B of greater than about 10, greater than about 100, or greater than about 500.

In another embodiment, the present invention relates to methods of treating an immune or autoimmune disorder, by administering an SSAO inhibitor which has a specificity for inhibition of SSAO as compared to MAO-A and/or MAO-B of about 10, about 100, or about 500, or a specificity for inhibition of SSAO as compared to MAO-A and/or MAO-B of greater than about 10, greater than about 100, or greater than about 500.

In another embodiment, the present invention relates to methods of treating inflammation, by administering one or more of the compounds described herein in formulas I, II, III, IV, V, VI, VII, VIII, IX, and/or X in a therapeutically effective amount, or in an amount sufficient to treat inflammation. In another embodiment, the present invention relates to methods of treating immune disorders, by administering one or more of the compounds described herein in formulas I, II, III, IV, V, VI, VII, VIII, IX, and/or X in a therapeutically effective amount, or in an amount sufficient to treat an immune disorder.

In one embodiment, the present invention relates to compounds of formula I: 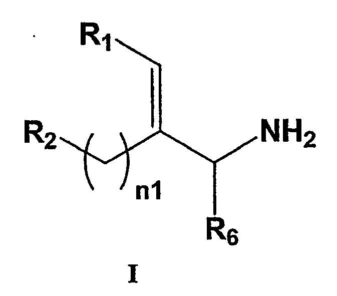 wherein:
R₁ is independently chosen from H, C₁-C₄ alkyl, Cl, F, or CF₃;
n1 is independently chosen from 0, 1, 2, and 3
R₂ is independently chosen from the moieties of formulas Ia, Ib, Ic, and Id: 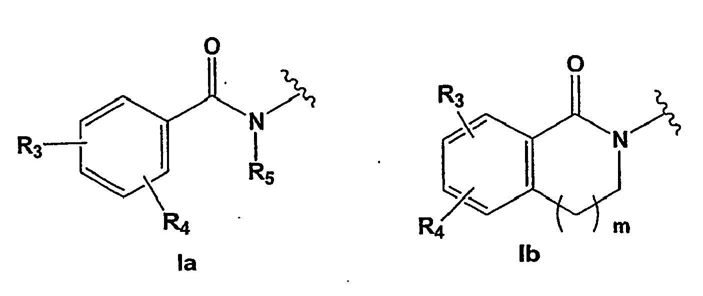 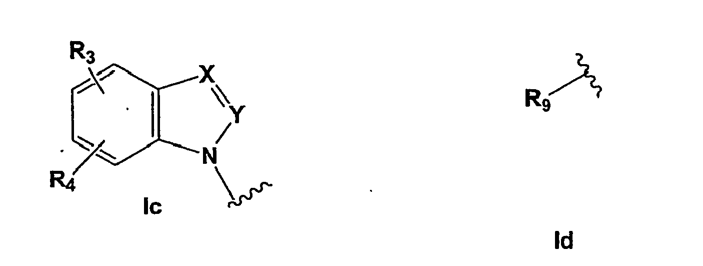 wherein:
   R₃ and R₄ are independently chosen from the group consisting ofH, C₁-C₄ alkyl, C₃-C₈ cycloalkyl, -O-C₁-C₄ alkyl, Cl, F, -OH, and -CF₃;
   R₅ is independently chosen from H, C₁-C₄ alkyl, C₃-C₈ cycloalkyl, C₆-C₁₄ aralkyl;
   R₆ is H when R₂ is chosen from Ia, Ib, and Ic, and R₆ is independently chosen from H, F, C₁-C₄ alkyl, C₆-C₁₀ aryl, C₆-C₁₆ substituted aryl, C₆-C₁₄ aralkyl, C₄-C₉ heteroaryl, and C₅-C₁₄ substituted heteroaryl when R₂ is Id;
   m is independently chosen from 0 and 1;
   R₉ is independently chosen from unsubstituted aryl, substituted aryl, monosubstituted aryl, disubstituted aryl,unsubstituted phenyl, substituted phenyl, monosubstituted phenyl, disubstituted phenyl, unsubstituted heteroaryl, substituted heteroaryl, monosubstituted heteroaryl, and disubstituted heteroaryl;
      and
   X and Y are independently chosen from N and CH;
      including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula I are also embraced by the invention. In one embodiment, R₆ is not H. In another embodiment, the formula is subject to the proviso that when n1 = 0 and R₂ is Id, then R₆ is not H.

The subset of compounds of formula I, where n = 0, R₂ is R₉, and R₉ is independently unsubstituted phenyl, monosubstituted phenyl, or disubstituted phenyl, is shown below in formula I-f: 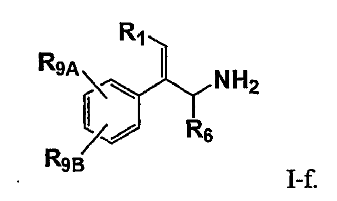 R_{9A} and R_{9B} are independently either hydrogen or are selected from -C₁-C₄ alkyl, halogen, -CF₃, -OH, or -O- C₁-C₄ alkyl; R₆ is independently chosen from F, C₁-C₄ alkyl, C₆-C₁₀ aryl, C₆-C₁₆ substituted aryl, C₆-C₁₄ aralkyl, C₄-C₉ heteroaryl, and
C₅-C₁₄ substituted heteroaryl; and R₁ is independently H, Cl, F or -CF₃; including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula I-f are also embraced by the invention.

In another embodiment, R_{9A} and R_{9B} are independently either hydrogen or are selected from -C₁-C₄ alkyl, F, Cl, -CF₃, -OH, or -O- C₁-C₄ alkyl. In another embodiment of the compounds of formula I-f, R₆ is -C₁-C₄ alkyl or halogen. In another embodiment of the compounds of formula I-f, R₆ is -C₁-C₄ alkyl or F.

In another embodiment, the present invention relates to methods of using the compounds of formula I to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the *in vitro* environment in an amount sufficient to inhibit SSAO activity or inhibit binding to VAP-1. The compounds can also be used for a method of inhibiting SSAO activity or inhibiting binding to VAP-1 *in vivo,* that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula I to treat inflammation or immune disorders. In another embodiment, the present invention relates to methods of using the compounds of formula I to suppress or reduce inflammation, or to suppress or reduce an inflammatory response. In another embodiment, the present invention relates to methods of treating inflammation, by administering one or more of the compounds described in formula I in a therapeutically effective amount, or in an amount sufficient to treat inflammation. In another embodiment, the present invention relates to methods of treating immune or autoimmune disorders, by administering one or more of the compounds described in formula I in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

In another embodiment, the present invention relates to compounds of general formula II: 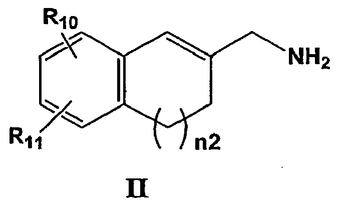 wherein:
R₁₀ and R₁₁ are independently chosen from the group consisting of H, C₁-C₄ alkyl, C₃-C₈ cycloalkyl, -O-C₁-C₄ alkyl, Cl, F, -OH, and -CF₃;
n2 is independently chosen from 0, 1, 2;
   including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula II are also embraced by the invention.

In another embodiment, the present invention relates to methods of using the compounds of formula II to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the in *vitro* environment in an amount sufficient to inhibit SSAO activity or inhibit binding to VAP-1. The compounds can also be used for a method of inhibiting SSAO activity or inhibiting binding to VAP-1 *in vivo,* that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula II to treat inflammation or immune disorders. In another embodiment, the present invention relates to methods of using the compounds of formula II to suppress or reduce inflammation, or to suppress or reduce an inflammatory response. In another embodiment, the present invention relates to methods of treating inflammation, by administering one or more of the compounds described in formula II in a therapeutically effective amount, or in an amount sufficient to treat inflammation. In another embodiment, the present invention relates to methods of treating immune or autoimmune disorders, by administering one or more of the compounds described in formula II in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

In another embodiment, the present invention relates to compounds of general formula III: 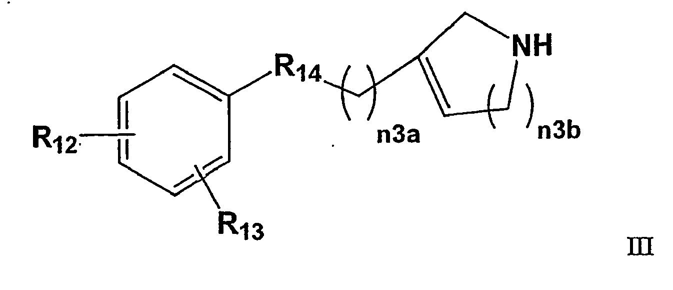 wherein:
R₁₂ and R₁₃ are independently chosen from the group consisting of H, C₁-C₄ alkyl,
C₃-C₈ cycloalkyl, -O-C₁-C₄ alkyl, Cl, F, -OH, and -CF₃;
R₁₄ is independently chosen from O, S, CH₂;
n3a and n3b are independently chosen from 1 or 2;
   including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula III are also embraced by the invention. In one embodiment, R₁₄ is independently CH₂. In another embodiment, R₁₄ is independently O. In another embodiment, R₁₂ is independently H. In another embodiment, R₁₂ is independently F. In another embodiment, R₁₂ is independently -O-CH₃. In another embodiment, R₁₃ is independently H. In another embodiment, R₁₃ is independently F. In another embodiment, R₁₃ is independently -O-CH₃. In another embodiment, n3a is independently 1. In another embodiment, n3a is independently 2. In another embodiment, n3b is independently 1. In another embodiment, n3b is independently 2.

In another embodiment, the present invention relates to methods of using the compounds of formula III to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the *in vitro* environment in an amount sufficient to inhibit SSAO activity or inhibit binding to VAP-1. The compounds can also be used for a method of inhibiting SSAO activity or inhibiting binding to VAP-1 *in vivo*, that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula III to treat inflammation or immune disorders. In another embodiment, the present invention relates to methods of using the compounds of formula III to suppress or reduce inflammation, or to suppress or reduce an inflammatory response. In another embodiment, the present invention relates to methods of treating inflammation, by administering one or more of the compounds described in formula III in a therapeutically effective amount, or in an amount sufficient to treat inflammation. In another embodiment, the present invention relates to methods of treating immune or autoimmune disorders, by administering one or more of the compounds described in formula III in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

In another embodiment, the invention includes compounds of formula IV: 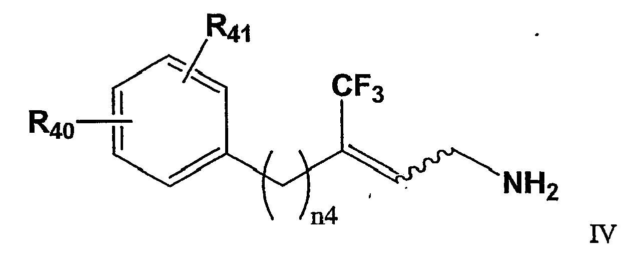 where R₄₀ and R₄₁ are independently chosen from the group consisting of H, C₁-C₄ alkyl, C₃-C₈ cycloalkyl, -O-C₁-C₄ alkyl, Cl, F, -OH, and -CF₃; and
n4 is independently 0, 1, or 2;
including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula IV are also embraced by the invention.

In another embodiment, the present invention relates to methods of using the compounds of formula IV to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the *in vitro* environment in an amount sufficient to inhibit SSAO activity or inhibit binding to VAP-1. The compounds can also be used for a method of inhibiting SSAO activity or inhibiting binding to VAP-1 *in vivo,* that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula IV to treat inflammation or immune disorders. In another embodiment, the present invention relates to methods of using the compounds of formula IV to suppress or reduce inflammation, or to suppress or reduce an inflammatory response. In another embodiment, the present invention relates to methods of treating inflammation, by administering one or more of the compounds described in formula IV in a therapeutically effective amount, or in an amount sufficient to treat inflammation. In another embodiment, the present invention relates to methods of treating immune or autoimmune disorders, by administering one or more of the compounds described in formula IV in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

In another embodiment, the invention includes compounds of formula V: 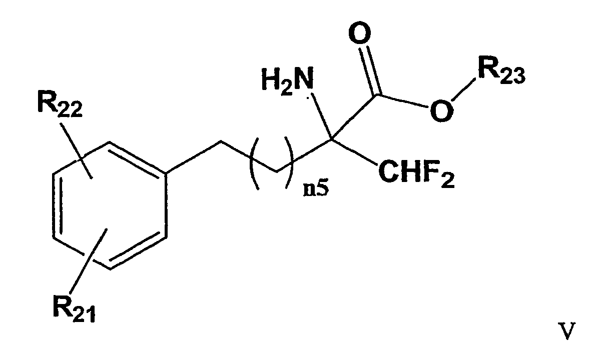 where R₂₁ and R₂₂ are independently chosen from the group consisting of H, C₁-C₄ alkyl, C₃-C₈ cycloalkyl, -O-C₁-C₄ alkyl, Cl, F, -OH, and -CF₃;
n5 is independently 0, 1, or 2;
and R₂₃ is independently H or C₁-C₈ alkyl;
including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula V are also embraced by the invention.

In another embodiment, the present invention relates to methods of using the compounds of formula V to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the *in vitro* environment in an amount sufficient to inhibit SSAO activity or inhibit binding to VAP-1. The compounds can also be used for a method of inhibiting SSAO activity or inhibiting binding to VAP-1 *in vivo,* that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula V to treat inflammation or immune disorders. In another embodiment, the present invention relates to methods of using the compounds of formula V to suppress or reduce inflammation, or to suppress or reduce an inflammatory response. In another embodiment, the present invention relates to methods of treating inflammation, by administering one or more of the compounds described in formula V in a therapeutically effective amount, or in an amount sufficient to treat inflammation. In another embodiment, the present invention relates to methods of treating immune or autoimmune disorders, by administering one or more of the compounds described in formula V in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

In another embodiment, the invention includes compounds of formula VI: 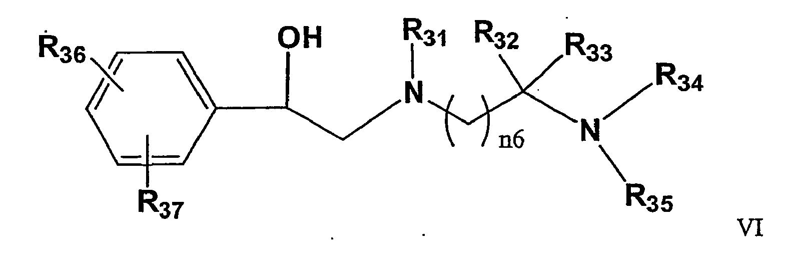 where R₃₆ and R₃₇ are independently chosen from the group consisting of H, C₁-C₄ alkyl, C₃-C₈ cycloalkyl, -O-C₁-C₄ alkyl, Cl, F, -OH, and -CF₃;
n6 is independently 0, 1, 2, or 3;
and R₃₁, R₃₂, R₃₃, R₃₄, and R₃₅ are independently chosen from the group consisting of H, C₁-C₄ alkyl, C₃-C₈ cycloalkyl, and C₆-C₁₄ aralkyl;
including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula VI are also embraced by the invention.

In another embodiment, the present invention relates to methods of using the compounds of formula VI to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the in *vitro* environment in an amount sufficient to inhibit SSAO activity or inhibit binding to VAP-1. The compounds can also be used for a method of inhibiting SSAO activity or inhibiting binding to VAP-1 *in vivo,* that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula VI to treat inflammation or immune disorders. In another embodiment, the present invention relates to methods of using the compounds of formula VI to suppress or reduce inflammation, or to suppress or reduce an inflammatory response. In another embodiment, the present invention relates to methods of treating inflammation, by administering one or more of the compounds described in formula VI in a therapeutically effective amount, or in an amount sufficient to treat inflammation. In another embodiment, the present invention relates to methods of treating immune or autoimmune disorders, by administering one or more of the compounds described in formula VI in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

In another embodiment, the invention includes compounds of formula VII 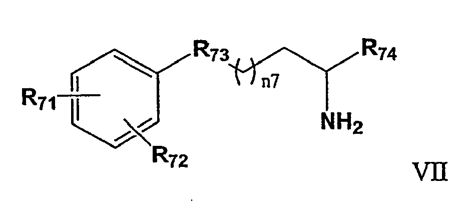 wherein:
R₇₁ and R₇₂ are independently chosen from the group consisting of H, C₁-C₄ alkyl, C₃-C₈ cycloalkyl, -O-C₁-C₄ alkyl, Cl, F, -OH, and -CF₃;
R₇₃ is independently chosen from O, S, CH₂, CHOH;
n7 is independently chosen from 1, 2, and 3;
R₇₄ is independently chosen from the moieties of formulas VIIa, VIIb, VIIc, and VIId 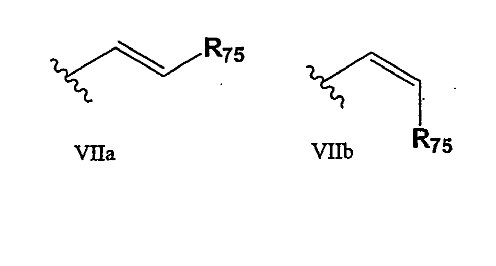 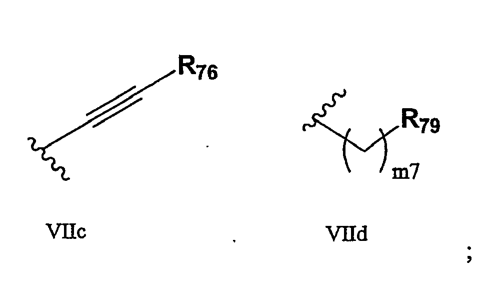 wherein:
   R₇₅ is independently chosen from H, C₁-C₄ alkyl, C₇-C₉ aralkyl, Cl, F, and -CF₃;
   R₇₆ is independently chosen from H, C₁-C₄ alkyl;
   m7 is independently chosen from 0, 1, and 2; and
   R₇₉ is independently chosen from unsubstituted aryl, substituted aryl, monosubstituted aryl, disubstituted aryl,unsubstituted phenyl, substituted phenyl, monosubstituted phenyl, disubstituted phenyl, unsubstituted heteroaryl, substituted heteroaryl, monosubstituted heteroaryl, and disubstituted heteroaryl;
      including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula VII are also embraced by the invention.

In one embodiment, R₇₄ is VIId. In one such embodiment, when R₇₉ is substituted, the substituents are independently chosen from the group consisting of H, F, Cl, -OH, -CF₃, C₁-C₄ alkyl, C₃-C₈ cycloalkyl, and -O-C₁-C₄ alkoxy. In another such embodiment, R₇₉ is independently unsubstituted phenyl, substituted phenyl, monosubstituted phenyl, or disubstituted phenyl. In another such embodiment, R₇₉ is unsubstituted phenyl, substituted phenyl, monosubstituted phenyl, or disubstituted phenyl, and the substituents on R₇₉ are independently chosen from the group consisting of H, F, Cl, -OH, -CF₃, C₁-C₄ alkyl, C₃-C₈ cycloalkyl, and -O-C₁-C₄ alkoxy.

In another embodiment, the present invention relates to methods of using the compounds of formula VII to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the *in vitro* environment in an amount sufficient to inhibit SSAO activity or inhibit binding to VAP-1. The compounds can also be used for a method of inhibiting SSAO activity or inhibiting binding to VAP-1 *in vivo,* that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula VII to treat inflammation or immune disorders. In another embodiment, the present invention relates to methods of using the compounds of formula VII to suppress or reduce inflammation, or to suppress or reduce an inflammatory response. In another embodiment, the present invention relates to methods of treating inflammation, by administering one or more of the compounds described in formula VII in a therapeutically effective amount, or in an amount sufficient to treat inflammation. In another embodiment, the present invention relates to methods of treating immune or autoimmune disorders, by administering one or more of the compounds described in formula VII in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

In another embodiment, the invention includes compounds of formula VIII: 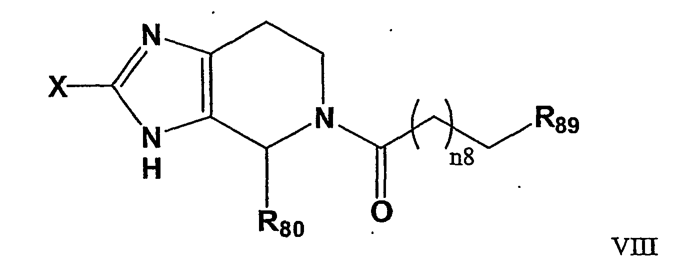 wherein R₈₀ is independently chosen from the group consisting of H, C₁-C₄ alkyl, C₃-C₈ cycloalkyl, C₆-C₁₀ aryl, C₆-C₁₄ aralkyl, C₄-C₉ heteroaryl, C₆-C₁₆ substituted aryl, and C₅-C₁₄ substituted heteroaryl;
X is independently chosen from the group consisting of H, NH₂, F, Cl, C₁-C₄ alkyl, C₃-C₈ cycloalkyl, C₆-C₁₀ aryl, C₆-C₁₄ aralkyl, C₄-C₉ heteroaryl, C₆-C₁₆ substituted aryl, and C₅-C₁₄ substituted heteroaryl;
R₈₉ is independently chosen from unsubstituted aryl, substituted aryl, monosubstituted aryl, disubstituted aryl, unsubstituted phenyl, substituted phenyl, monosubstituted phenyl, disubstituted phenyl, unsubstituted heteroaryl, substituted heteroaryl, monosubstituted heteroaryl, and disubstituted heteroaryl;
and
n8 is independently chosen from 0, 1, 2, and 3;
including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula VIII are also embraced by the invention.

In one embodiment, when R₈₉ is substituted, the substituents are independently chosen from the group consisting ofH, F, Cl, -OH, -CF₃, C₁-C₄ alkyl, C₃-C₈ cycloalkyl, and -O-C₁-C₄ alkoxy. In another embodiment, R₈₉ is independently unsubstituted phenyl, substituted phenyl, monosubstituted phenyl, or disubstituted phenyl. In another embodiment, R₈₉ is unsubstituted phenyl, substituted phenyl, monosubstituted phenyl, or disubstituted phenyl, and the substituents on R₈₉ are independently chosen from the group consisting of H, F, Cl, -OH, -CF₃, C₁-C₄ alkyl, C₃-C₈ cycloalkyl, and -O-C₁-C₄ alkoxy.

In another embodiment, the present invention relates to methods of using the compounds of formula VIII to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the *in vitro* environment in an amount sufficient to inhibit SSAO activity or inhibit binding to VAP-1. The compounds can also be used for a method of inhibiting SSAO activity or inhibiting binding to VAP-1 *in vivo,* that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula VIII to treat inflammation or immune disorders. In another embodiment, the present invention relates to methods of using the compounds of formula VIII to suppress or reduce inflammation, or to suppress or reduce an inflammatory response. In another embodiment, the present invention relates to methods of treating inflammation, by administering one or more of the compounds described in formula VIII in a therapeutically effective amount, or in an amount sufficient to treat inflammation. In another embodiment, the present invention relates to methods of treating immune or autoimmune disorders, by administering one or more of the compounds described in formula VIII in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

In another embodiment, the invention includes compounds of formula IX: 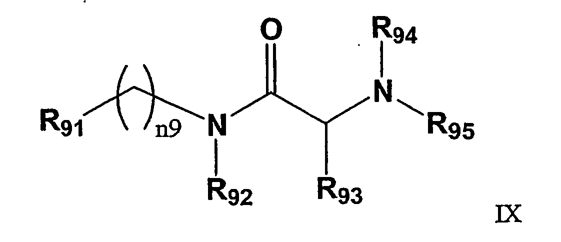 wherein
R₉₁ is independently chosen from C₆-C₁₀ unsubstituted aryl, C₆-C₁₇ substituted aryl, C₆-C₁₇ monosubstituted aryl, C₆-C₁₇ disubstituted aryl, C₆-C₁₇ trisubstituted aryl, C₆-C₁₄ aralkyl, C₄-C₉ unsubstituted heteroaryl, and C₄-C₁₅ substituted heteroaryl;
R₉₂ is independently chosen from H, C₁-C₄ alkyl, C₃-C₈ cycloalkyl, C₇-C₁₄ aralkyl;
R₉₃ is independently chosen from H, F, C₁-C₄ alkyl, C₃-C₈ cycloakyl, C₇-C₁₄ aralkyl, C₆-C₁₀ unsubstituted aryl, C₆-C₁₇ substituted aryl;
R₉₄ and R₉₅ are independently chosen from H, C₁-C₄ alkyl, C₃-C₈ cycloalkyl, and C₇-C₁₄ aralkyl; and
n9 is independently chosen from 1 and 2;
including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula IX are also embraced by the invention. In one embodiment, R₉₁ is independently C₆-C₁₀ unsubstituted aryl. In another embodiment, R₉₁ is independently C₆-C₁₇ substituted aryl. In another embodiment, R₉₁ is independently C₆ unsubstituted aryl (i.e., unsubstituted phenyl). In another embodiment, R₉₁ is independently C₆ substituted aryl (i.e., substituted phenyl). In another embodiment, R₉₁ is independently C₄-C₉ unsubstituted heteroaryl. In another embodiment, R₉₁ is independently unsubstituted pyridyl. In another embodiment, R₉₁ is independently unsubstituted pyridyl, attached to the remainder of the molecule at the 3-position (i.e., 3-pyridyl). When R₉₁ is substituted, preferred substituents are -F, -Cl, -CF₃, -OH, -C₁-C₄ alkyl, and -O-C₁-C₄ alkyl, more preferably -F, -Cl, and -CF₃, most preferably -F. In another embodiment, R₉₂ is independently chosen from H and C₁-C₄ alkyl. In another embodiment, R₉₂ is independently H. In another embodiment, R₉₃ is independently chosen from H and C₁-C₄ alkyl. In another embodiment, R₉₃ is independently H. In another embodiment, R₉₄ is independently chosen from H and C₁-C₄ alkyl. In another embodiment, R₉₄ is independently H. In another embodiment, R₉₅ is independently chosen from H and C₁-C₄ alkyl. In another embodiment, R₉₅ is independently H. In another embodiment, n9 is 1.

In another embodiment, the present invention relates to methods of using the compounds of formula IX to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the in *vitro* environment in an amount sufficient to inhibit SSAO activity or inhibit binding to VAP-1. The compounds can also be used for a method of inhibiting SSAO activity or inhibiting binding to VAP-1 *in vivo,* that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula IX to treat inflammation or immune disorders. In another embodiment, the present invention relates to methods of using the compounds of formula IX to suppress or reduce inflammation, or to suppress or reduce an inflammatory response. In another embodiment, the present invention relates to methods of treating inflammation, by administering one or more of the compounds described in formula IX in a therapeutically effective amount, or in an amount sufficient to treat inflammation. In another embodiment, the present invention relates to methods of treating immune or autoimmune disorders, by administering one or more of the compounds described in formula IX in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

In another embodiment, the compounds of formula IX are chosen from the subset designated IX-a: 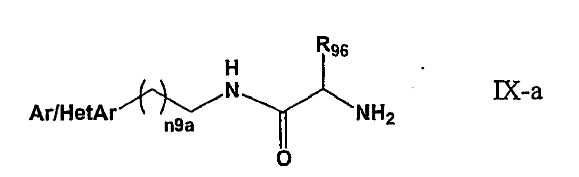 where Ar/HetAr is independently selected from substituted aryl, unsubstituted aryl, substituted hetereoaryl, and unsubstituted heteroaryl; n9a is independently 0 or 1 (note that n9a = n9 - 1); and R₉₆ is independently selected from H, F, and C₁-C₈ alkyl; including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula IX-a are also embraced by the invention.

In another embodiment, the invention includes compounds of formula IX-b: 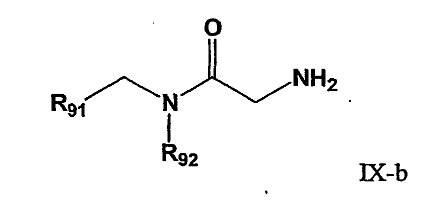 corresponding to formula IX where R₉₃, R₉₄, and R₉₅ are H, n9 is 1, and wherein:
R₉₁ is independently chosen from the group consisting of unsubstituted aryl, substituted aryl, monosubstituted aryl, disubstituted aryl, trisubstituted aryl, unsubstituted heteroaryl, substituted heteroaryl; and
R₉₂ is independently chosen from the group of consisting of H, -C₁-C₄ alkyl, C₃-C₈ cycloalkyl, and C₆-C₁₄ aralkyl; or
R₉₁ and R₉₂ together with the atoms to which they are bonded form a tetrahydropyridine, tetrahydropyrrole ring (pyrrolidine) or 2,5-dihydropyrrole ring (3-pyrroline), optionally fused to an aryl or hetereoaryl ring; including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula IX-b are also embraced by the invention.

In one embodiment of the compounds of formula IX-b, the substituents are independently chosen from H, -OH, -CF₃, halogen, -C₁-C₄ alkyl, and -O-C₁-C₄ alkyl. In another embodiment of the compounds of formula IX-b, the substituents are independently chosen from H, -OH, -CF₃, F, Cl, -C₁-C₄ alkyl, and -O-C₁-C₄ alkyl. In another embodiment, R₉₁ and R₉₂ together with the atoms to which they are bonded form the group 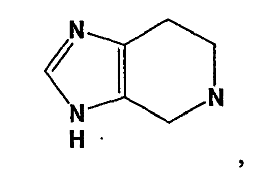 which is attached to the remainder of the molecule at the tetrahydropyridyl nitrogen, to form the compound

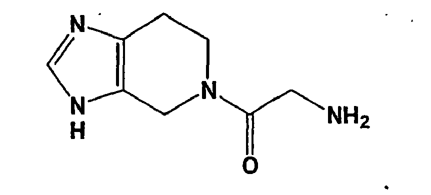

In another embodiment, the invention includes compounds of formula X: 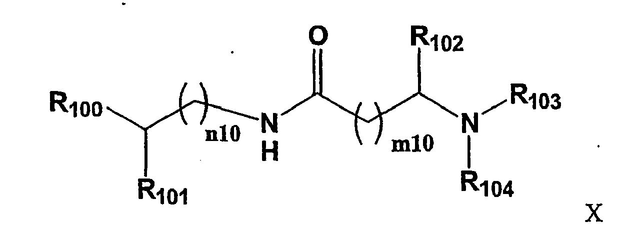 wherein:
R₁₀₀ is independently chosen from C₆-C₁₀ unsubstituted aryl, C₆-C₁₇ substituted aryl, C₆-C₁₄ aralkyl, C₄-C₉ unsubstituted heteroaryl, and C₄-C₁₅ substituted heteroaryl;
R₁₀₁ is independently chosen from H, -OH, C₁-C₄alkyl, -O-C₁-C₄ alkyl, C₃-C₈ cycloalkyl, C₇-C₁₄ aralkyl, C₆-C₁₀ aryl, C₆-C₁₇ substituted aryl;
R₁₀₂ is independently chosen from H, F, C₁-C₄ alkyl, C₃-C₈ cycloalkyl, C₇-C₁₄ aralkyl, C₆-C₁₀ aryl, C₆-C₁₇ substituted aryl;
R₁₀₃ and R₁₀₄ are independently chosen from H, C₁-C₄ alkyl, C₃-C₈ cycloalkyl, C₇-C₁₄ aralkyl, C₆-C₁₀ aryl, C₆-C₁₇ substituted aryl;
n10 is independently chosen from 0 and 1; and
m10 is independently chosen from 0 and 1;
   including all stereoisomers thereof, all E/Z (cis/trans) isomers thereof, all solvates and hydrates thereof, all crystalline and non-crystalline forms thereof, and all salts thereof, particularly pharmaceutically-acceptable salts. Metabolites and prodrugs of the compounds of formula X are also embraced by the invention.

When R₁₀₀ is substituted, preferred substituents are -F, -Cl, -CF₃, -OH, -C₁-C₄ alkyl, and -O-C₁-C₄ alkyl, more preferably -F, -Cl, -CF₃, and methyl, most preferably -F. When R₁₀₀ is C₄-C₉ unsubstituted heteroaryl, R₁₀₀ is preferably 2-pyridyl, 3-pyridyl, or 4-pyridyl. When R₁₀₀ is C₄-C₁₅ substituted heteroaryl, preferred substituents are -Cl. In another embodiment, R₁₀₀ is independently unsubstituted phenyl. In another embodiment, R₁₀₀ is independently substituted phenyl. In another embodiment, R₁₀₀ is independently monosubstituted phenyl. In another embodiment, R₁₀₀ is independently disubstituted phenyl. In another embodiment, R₁₀₀ is independently trisubstituted phenyl. In another embodiment, R₁₀₀ is independently 4-Me-phenyl. In another embodiment, R₁₀₀ is independently 2-F-phenyl. In another embodiment, R₁₀₀ is independently 3-F-phenyl. In another embodiment, R₁₀₀ is independently 4-F-phenyl. In another embodiment, R₁₀₀ is independently 3-CF₃-phenyl. In another embodiment, R₁₀₀ is independently 4-CF₃-phenyl. In another embodiment, R₁₀₀ is independently 2-F-3-CF₃-phenyl. In another embodiment, R₁₀₀ is independently 2-F-4-CF₃-phenyl. In another embodiment, R₁₀₀ is independently 2-F-5-CF₃-phenyl. In another embodiment, R₁₀₀ is independently 3-5-di-CF₃-phenyl. In another embodiment, R₁₀₀ is independently 3-F-4-CF₃-phenyl. In another embodiment, R₁₀₀ is independently 3-F-5-CF₃-phenyl. In another embodiment, R₁₀₀ is independently 4-F-2-CF₃-phenyl. In another embodiment, R₁₀₀ is independently 4-F-3-CF₃-phenyl. In another embodiment, R₁₀₀ is independently 2-pyridyl. In another embodiment, R₁₀₀ is independently 3-pyridyl. In another embodiment, R₁₀₀ is independently 4-pyridyl. In another embodiment, R₁₀₀ is independently 6-Cl-3-pyridyl. When R₁₀₀ is C₄-C₉ unsubstituted heteroaryl, R₁₀₀ is preferably 2-pyridyl, 3-pyridyl, or 4-pyridyl. When R₁₀₀ is C₄-C₁₅ substituted heteroaryl, preferred substituents are -Cl. In another embodiment, R₁₀₀ is independently unsubstituted phenyl. In another embodiment, R₁₀₀ is independently substituted phenyl. In another embodiment, R₁₀₀ is independently monosubstituted phenyl. In another embodiment, R₁₀₀ is independently disubstituted phenyl. In another embodiment, R₁₀₀ is independently trisubstituted phenyl. In another embodiment, R₁₀₀ is independently 4-Me-phenyl. In another embodiment, R₁₀₀ is independently 2-F-phenyl. In another embodiment, R₁₀₀ is independently 3-F-phenyl. In another embodiment, R₁₀₀ is independently 4-F-phenyl. In another embodiment, R₁₀₀ is independently 3-CF₃-phenyl. In another embodiment, R₁₀₀ is independently 4-CF₃-phenyl. In another embodiment, R₁₀₀ is independently 2-F-3-CF₃-phenyl. In another embodiment, R₁₀₀ is independently 2-F-4-CF₃-phenyl. In another embodiment, R₁₀₀ is independently 2-F-5-CF₃-phenyl. In another embodiment, R₁₀₀ is independently 3-5-di-CF₃-phenyl. In another embodiment, R₁₀₀ is independently 3-F-4-CF₃-phenyl. In another embodiment, R₁₀₀ is independently 3-F-5-CF₃-phenyl. In another embodiment, R₁₀₀ is independently 4-F-2-CF₃-phenyl. In another embodiment, R₁₀₀ is independently 4-F-3-CF₃-phenyl. In another embodiment, R₁₀₀ is independently 2-pyridyl. In another embodiment, R₁₀₀ is independently 3-pyridyl. In another embodiment, R₁₀₀ is independently 4-pyridyl. In another embodiment, R₁₀₀ is independently 6-Cl-3-pyridyl. In one embodiment, R₁₀₁ is independently H. In another embodiment, R₁₀₁ is independently -OH. In another embodiment, R₁₀₁ is independently C₁-C₄ alkyl. In another embodiment, R₁₀₁ is independently methyl. In one embodiment, R₁₀₂ is independently H. In another embodiment, R₁₀₂ is independently C₁-C₄ alkyl. In another embodiment, R₁₀₂ is independently methyl. In another embodiment, R₁₀₂ is independently F. In one embodiment, R₁₀₃ is independently H. In another embodiment, R₁₀₃ is independently C₁-C₄ alkyl. In another embodiment, R₁₀₃ is independently methyl. In another embodiment, R₁₀₃ is independently ethyl. In another embodiment, R₁₀₃ is independently n-propyl. In another embodiment, R₁₀₃ is independently isopropyl. In another embodiment, R₁₀₃ is independently C₇-C₁₄ aralkyl. In another embodiment, R₁₀₃ is independently C₆-C₁₀ aryl. In another embodiment, R₁₀₃ is independently C₆-C₁₇ substituted aryl. In another embodiment, R₁₀₃ is independently benzyl. In another embodiment, R₁₀₃ is independently unsubstituted phenyl. In another embodiment, R₁₀₃ is independently substituted phenyl. In another embodiment, R₁₀₃ is independently monosubstituted phenyl. In another embodiment, R₁₀₃ is independently disubstituted phenyl. In another embodiment, R₁₀₃ is independently trisubstituted phenyl. In another embodiment, R₁₀₃ is independently 4-fluorophenyl (4-F-Ph). In another embodiment, R₁₀₃ is independently 4-methylphenyl (4-Me-Ph). In one embodiment, R₁₀₄ is independently H. In another embodiment, R₁₀₄ is independently C₁-C₄ alkyl. In another embodiment, R₁₀₄ is independently methyl. In another embodiment, R₁₀₄ is independently ethyl. In another embodiment, R₁₀₄ is independently n-propyl. In another embodiment, R₁₀₄ is independently isopropyl. In another embodiment, R_{10\4} is independently C₇-C₁₄ aralkyl. In another embodiment, R₁₀₄ is independently C₆-C₁₀ aryl. In another embodiment, R₁₀₄ is independently C₆-C₁₇ substituted aryl. In another embodiment, R₁₀₄ is independently benzyl. In another embodiment, R₁₀₄ is independently unsubstituted phenyl. In another embodiment, R₁₀₄ is independently substituted phenyl. In another embodiment, R₁₀₄ is independently monosubstituted phenyl. In another embodiment, R₁₀₄ is independently disubstituted phenyl. In another embodiment, R₁₀₄ is independently trisubstituted phenyl. In another embodiment, R₁₀₄ is independently 4-fluorophenyl (4-F-Ph). In another embodiment, R₁₀₄ is independently 4-methylphenyl (4-Me-Ph). In another embodiment, n10 is 0. In another embodiment, n10 is 1. In another embodiment, m10 is 0. In another embodiment, m10 is 1.

In another embodiment, the present invention relates to methods of using the compounds of formula X to inhibit SSAO enzyme activity (whether the enzyme activity is due either to soluble SSAO enzyme or membrane-bound VAP-1 protein, or due to both) and/or inhibit binding to VAP-1 protein, by administering one or more of the compounds in an amount sufficient to inhibit SSAO enzyme activity and/or inhibit binding to VAP-1 protein. The compounds can be used for a method of inhibiting SSAO activity or inhibiting binding to VAP-1 *in vitro,* by supplying the compound to the *in vitro* environment in an amount sufficient to inhibit SSAO activity or inhibit binding to VAP-1. The compounds can also be used for a method of inhibiting SSAO activity or inhibiting binding to VAP-1 *in vivo*, that is, in a living organism, such as a vertebrate, mammal, or human, by administering the compounds to the organism in an amount sufficient to inhibit SSAO activity or inhibit binding to VAP-1. In another embodiment, the present invention relates to methods of using the compounds of formula X to treat inflammation or immune disorders. In another embodiment, the present invention relates to methods of using the compounds of formula X to suppress or reduce inflammation, or to suppress or reduce an inflammatory response. In another embodiment, the present invention relates to methods of treating inflammation, by administering one or more of the compounds described in formula X in a therapeutically effective amount, or in an amount sufficient to treat inflammation. In another embodiment, the present invention relates to methods of treating immune or autoimmune disorders, by administering one or more of the compounds described in formula X in a therapeutically effective amount, or in an amount sufficient to treat the immune or autoimmune disorder. In another embodiment, the disease to be treated is an ischemic disease (for example, stroke) and/or the sequelae thereof (for example, an inflammatory response). In another embodiment, the disease to be treated is multiple sclerosis (e.g., chronic multiple sclerosis).

For all of the compounds described above, that is, for formulas I, II, III, IV, V, VI, VII, VIII, IX, and X, and any subsets thereof, when a substituent can be selected from aryl, a preferred aryl substituent is phenyl. "Aryl" in the formulas above can refer to either unsubstituted aryl or substituted aryl, unless already qualified as either unsubstituted or substituted. Likewise, "phenyl" in the formulas above can refer to either unsubstituted phenyl or substituted phenyl, unless already qualified as either unsubstituted or substituted.

In another embodiment, the inflammatory disease or immune disorder to be treated by one or more of the compounds of formulas I, II, III, IV, V, VI, VII, VIII, IX, and/or X of the present invention is selected from the group consisting of multiple sclerosis (including chronic multiple sclerosis); synovitis; systemic inflammatory sepsis; inflammatory bowel diseases; Crohn's disease; ulcerative colitis; Alzheimer's disease; vascular dementia; atherosclerosis; rheumatoid arthritis; juvenile rheumatoid arthritis; pulmonary inflammatory conditions; asthma; skin inflammatory conditions and diseases; contact dermatitis; liver inflammatory and autoimmune conditions; autoimmune hepatitis; primary biliary cirrhosis; sclerosing cholangitis; autoimmune cholangitis; alcoholic liver disease; Type I diabetes and/or complications thereof; Type II diabetes and/or complications thereof; atherosclerosis; chronic heart failure; congestive heart failure; ischemic diseases such as stroke and/or complications thereof; and myocardial infarction and/or complications thereof. In another embodiment, the inflammatory disease or immune disorder to be treated by the present invention is multiple sclerosis (including chronic multiple sclerosis). In another embodiment, the inflammatory disease or immune disorder to be treated by the present invention is the inflammatory complications resulting from stroke.

A compound of formula I, II, III, IV, V, VI, VII, VIII, IX, or X as described above can be administered singly in a therapeutically effective amount. A compound of formula I, II, III, IV, V, VI, VII, VIII, IX, or X as described above can be administered with one or more additional compounds of formulas I, II, III, IV, V, VI, VII, VIII, IX, or X, in a therapeutically effective amount. When administered in combination, the compounds can be administered in amounts that would therapeutically effective were the compounds to be administered singly. Alternatively, when administered in combination, any or all of compounds can be administered in amounts that would not be therapeutically effective were the compounds to be administered singly, but which are therapeutically effective in combination. One or more compounds of formulas I, II, III, IV, V, VI, VII, VIII, IX, or X can also be administered with other compounds not included in formulas I, II, III, IV, V, VI, VII, VIII, IX, or X; the compounds can be administered in amounts that are therapeutically effective when used as single drugs, or in amounts which are not therapeutically effective as single drugs, but which are therapeutically effective in combination. Also provided are pharmaceutically acceptable compositions comprising a therapeutically effective amount of one or more of the compounds disclosed herein or a therapeutically effective combination of two or more of the compounds disclosed herein, including the compounds of formulas I, II, III, IV, V, VI, VII, VIII, IX, and/or X above, and a pharmaceutically acceptable carrier; and human unit dosages thereof.

A compound of formula I, II, III, IV, V, VI, VII, VIII, IX, and/or X as described above can be prepared as an isolated pharmaceutical composition, and administered as an isolated pharmaceutical composition in conjunction with vehicles or other isolated compounds. That is, a compound of formula I, II, III, IV, V, VI, VII, VIII, IX, and/or X as described above can be isolated from other compounds (e.g., a compound which is discovered in a library screening assay can be purified out of the library, or synthesized *de novo* as a single compound). The degree of purification can be 90%, 95%, 99%, or whatever percentage of purity is required for pharmaceutical use of the compound. The isolated compound can then be combined with pharmaceutically acceptable vehicles, or can be combined with one or more isolated compounds of formulas I, II, III, IV, V, VI, VII, VIII, IX, and/or X, or with another therapeutic substance. A compound of formula I, II, III, IV, V, VI, VII, VIII, IX, and/or X as described above can be administered orally, in a pharmaceutical human unit dosage formulation.

In another embodiment, the invention embraces compounds of formula I for use in therapy. In another embodiment, the invention embraces compounds of formula I for manufacture of a medicament for treatment of inflammatory diseases. In another embodiment, the invention embraces compounds of formula I for manufacture of a medicament for treatment of immune or autoimmune diseases. In another embodiment, the invention embraces compounds of formula I for manufacture of a medicament for treatment of multiple sclerosis or chronic multiple sclerosis. In another embodiment, the invention embraces compounds of formula I for manufacture of a medicament for treatment of ischemic diseases (such as stroke) or the sequelae of ischemic diseases.

In another embodiment, the invention embraces compounds of formula II for use in therapy. In another embodiment, the invention embraces compounds of formula II for manufacture of a medicament for treatment of inflammatory diseases. In another embodiment, the invention embraces compounds of formula II for manufacture of a medicament for treatment of immune or autoimmune diseases. In another embodiment, the invention embraces compounds of formula II for manufacture of a medicament for treatment of multiple sclerosis or chronic multiple sclerosis. In another embodiment, the invention embraces compounds of formula II for manufacture of a medicament for treatment of ischemic diseases (such as stroke) or the sequelae of ischemic diseases.

In another embodiment, the invention embraces compounds of formula III for use in therapy. In another embodiment, the invention embraces compounds of formula III for manufacture of a medicament for treatment of inflammatory diseases. In another embodiment, the invention embraces compounds of formula III for manufacture of a medicament for treatment of immune or autoimmune diseases. In another embodiment, the invention embraces compounds of formula III for manufacture of a medicament for treatment of multiple sclerosis or chronic multiple sclerosis. In another embodiment, the invention embraces compounds of formula III for manufacture of a medicament for treatment of ischemic diseases (such as stroke) or the sequelae of ischemic diseases.

In another embodiment, the invention embraces compounds of formula IV for use in therapy. In another embodiment, the invention embraces compounds of formula IV for manufacture of a medicament for treatment of inflammatory diseases. In another embodiment, the invention embraces compounds of formula IV for manufacture of a medicament for treatment of immune or autoimmune diseases. In another embodiment, the invention embraces compounds of formula IV for manufacture of a medicament for treatment of multiple sclerosis or chronic multiple sclerosis. In another embodiment, the invention embraces compounds of formula IV for manufacture of a medicament for treatment of ischemic diseases (such as stroke) or the sequelae of ischemic diseases.

In another embodiment, the invention embraces compounds of formula V for use in therapy. In another embodiment, the invention embraces compounds of formula V for manufacture of a medicament for treatment of inflammatory diseases. In another embodiment, the invention embraces compounds of formula V for manufacture of a medicament for treatment of immune or autoimmune diseases. In another embodiment, the invention embraces compounds of formula V for manufacture of a medicament for treatment of multiple sclerosis or chronic multiple sclerosis. In another embodiment, the invention embraces compounds of formula V for manufacture of a medicament for treatment of ischemic diseases (such as stroke) or the sequelae of ischemic diseases.

In another embodiment, the invention embraces compounds of formula VI for use in therapy. In another embodiment, the invention embraces compounds of formula VI for manufacture of a medicament for treatment of inflammatory diseases. In another embodiment, the invention embraces compounds of formula VI for manufacture of a medicament for treatment of immune or autoimmune diseases. In another embodiment, the invention embraces compounds of formula VI for manufacture of a medicament for treatment of multiple sclerosis or chronic multiple sclerosis. In another embodiment, the invention embraces compounds of formula VI for manufacture of a medicament for treatment of ischemic diseases (such as stroke) or the sequelae of ischemic diseases.

In another embodiment, the invention embraces compounds of formula VII for use in therapy. In another embodiment, the invention embraces compounds of formula VII for manufacture of a medicament for treatment of inflammatory diseases. In another embodiment, the invention embraces compounds of formula VII for manufacture of a medicament for treatment of immune or autoimmune diseases. In another embodiment, the invention embraces compounds of formula VII for manufacture of a medicament for treatment of multiple sclerosis or chronic multiple sclerosis. In another embodiment, the invention embraces compounds of formula VII for manufacture of a medicament for treatment of ischemic diseases (such as stroke) or the sequelae of ischemic diseases.

In another embodiment, the invention embraces compounds of formula VIII for use in therapy. In another embodiment, the invention embraces compounds of formula VIII for manufacture of a medicament for treatment of inflammatory diseases. In another embodiment, the invention embraces compounds of formula VIII for manufacture of a medicament for treatment of immune or autoimmune diseases. In another embodiment, the invention embraces compounds of formula VIII for manufacture of a medicament for treatment of multiple sclerosis or chronic multiple sclerosis. In another embodiment, the invention embraces compounds of formula VIII for manufacture of a medicament for treatment of ischemic diseases (such as stroke) or the sequelae of ischemic diseases.

In another embodiment, the invention embraces compounds of formula IX for use in therapy. In another embodiment, the invention embraces compounds of formula IX for manufacture of a medicament for treatment of inflammatory diseases. In another embodiment, the invention embraces compounds of formula IX for manufacture of a medicament for treatment of immune or autoimmune diseases. In another embodiment, the invention embraces compounds of formula IX for manufacture of a medicament for treatment of multiple sclerosis or chronic multiple sclerosis. In another embodiment, the invention embraces compounds of formula IX for manufacture of a medicament for treatment of ischemic diseases (such as stroke) or the sequelae of ischemic diseases.

In another embodiment, the invention embraces compounds of formula X for use in therapy. In another embodiment, the invention embraces compounds of formula X for manufacture of a medicament for treatment of inflammatory diseases. In another embodiment, the invention embraces compounds of formula X for manufacture of a medicament for treatment of immune or autoimmune diseases. In another embodiment, the invention embraces compounds of formula X for manufacture of a medicament for treatment of multiple sclerosis or chronic multiple sclerosis. In another embodiment, the invention embraces compounds of formula X for manufacture of a medicament for treatment of ischemic diseases (such as stroke) or the sequelae of ischemic diseases.

### BRIEF DESCRIPTION OF THE DRAWINGS

Fig. 1A depicts the effect of mofegiline (Example 8) on experimental autoimmune encephalitis (EAE) development as assessed by clinical severity, versus vehicle control and methotrexate.

Fig. 1B depicts the effect of mofegiline (Example 8) on EAE development as assessed by percent incidence, versus vehicle control and methotrexate.

Fig. 1C depicts the effect of mofegiline (Example 8) on EAE development as assessed by body weight, versus vehicle control and methotrexate.

Figure 2A depicts the effects of LJP 1383 p.o. and LJP 1379 p.o. on paw edema following carrageenan injection.

Figure 2B depicts the effects of LJP 1406 p.o. on paw edema following carrageenan injection.

Figure 2C depicts the effects of LJP 1379 i.p. on paw edema following carrageenan injection.

### MODES FOR CARRYING OUT THE INVENTION

The present invention relates to various compounds which are useful for inhibiting SSAO enzyme activity (where the enzyme activity is due either to soluble SSAO enzyme or membrane-bound VAP-1 protein, or due to both) and/or inhibition of binding to membrane-bound VAP-1 protein. The present invention also relates to methods of using various compounds to inhibit SSAO enzyme activity (where the enzyme activity is due either to soluble SSAO enzyme or membrane-bound VAP-1 protein, or due to both) and/or inhibit binding to VAP-1 protein. The present invention also relates to methods of using various compounds to treat inflammation or immune disorders, and to reduce or suppress inflammation and/or inflammatory responses. The present invention also relates to methods of using various compounds to treat ischemic diseases, such as stroke, and/or to treat the sequelae of ischemic diseases, such as stroke.

Compounds for use in the invention can be assayed for SSAO inhibitory activity by the protocol in Example 4 below. The substrate specificity of SSAO versus monoamine oxidase partially overlap. Thus it is preferable to use compounds which specifically inhibit SSAO over monoamine oxidase. The specificity of the compounds for SSAO inhibitory activity versus MAO-A and MAO-B inhibitory activity can be assayed by the protocol in Example 5 below.

Compounds for use in the invention have an inhibitory activity (IC₅₀) against SSAO of less than or equal to about 1 µM, more preferably of less than or equal to about 100 nM, and more preferably of less than or equal to about 10 nM. Preferably, compounds for use in the invention also have a specificity for SSAO versus MAO-A of less than or equal to about 10, more preferably less than or equal to about 100, more preferably less than or equal to about 500 (where specificity for SSAO versus MAO-A is defined as the ratio of the IC₅₀ of a compound for MAO-A to the IC₅₀ of the same compound for SSAO; that is, a compound with an IC₅₀ of 10 µM for MAO-A and an IC₅₀ of 20 nM for SSAO has a specificity of 500 for SSAO versus MAO-A). Compounds for use in the invention also have a specificity for SSAO versus MAO-B of less than or equal to about 10, more preferably of less than or equal to about 100, more preferably of less than or equal to about 500 (where specificity for SSAO versus MAO-B is defined as the ratio of the IC₅₀ of a compound for MAO-B to the IC₅₀ of the same compound for SSAO).

The term "inhibit binding to VAP-1 protein" is meant to indicate inhibition (which can include partial to complete inhibition) of binding between, for example, a cell expressing the SSAO/VAP-1 protein on its surface, and a binding partner of SSAO/VAP-1 protein. Such binding occurs, for example, when a cell expressing the SSAO/VAP-1 protein on its surface interacts with another cell expressing a binding partner of SSAO/VAP-1 protein, such as a high endothelial cell (HEC). Thus "inhibit binding to VAP-1 protein" embraces inhibition of adhesion between a cell expressing the SSAO/VAP-1 protein on its surface, and another cell expressing a binding partner of SSAO/VAP-1 protein. Such adhesion events include, for example, cell rolling. As this disclosure (including the examples) clearly indicates, such inhibition can occur either *in vitro* or *in vivo.*

The invention includes all salts of the inventive compounds described herein, as well as methods of using such salts of the compounds. The invention also includes the non-salt compound of any salt of an inventive compound described herein, as well as all other salts of any salt of an inventive compound named herein. In one embodiment, the salts of the compounds comprise pharmaceutically acceptable salts. Pharmaceutically acceptable salts are those salts which retain the biological activity of the free compounds and which are not biologically or otherwise undesirable. The desired salt of a basic compound may be prepared by methods known to those of skill in the art by treating the compound with an acid. Examples of inorganic acids include, but are not limited to, hydrochloric acid, hydrobromic acid, sulfuric acid, nitric acid, and phosphoric acid. Examples of organic acids include, but are not limited to, formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, sulfonic acids, and salicylic acid. Salts of basic compounds with amino acids, such as aspartate salts and glutamate salts, can also be prepared. The desired salt of an acidic compound can be prepared by methods known to those of skill in the art by treating the compound with a base. Examples of inorganic salts of acid compounds include, but are not limited to, alkali metal and alkaline earth salts, such as sodium salts, potassium salts, magnesium salts, and calcium salts; ammonium salts; and aluminum salts. Examples of organic salts of acid compounds include, but are not limited to, procaine, dibenzylamine, N-ethylpiperidine, N,N'-dibenzylethylenediamine, and triethylamine salts. Salts of acidic compounds with amino acids, such as lysine salts, can also be prepared.

The invention also includes all stereoisomers of the compounds, including diastereomers and enantiomers, as well as mixtures of stereoisomers, including, but not limited to, racemic mixtures. Unless stereochemistry is explicitly indicated in a chemical structure or chemical name, the chemical structure or chemical name is intended to embrace all possible stereoisomers of the compound depicted. Also, while the general formula I is drawn with only one of the *cis-trans* isomers depicted (with R₁ and R₂ depicted as *cis* to each other), the drawing is intended to embrace both the compounds with R₁ and R₂ in the *cis* position as well as R₁ and R₂ in the *trans* position (that is, the single drawing is used to represent both the E and Z isomers, although only one isomer is drawn). General formula IV is also intended to embrace both cis and trans isomers (that is, with the groups bearing the CF₃ and NH₂ functions either *cis* or *trans* to each other).

The term "alkyl" refers to saturated aliphatic groups including straight-chain, branched-chain, cyclic groups, and combinations thereof, having the number of carbon atoms specified, or if no number is specified, having up to 12 carbon atoms. "Straight-chain alkyl" or "linear alkyl" groups refers to alkyl groups that are neither cyclic nor branched, commonly designated as "n-alkyl" groups. Examples of alkyl groups include, but are not limited to, groups such as methyl, ethyl, n-propyl, isopropyl, butyl, n-butyl, isobutyl, sec-butyl, t-butyl, pentyl, n-pentyl, hexyl, heptyl, octyl, nonyl, decyl, undecyl, dodecyl, neopentyl, cyclopropyl, cyclobutyl, cyclopentyl, cyclohexyl, and adamantyl. Cycloalkyl groups can consist of one ring, including, but not limited to, groups such as cycloheptyl, or multiple fused rings, including, but not limited to, groups such as adamantyl or norbornyl.

"Substituted alkyl" refers to alkyl groups substituted with one or more substituents including, but not limited to, groups such as halogen (fluoro, chloro, bromo, and iodo), alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group. Examples of substituted alkyl groups include, but are not limited to, -CF₃, -CF₂-CF₃, and other perfluoro and perhalo groups; -CH₂-OH; -CH₂CH₂CH(NH₂)CH₃, etc.

The term "alkenyl" refers to unsaturated aliphatic groups including straight-chain (linear), branched-chain, cyclic groups, and combinations thereof, having the number of carbon atoms specified, or if no number is specified, having up to 12 carbon atoms, which contain at least one double bond (-C=C-). Examples of alkenyl groups include, but are not limited to, -CH₂-CH=CH-CH₃; and -CH₂-CH₂-cyclohexenyl, where the ethyl group can be attached to the cyclohexenyl moiety at any available carbon valence. The term "alkynyl" refers to unsaturated aliphatic groups including straight-chain (linear), branched-chain, cyclic groups, and combinations thereof, having the number of carbon atoms specified, or if no number is specified, having up to 12 carbon atoms, which contain at least one triple bond (-C≡C-). "Hydrocarbon chain" or "hydrocarbyl" refers to any combination of straight-chain, branched-chain, or cyclic alkyl, alkenyl, or alkynyl groups, and any combination thereof. "Substituted alkenyl," "substituted alkynyl," and "substituted hydrocarbon chain" or "substituted hydrocarbyl" refer to the respective group substituted with one or more substituents, including, but not limited to, groups such as halogen, alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group.

"Aryl" or "Ar" refers to an aromatic carbocyclic group having a single ring (including, but not limited to, groups such as phenyl) or two or more condensed rings (including, but not limited to, groups such as naphthyl or anthryl), and includes both unsubstituted and substituted aryl groups. Aryls, unless otherwise specified, contain from 6 to 12 carbon atoms in the ring portion. A preferred range for aryls is from 6 to 10 carbon atoms in the ring portion. "Substituted aryls" refers to aryls substituted with one or more substituents, including, but not limited to, groups such as alkyl, alkenyl, alkynyl, hydrocarbon chains, halogen, alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group. "Aralkyl" designates an alkyl-substituted aryl group, where any aryl can attached to the alkyl; the alkyl portion is a straight or branched chain of 1 to 6 carbon atoms, preferably the alkyl chain contains 1 to 3 carbon atoms. When an aralkyl group is indicated as a substituent, the aralkyl group can be connected to the remainder of the molecule at any available valence on either its alkyl moiety or aryl moiety; e.g., the tolyl aralkyl group can be connected to the remainder of the molecule by replacing any of the five hydrogens on the aromatic ring moiety with the remainder of the molecule, or by replacing one of the alpha-hydrogens on the methyl moiety with the remainder of the molecule. Preferably, the aralkyl group is connected to the remainder of the molecule via the alkyl moiety.

A preferred aryl group is phenyl, which can be substituted or unsubstituted. Preferred substitutents for aryl groups and substituted phenyl groups' are lower alkyl (-C₁-C₄ alkyl) such as methyl, ethyl, propyl (either n-propyl or i-propyl), and butyl (either n-butyl, i-butyl, sec-butyl, or tert-butyl); trifluoromethyl (-CF₃); or a halogen (chlorine (-Cl), bromine (-Br), iodine (-I), or fluorine (-F); preferred halogen substituents for phenyl groups are chlorine and fluorine); hydroxy (-OH), or lower alkoxy (-C₁-C₄ alkoxy), such as methoxy, ethoxy, propyloxy (propoxy) (either n-propoxy or i-propoxy), and butoxy (either n-butoxy, i-butoxy, sec-butoxy, or tert-butoxy), a preferred alkoxy substituent is methoxy. Substituted phenyl groups preferably have one or two substituents; more preferably, one substituent.

"Heteroalkyl," "heteroalkenyl," and "heteroalkynyl" refer to alkyl, alkenyl, and alkynyl groups, respectively, that contain the number of carbon atoms specified (or if no number is specified, having up to 12 carbon atoms) which contain one or more heteroatoms as part of the main, branched, or cyclic chains in the group. Heteroatoms include, but are not limited to, N, S, O, and P; N and O are preferred. Heteroalkyl, heteroalkenyl, and heteroalkynyl groups may be attached to the remainder of the molecule either at a heteroatom (if a valence is available) or at a carbon atom. Examples of heteroalkyl groups include, but are not limited to, groups such as -O-CH₃, -CH₂-O-CH₃, -CH₂-CH₂-O-CH₃, -S-CH₂-CH₂-CH₃, -CH₂-CH(CH₃)-S-CH₃, -CH₂-CH₂-NH-CH₂-CH₂-, 1-ethyl-6-propylpiperidino, and morpholino. Examples of heteroalkenyl groups include, but are not limited to, groups such as. -CH=CH-NH-CH(CH₃)-CH₂-. "Heteroaryl" or "HetAr" refers to an aromatic carbocyclic group having a single ring (including, but not limited to, examples such as pyridyl, imidazolyl, thiophene, or furyl) or two or more condensed rings (including, but not limited to, examples such as indolizinyl or benzothienyl) and having at least one hetero atom, including, but not limited to, heteroatoms such as N, O, P, or S, within the ring. Unless otherwise specified, heteroalkyl, heteroalkenyl, heteroalkynyl, and heteroaryl groups have between one and five heteroatoms and between one and twelve carbon atoms. "Substituted heteroalkyl," "substituted heteroalkenyl," "substituted heteroalkynyl," and "substituted heteroaryl" groups refer to heteroalkyl, heteroalkenyl, heteroalkynyl, and heteroaryl groups substituted with one or more substituents, including, but not limited to, groups such as alkyl, alkenyl, alkynyl, benzyl, hydrocarbon chains, halogen, alkoxy, acyloxy, amino, hydroxyl, mercapto, carboxy, benzyloxy, phenyl, benzyl, cyano, nitro, thioalkoxy, carboxaldehyde, carboalkoxy and carboxamide, or a functionality that can be suitably blocked, if necessary for purposes of the invention, with a protecting group. Examples of such substituted heteroalkyl groups include, but are not limited to, piperazine, substituted at a nitrogen or carbon by a phenyl or benzyl group, and attached to the remainder of the molecule by any available valence on a carbon or nitrogen, -NH-SO₂-phenyl, -NH-(C=O)O-alkyl, -NH-(C=O)O-alkyl-aryl, and -NH-(C=O)-akyl. If chemically possible, the heteroatom(s) and/or the carbon atoms of the group can be substituted. The heteroatom(s) can also be in oxidized form, if chemically possible. Preferred substituents for heteroaryl groups are the same as the preferred substituents for aryl and substituted phenyl groups.

The term "alkoxy" as used herein refers to an alkyl, alkenyl, alkynyl, or hydrocarbon chain linked to an oxygen atom and having the number of carbon atoms specified, or if no number is specified, having up to 12 carbon atoms. Examples of alkoxy groups include, but are not limited to, groups such as methoxy, ethoxy, propyloxy (propoxy) (either n-propoxy or i-propoxy), and butoxy (either n-butoxy, i-butoxy, sec-butoxy, or tert-butoxy). The groups listed in the preceding sentence are preferred alkoxy groups; a particularly preferred alkoxy substituent is methoxy.

The terms "halo" and "halogen" as used herein refer to the Group VIIa elements (Group 17 elements in the 1990 IUPAC Periodic Table, IUPAC Nomenclature of Inorganic Chemistry, Recommendations 1990) and include Cl, Br, F and I substituents. Preferred halogen substituents are Cl and F.

"Protecting group" refers to a chemical group that exhibits the following characteristics: 1) reacts selectively with the desired functionality in good yield to give a protected substrate that is stable to the projected reactions for which protection is desired; 2) is selectively removable from the protected substrate to yield the desired functionality; and 3) is removable in good yield by reagents compatible with the other functional group(s) present or generated in such projected reactions. Examples of suitable protecting groups can be found in Greene et al. (1991) Protective Groups in Organic Synthesis, 3rd Ed. (John Wiley & Sons, Inc., New York). Amino protecting groups include, but are not limited to, mesitylenesulfonyl (Mts), benzyloxycarbonyl (CBz or Z), t-butyloxycarbonyl (Boc), t-butyldimethylsilyl (TBS or TBDMS), 9-fluorenylmethyloxycarbonyl (Fmoc), tosyl, benzenesulfonyl, 2-pyridyl sulfonyl, or suitable photolabile protecting groups such as 6-nitroveratryloxy carbonyl (Nvoc), nitropiperonyl, pyrenylmethoxycarbonyl, nitrobenzyl, dimethyl dimethoxybenzil, 5-bromo-7-nitroindolinyl, and the like. Hydroxyl protecting groups include, but are not limited to, Fmoc, TBS, photolabile protecting groups (such as nitroveratryl oxymethyl ether (Nvom)), Mom (methoxy methyl ether), and Mem (methoxy ethoxy methyl ether), NPEOC (4-nitrophenethyloxycarbonyl) and NPEOM
(4-nitrophenethyloxymethyloxycarbonyl).

### General Synthetic Methods

Compounds of formula I, II, III, IV, V, VI, ,VII, VIII, IX, and X can be synthesized by various methods. Specific synthetic examples are provided below in the Examples. General methods of synthesis are provided here.

### General procedures for the preparation of compounds of formula I

2-(2-bromomethyl-allyl)-isoindole-1,3-dione (**4**) (see Example 1 for synthesis) is a useful intermediate in synthesizing many of the compounds of formula I.

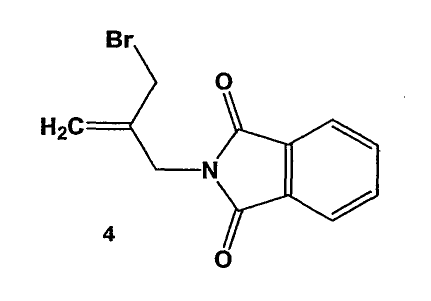

This intermediate can be reacted with a wide variety of amides under basic conditions (e.g., using NaH). For compounds bearing groups of the form Ia, the amide corresponding to the precursor to the group of form Ia which is used is of the form **5:**

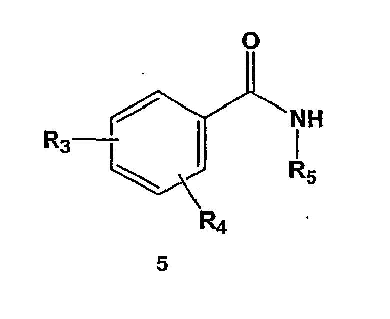

An example of an amide which can be used is commercially available 4-chloro-N-methylbenzamide (Aldrich). This amide is used to synthesize compounds where the group of formula Ia is of the form **6:**

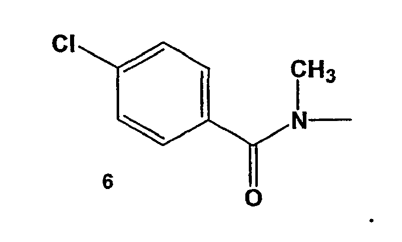

Other amides of the form Ia can be easily synthesized via their commercially available benzoic acid precursors. For example, 3-methoxybenzoic acid (m-anisic acid; Aldrich) can be converted to its corresponding acid chloride with thionyl chloride. The acid chloride can then be reacted with a compound of the form H₂NR₅ (where R₅ is chosen from H, C₁-C₄ alkyl, C₃-C₈ cycloalkyl, or C₆-C₁₄ aralkyl) to form the desired amide of the form Ia. A wide variety of other reagents can also be used to form the amide from the benzoic acid and the amine compound. For example, dicyclohexylcarbodiimide (DCC), diisopropylcarbodiimide (DIPCDI), 1-(3-dimethylaminopropyl)-3-ethyl-carbodiimide hydrochloride (EDC), benzotriazole-1-yl-oxy-tris-(dimethylamino)-phosphonium hexafluorophosphate (BOP), 2-(1H-benzotriazole-1-yl)-1,1,3,3-tetramethyluronium hexafluorophosphate (HBTU), 2-(1H-benzotriazole-1-yl)-1,1,3,3-tetramethyluronium tetrafluoroborate (TBTU), or O-(7-azabenzotriazol-1-yl)-N,N,N',N'-tetramethyluronium hexafluorophosphate (HATU) can be used to condense the acid and amine into an amide.

For compounds bearing groups of the form Ib, the amide corresponding to the precursor to the group of form Ib which is used in this procedure is of the form **7:**

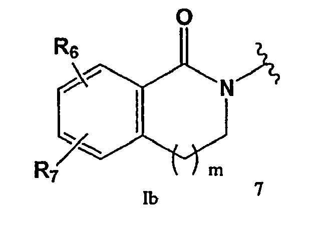

Examples of amides which can be used are commercially available 6,7-dimethoxy-3,4-dihydro-2(1H)-isoquinolinone (Aldrich) or methyl-7-methoxy-1-oxo-1,2,3,4-tetrahydro-5-isoquinolinecarboxylate (Aldrich). These amides are used to synthesize compounds where the groups of formula Ib are of the form **8** or **9,** respectively. Other isoquinolinone derivatives can be prepared as described in Tetrahedron Letters, 39:6609-6612 (1998).

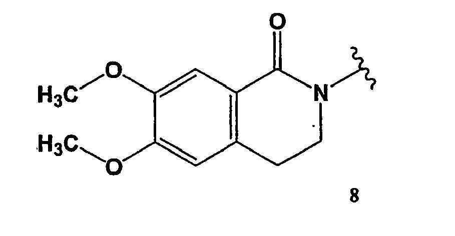

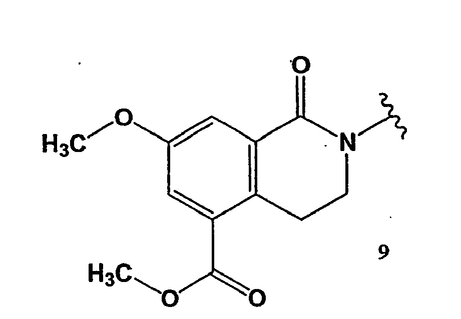

For compounds bearing groups of the form Ic, a wide variety of methods can be used to couple the intermediate **4** with the sp³ nitrogen of the precursor to the group of form Ic. The indole **(10),** benzimidazole **(11),** benzpyrazole **(12),** or benzotriazole **(13)** precursor to the group of form Ic is treated with a strong base, and the compound is then alkylated with the intermediate **4.** Other procedures are given in the references T. L. Gilchrist, Heterocyclic chemistry, Longman, 2nd edition, 1992; E. V. Dehmlow, S. Dehmlow, Phase Transfer Catalysis, VCH, 2nd edition, 1993; J. A. Joule, K. Mills, G. F. Smith, Heterocyclic chemisty, 3rd edition, 1995.; Jonczyk, A., et al., Rocz. Chem., 1975 49:1203; Pielichowski, J. et al., J. Polym. Sci., Lett Ed., 1985 23:387; Pielichowski, J. et al., J. Prakt. Chem., 1989 311:145; Pielichowski, J. et al., Lieb. Ann. Chem., 1988, 579; Pielichowski, J. et al., Bull. Pol. Acad. Sci., Ser. Sci. Chem., 1989 37:123; Bogdal, D. et al., Synlett. (1996) 37:873; and Bogdal, D. et al., Heterocycles, (1997) 45:715-722.

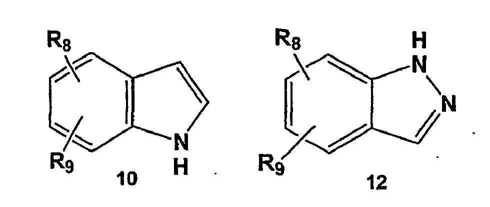

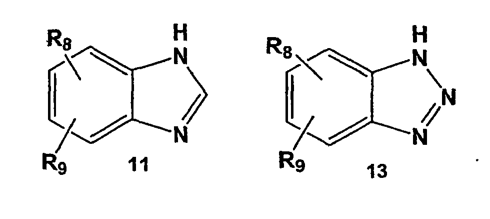

Halogen-containing 2-(2-bromomethyl-allyl) isoindole-1,3-dione derivatives (such as 2-(2-bromomethyl-3-fluoro-allyl)-isoindole-1,3-dione and 2-(2-bromomethyl-3-chloro-allyl)-isoindole-1,3-dione) are prepared according to the procedures in the following references: McDonald, I. A., Lacoste, J. M., Bey, P., Palfreyman, M. G., and Zreika, M., J. Med. Chem., 1985, 28, 186-193; McDonald, I. A., Bey, P., Tetrahedron Letters 1985, 26, 3807-3810; and McDonald, I.A., U.S. Patent No. 4,699,928. Specifically, for compounds of formula I where R₁ is F, the mixed ester tert-butyl 2-carbethoxypropionate (t-butyl ethyl 2-methylmalonate) **14** is prepared as described in U.S. Patent No. 4,699,928, starting from diethyl methylmalonate (Aldrich). The mixed ester is converted into the monosubstituted alkene compound **15** as described in McDonald, I. A., Lacoste, J. M., Bey, P., Palfreyman, M. G., and Zreika, M., J. Med. Chem., 1985, 28, 186-193; and McDonald, I. A., Bey, P., Tetrahedron Letters 1985, 26, 3807-3810. Briefly, the mixed ester **14** is treated with sodium t-butoxide, then with ClCHF₂; the t-butyl protecting group is removed with trifluoroacetic acid; and the compound is decarboxylated, followed by removal of HF, using sodium hydroxide to produce the fluoroalkene compound **15.** Dibal reduction of the ethyl ester to an alcohol, followed by the Mitsunobu reaction (Hughes, D. L., Org. Reac. (1992) 42:335-656; Hughes, D. L., Org. Prep. (1996) 28:127-164) with triphenyl phosphine, phthalimide, and diethyl azodicarboxylate (DEAD), followed by bromination of the methyl group with N-bromosuccinimide, yields the desired intermediates **16a** and **16b.** (The isomers are separated by methods known in the art, such as chromatographic or recrystallization techniques.)

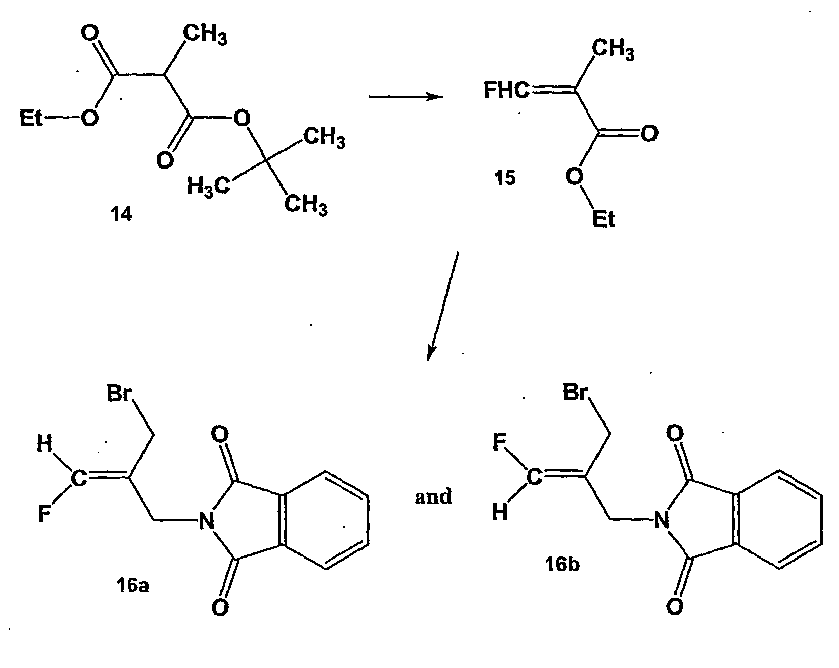

For compounds of formula I where R₁ is Cl, a compound of the form **17:** 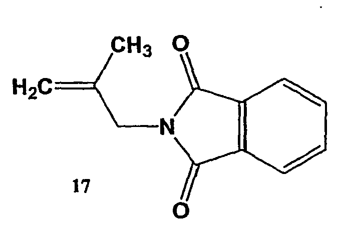 is used as the starting material.

For, e.g., the chloro-containing compound, it is converted into **18:** 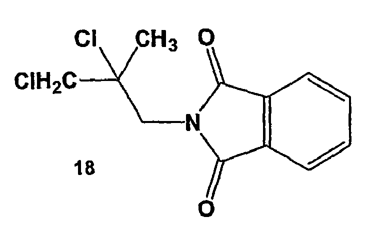 by reaction with Cl₂. Treatment with 1,8-diazabicyclo[5.4.0]undec-7-ene (DBU) yields **19a** and **19b:**

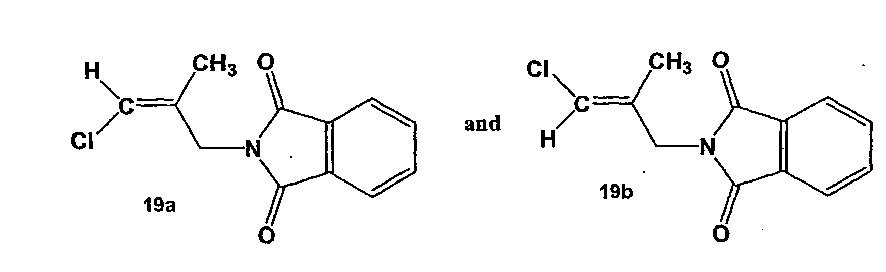

The methyl group of the compound can then be brominated as before with N-bromosuccinimide.

When R₁ is alkyl, the procedure for preparing the compounds of formula I outline above must be modified so as not to halogenate the R₁ alkyl group. An alternate method for these compounds is described here. 1-bromo-2,2-dimethoxypropane **20** (commercially available from Aldrich) is treated with potassium phthalimide in DMF at 90°C for 3 hrs. The product, 2-(2,2-Dimethoxy-propyl)-isoindole-1,3-dione **21,** is isolated by recrystallization from EtOAc/hexanes.

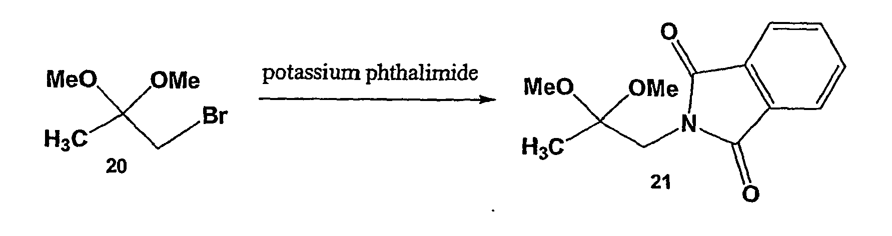

2-(2,2-Dimethoxy-propyl)-isoindole-1,3-dione **21** is treated with 50% TFA/CHCl₃/H₂O at 0°C for 2 hrs. 2-(2-Oxo-propyl)-isoindole-1,3-dione **22** is obtained.

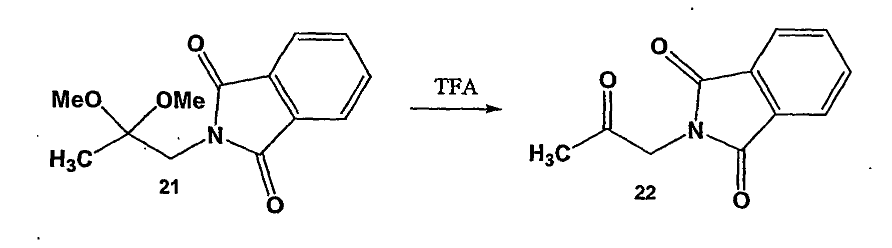

2-(2-Oxo-propyl)-isoindole-1,3-dione **22** is then reacted with Br₂ in CCl₄ to give 2-(3-Bromo-2-oxo-propyl)-isoindole-1,3-dione **23.**

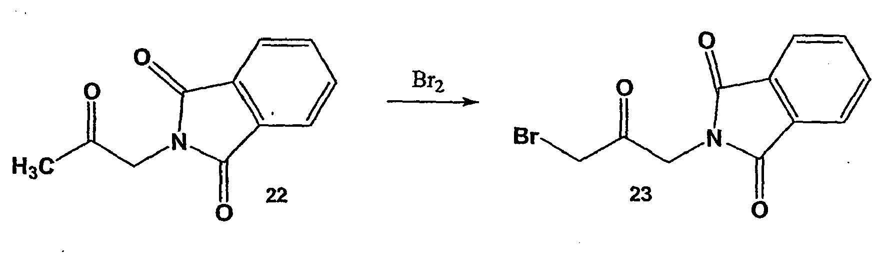

2-(3-Bromo-2-oxo-propyl)-isoindole-1,3-dione **23** is treated with ethylene glycol in the presence of p-toluenesulfonic acid (PTSA) in a reaction vessel equipped with a Dean-Stark trap at 110°C. The product, 2-(2-Bromomethyl-[1,3]dioxolan-2-ylmethyl)-isoindole-1,3-dione **24,** is obtained.

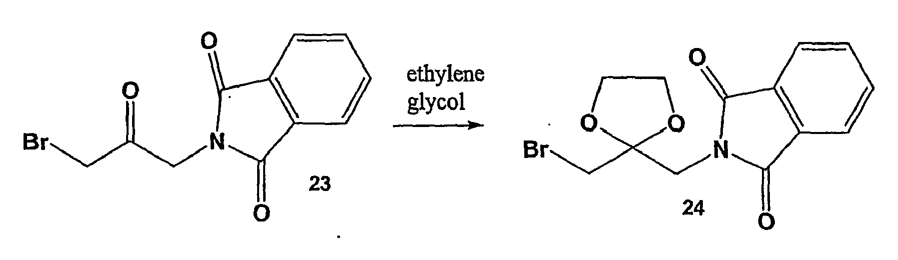

2-(2-Bromomethyl-[1,3]dioxolan-2-ylmethyl)-isoindole-1,3-dione **24** is then be reacted with amides corresponding to the precursor to the groups of form Ia or form Ib as follows:
To a suspension of NaH (1.1 eq) in DMF (30 ml) is added a solution of amide (1 eq) in DMF (5.0 ml). The resulting mixture is stirred at room temperature was 30 min. To this solution is added a solution of 2-(2-Bromomethyl-[1,3]dioxolan-2-ylmethyl)-isoindole-1,3-dione **24** (1.2 eq) in DMF (5.0 ml). The reaction mixture is stirred at room temperature under N₂ overnight, and then concentrated *in vacuo.* The residue is purified on column (silica gel, 20-40% EtOAc/hexanes) to give the corresponding alkylated amide. This reaction is illustrated below with an amide **25** corresponding to the precursor to the group of form Ia.

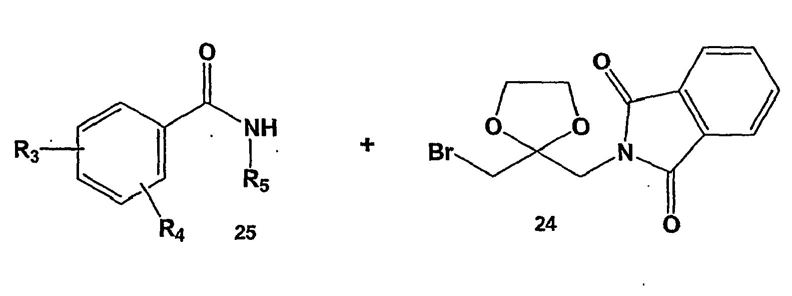

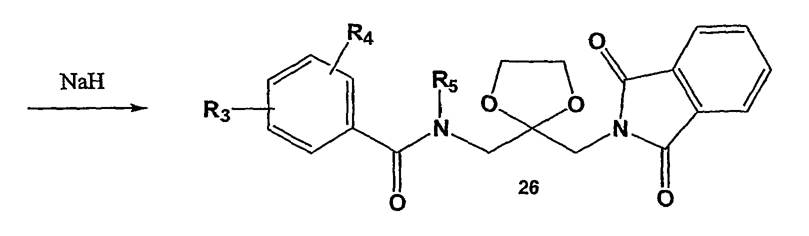

The alkylated amide **26** is treated with 5% HCl in THF to convert the ketal to the corresponding ketone 27.

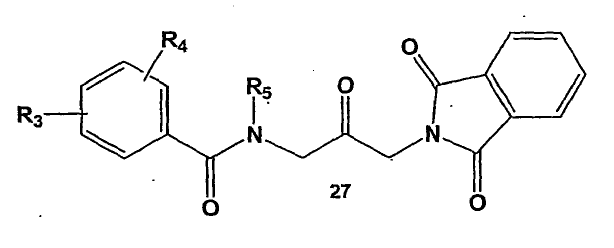

The ketone **27** is then reacted with a Wittig reagent of the form (C₆H₅)₃P=CH-R₁ to give the corresponding alkylated alkenes **28a** and **28b.** The isomers are separated by either chromatographic techniques (e.g., column chromatography) or recrystallization.

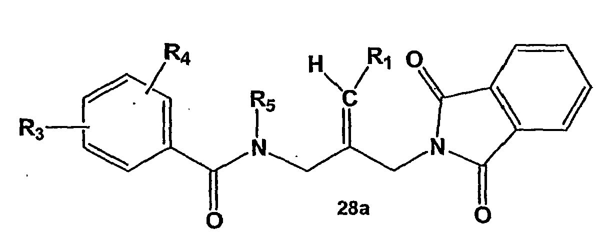

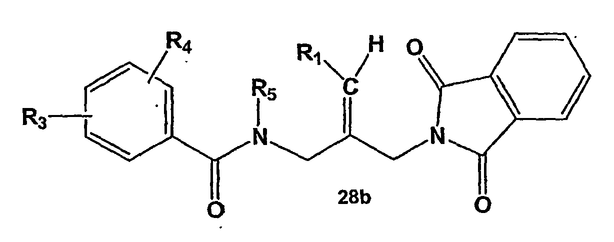

Finally, compounds of formula I **29a** and **29b** are obtained by removing the phthalimido group according to published procedures (e.g., by hydrazinolysis). 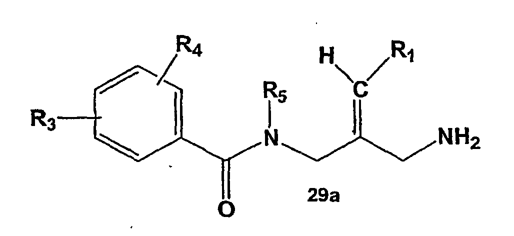 or

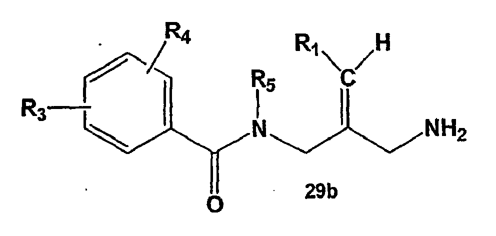

For compounds bearing groups of the form Id, the following procedure can be used.

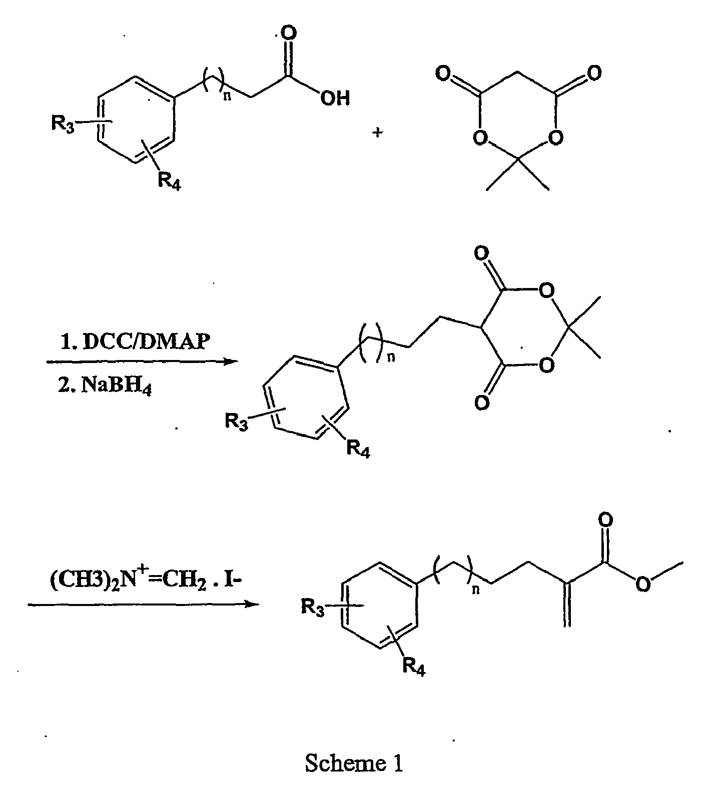

α-substituted acrylates can be prepared according to the synthetic route shown in Scheme 1 (J. Org. Chem. 2002, 67, 7365-7368). The resulting esters are then reduced to alcohols following oxidation with MnO₂ to provide corresponding α,β-unsaturated aldehydes (Scheme 2, Synthetic Communications, 2002, 32 (23), 3667-3674). The aldehydes are then reacted with Grignard reagents to give addition products.

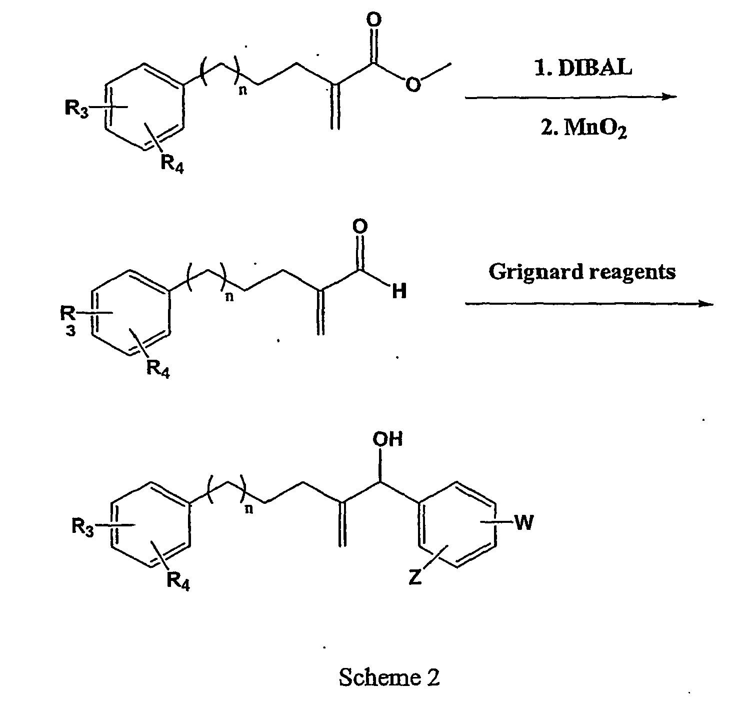

Subjecting the alcohols formed to the Mitsunobu reaction gives phthalimide derivatives. The N protecting group is removed by treating with hydrazine, followed by acidification to give the final compounds (as hydrochloride salts) (Scheme 3).

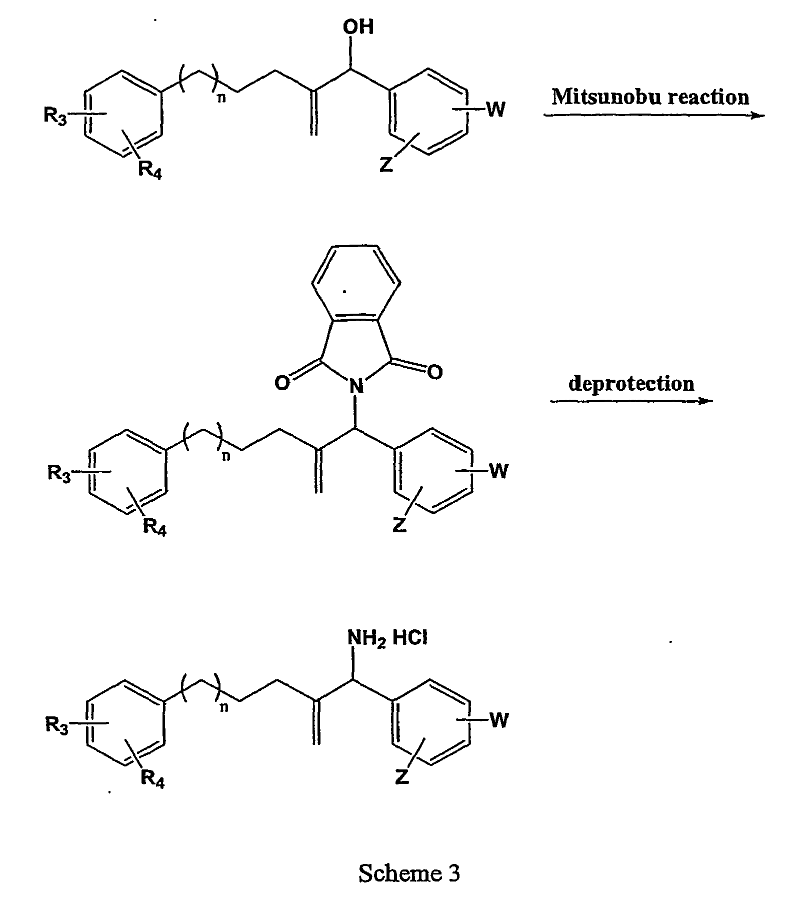

Removal of the phthalimido group is described in numerous publications; see, e.g., McDonald, I. A.; Lacoste, J. M.; Bey, P.; Palfreyman, M. G.; and Zreika, M. J. Med. Chem., 1985, 28, 186-193; or Bodansky, M; Bodansky, A., The Practice of Peptide Synthesis, Springer-Verlag: New York, 1984, at p. 163, "Removal of the Phthalyl (Phthaloyl) Group by Hydrazinolysis." Briefly, the phthalimido-protected compound is exposed to hydrazine hydrate. The phthalyl group is removed by refluxing for about 1 hour in a 1M solution of hydrazine hydrate in absolute ethanol, or by heating for about 2 hours in a 2M solution of hydrazine hydrate at 50°C. The residue is treated with 2N HCl at 50°C for 10 minutes, then left at room temperature for 30 minutes. The insoluble phthalylhydrazine is removed by filtration, and the filtrate is concentrated and purified by chromatography or recrystallization.

Synthesis of compounds of formula I-f can be performed as follows. A substituted or unsubstituted styrene is used as starting material. Reaction with sodium hydroxide and triethylbenzylammonium chloride in chloroform can be used to prepare a dichlorophenylcyclopropane:

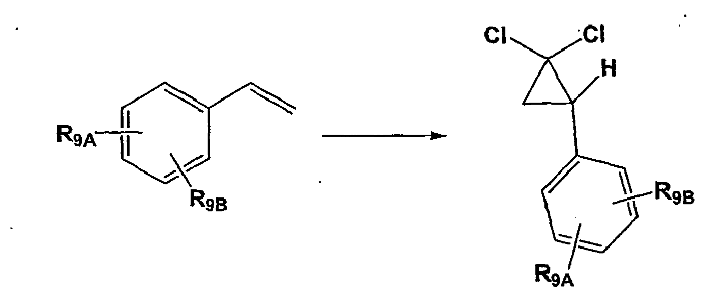

The dichlorophenyl cyclopropane is then refluxed with sodium hydroxide in ethanol to yield a diethoxyethylvinyl benzene:

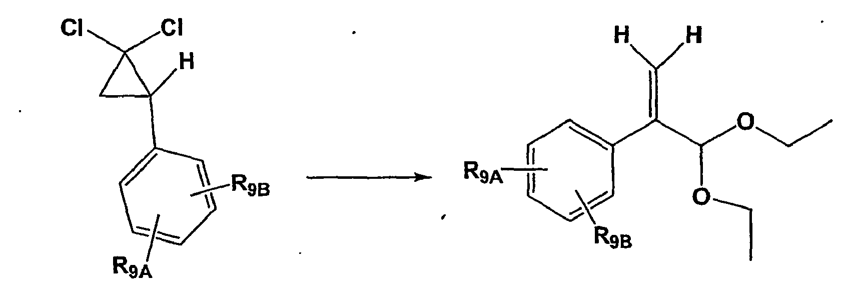

The diethoxylvinyl benzene is then treated with formic acid and water to yield an atropaldehyde (2-phenylpropenal):

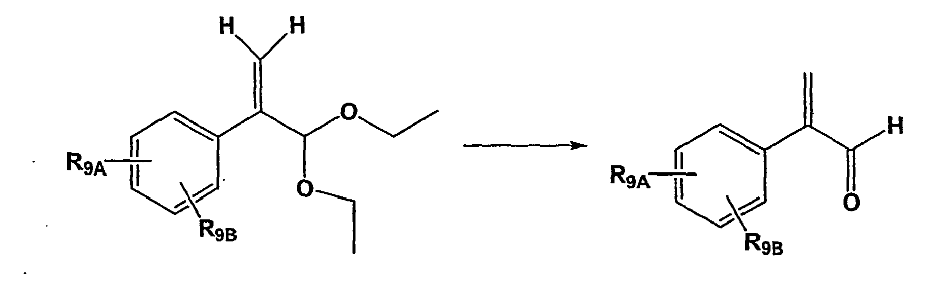

The atropaldehyde intermediate can then be derivatized at the aldehyde carbon to introduce a variety of R₆ substituents. For example, when R₆ is C₁-C₄ alkyl, designated as R_{6ALK}, the bromo compound of the form R_{6ALK}-Br can be used to form the Grignard reagent R_{6ALK}-MgBr to introduce R_{6ALK}:

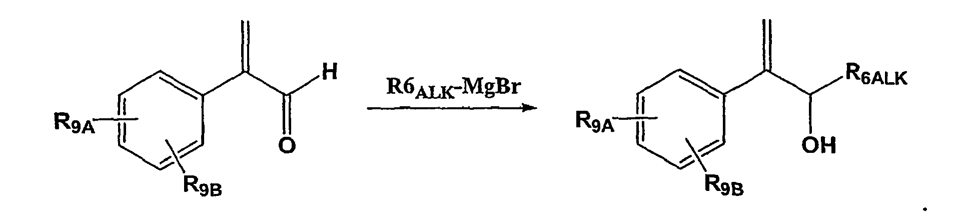

The resulting 2-phenyl-1-en-3-hydroxy alkyl compound can then be subjected to a Mitsunobu reaction to replace the hydroxy group with a nitrogen:

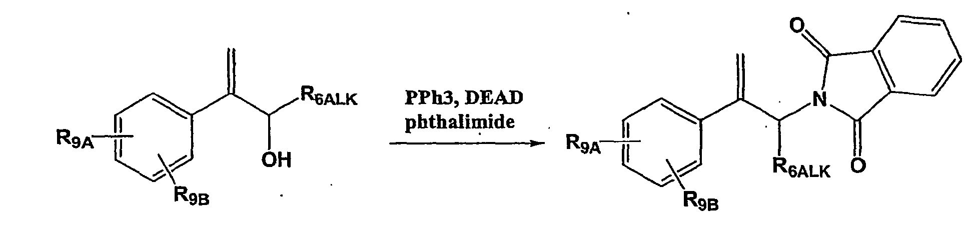

The phthalimide group can then be removed with hydrazine to yield the compound with R1= H:

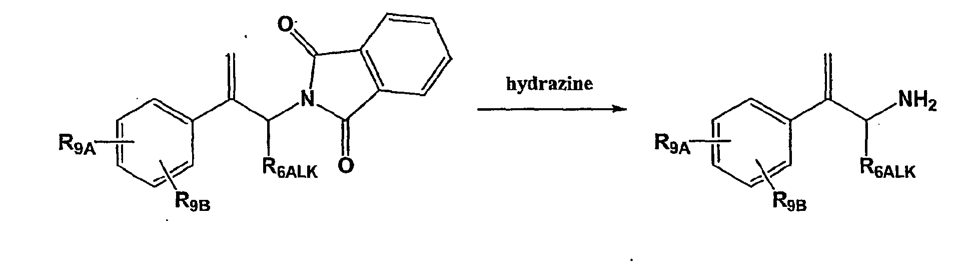

Alternatively, the alkene moiety can be chlorinated:

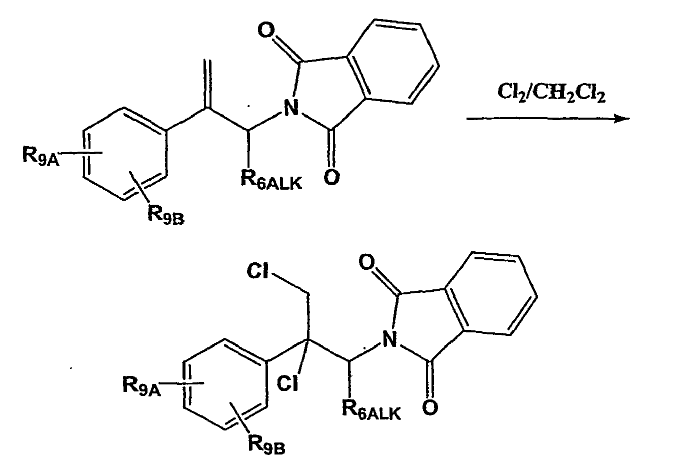

The dichloro can then be converted to the monochloro compounds by heating in dimethylsulfoxide with DBU (1,8-diazabicyclo[5.4.0]undec-7-ene), as described in McDonald, I. A., Lacoste, J. M., Bey, P., Palfreyman, M. G., and Zreika, M., J. Med. Chem., 1985, 28, 186-193 (see page 191, second column, syntheses of compounds 21 and 22), followed by deprotection (*ibid*.) to yield the desired compounds of formula I-f.

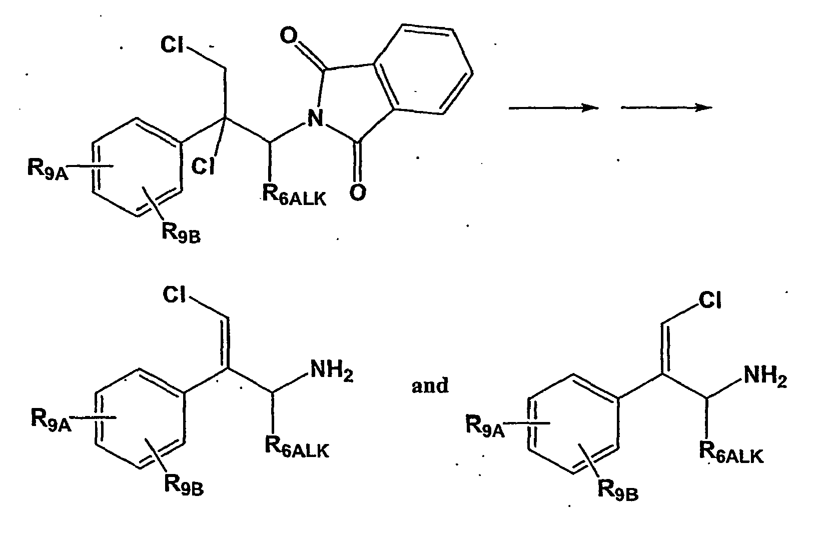

A specific example of the synthesis of a compound of formula 1-fis described in Example 1B.

### General procedures for the preparation of compounds of fonnula II

Compounds of formula II are prepared using compounds of the following structure as starting material: 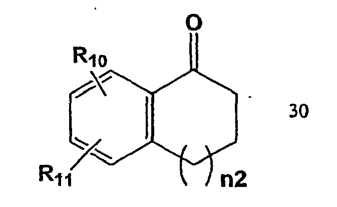 where n2 is 0, 1, or 2. Example 2 gives further details of the transformation of compounds of the form 30 into compounds of formula II. The starting materials embraced by **30** are widely available; for example, for n2=0; the compounds 1-indanone, 4-methyl-1-indanone, 6-methyl-1-indanone, 4-hydroxy-1-indanone, 5-hydroxy-1-indanone, 5-fluoro-1-indanone, 5,7-dimethyl-1-indanone, 5,6-dimethyl-1-indanone, 5-methoxy-1-indanone, 4-methoxy-1-indanone, 6-methoxy-1-indanone, 4-hydroxy-7-methyl-1-indanone, 5-chloro-1-indanone, 5,7-dimethoxy-1-indanone, 4,5-dimethoxy-1-indanone, 5,6-dimethoxy-1-indanone, 5-bromo-1-indanone, and 4-bromo-6,7-dimethoxy-1-indanone are commercially available from Aldrich Chemical Company (Sigma-Aldrich, St. Louis, Missouri). Syntheses of substituted 1-indanones (which syntheses either produce precursors of form **30,** or which can be modified to produce precursors of form **30**) are also described in U.S. Patent Nos. 4,016,281, 4,291,050, 5,329,049, 6,157,761, 6,492,539, and 6,548,710.

For n2=1, alpha-tetralone (3,4-dihydro-1(2H)-naphthalenone), 7-methyl-3,4-dihydro-1(2H)-naphthalenone, 6-methyl-3,4-dihydro-1(2H)-naphthalenone, 6-hydroxy-1-tetralone (6-hydroxy-3,4-dihydro-1(2H)-naphthalenone), 5-hydroxy-1-tetralone (5-hydroxy-3,4-dihydro-1(2H)-naphthalenone), 5,7-dimethyl-1-tetralone (5,7-dimethyl-3,4-dihydro-1 (2H)-naphthalenone), 6,7-dimethyl-3,4-dihydro-1 (2H)-naphthalenone, 5,8-dimethyl-3,4-dihydro-1 (2H)-naphthalenone, 7-methoxy-1-tetralone (7-methoxy-3,4-dihydro-1(2H)-naphthalenone), 5-methoxy-1-tetralone (5-methoxy-3,4-dihydro-1(2H)-naphthalenone), 6-methoxy-1-tetralone (6-methoxy-3,4-dihydro-1 (2H)-naphthalenone), 6-methoxy-5-methyl-3,4-dihydro-1(2H)-naphthalenone, 6,7-dimethoxy-1-tetralone (6,7-dimethoxy-3,4-dihydro-1(2H)-naphthalenone), and 5,8-dimethoxy-3,4-dihydro-1(2H)-naphthalenone are commercially available from Aldrich. Syntheses of substituted 1-tetralones (which syntheses either produce precursors of form **30,** or which can be modified to produce precursors of form **30**) are also described in U.S. Patent Nos. 3,833,726, 4,016,281, 4,603,221, 5,208,246, 6,157,761, and Australian Patent No. AU 527232 (corresponding to Australian Patent Application No. AU 56291/80).

For n2=2, 1-benzosuberone (6,7,8,9-tetrahydro-5H-benzo[a]cyclohepten-5-one), 8-fluoro-1-benzosuberone (3-fluoro-6,7,8,9-tetrahydro-5H-benzo[a]cyclohepten-5-one), 2-hydroxy-3-methoxy-6,7,8,9-tetrahydro-5H-benzo[a]cyclohepten-5-one, and 1,2-dimethoxy-6,7,8,9-tetrahydro-5H-benzo[a]cyclohepten-5-one are commercially available from Aldrich. Syntheses of substituted 1-benzosuberones (which syntheses either produce precursors of form **30,** or which can be modified to produce precursors of form **30**) are also described in U.S. Patent No. 6,157,761, Japanese Patent Publications 2000/007606 A and 2000/007607 A, and in Zhang et al., Synthetic Communications (1999), 29(16), 2903-2913.

### General procedures for the preparation of compounds of formula III

Compounds of formula III can be prepared by the following procedure. An ω-phenyl alkyl alcohol **37** is treated with CBr₄/PPh₃ in CH₂Cl₂. The bromide **38** can be isolated via column chromatography. The dry bromide **38** is then added to a mixture of magnesium metal in ether to prepare an ω-alkyl magnesium bromide **39.**

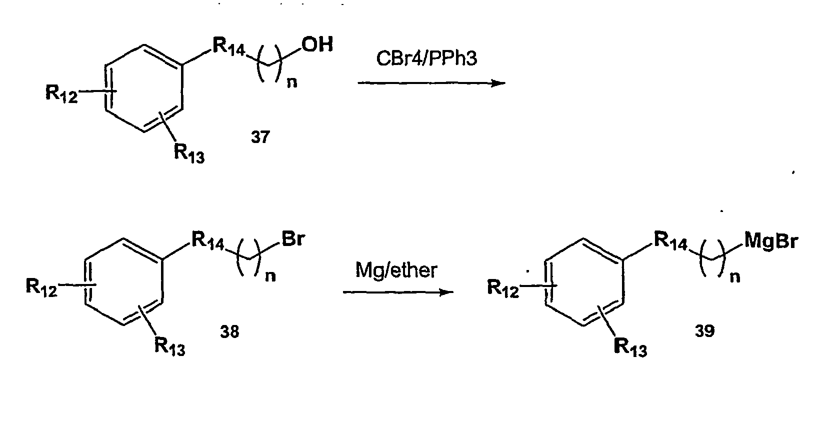

To generate compounds of formula III where the nitrogen containing ring is a 2,5-dihydro-1*H*-pyrrole ring (5-membered ring), the ω-alkyl magnesium bromide **39** is used directly in the next step to react with N-(carboethoxy)-3-pyrrolidone **40** (ethyl-3-oxo-1-pyrrolidinecarboxylate), with subsequent treatment of the product **41** with KOH to remove the carboethoxy group and then with hydrochloric acid to generate the 2,5-dihydro-1*H*-pyrrole group-containing compound **42** of formula III, as described in Lee, Y. et al., J. Am. Chem. Soc. (2002) 124:12135-12143.

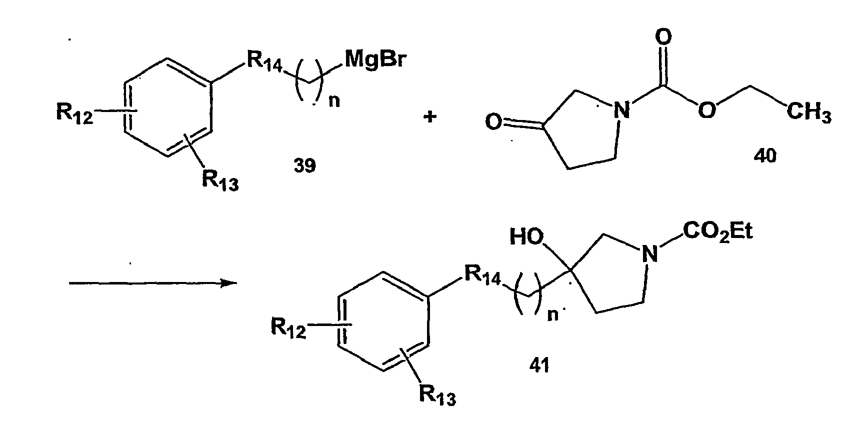

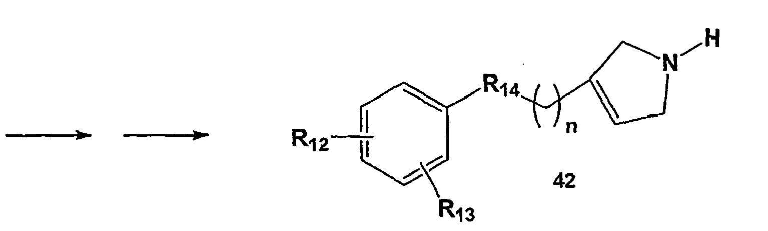

To generate compounds of formula III where the nitrogen containing ring is a 1,2,3,6-tetrahydropyridine ring (6-membered ring), the ω-alkyl magnesium bromide **39** is used directly in the next step to react with N-Boc-3-piperidinone **52** (3-oxo-piperidine-1-carboxylic acid tert-butyl ester), with subsequent treatment of the product **53** with trifluoroacetic acid to remove the Boc group and then with hydrochloric acid to generate the 1,2,3,6-tetrahydropyridine group-containing compound **54** of formula III, as described in Kehler, J. et al., Bioorg. & Med. Chem. Lett. (1999) 9, 811-814; de Costa, Dominguez, C. et al., J. Med. Chem. (1992), 35, 4334, and in similar fashion to Lee, Y. et al., J. Am. Chem. Soc. (2002) 124:12135-12143.

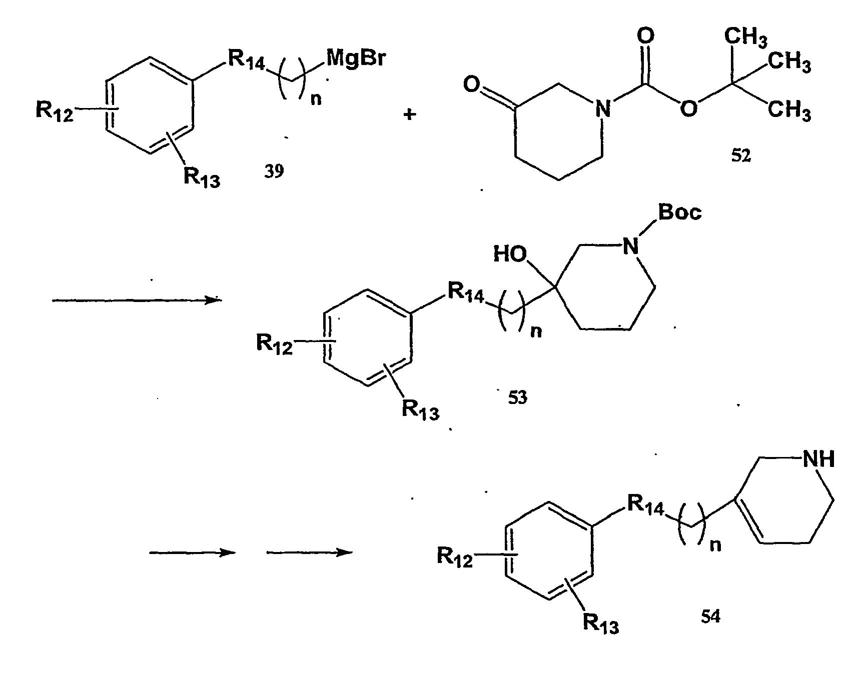

Examples of commercially available alcohols, which can be used to introduce R₁₂ and R₁₃ groups of the form -CH3, -CH3, and -Cl, are shown below.

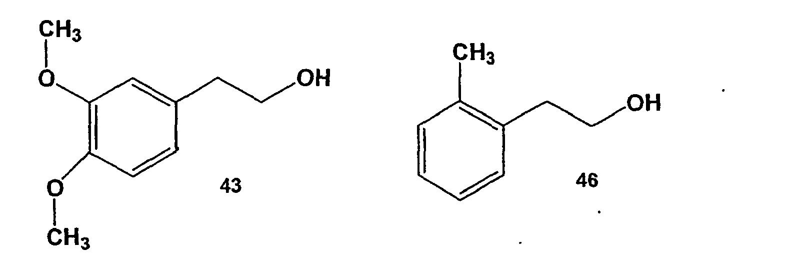

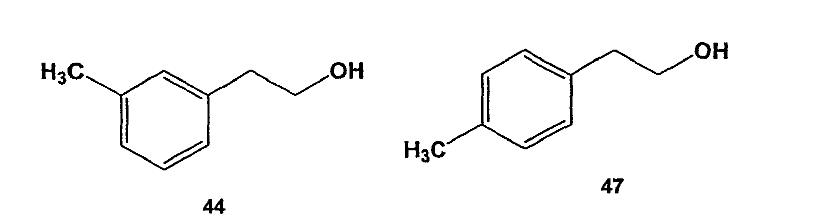

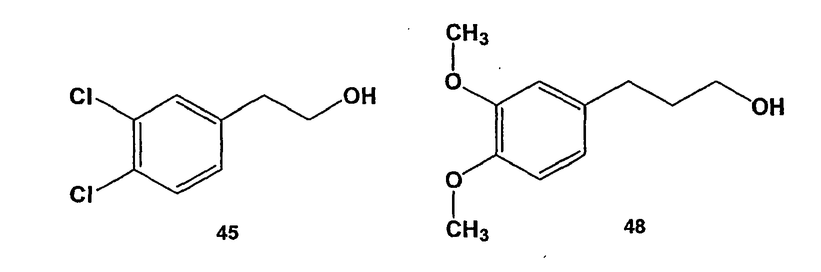

Other starting materials commercially available for synthesis include 2-phenoxyethanol, 2-(3-methylphenoxy)ethanol, 2-(4-methylphenoxy)ethanol, 2-(2-isopropylphenoxy)ethanol, 2-(4-methoxyphenoxy)ethanol, 2-(4-(tert-butyl)-phenoxy)-ethanol, α,4-dichloroanisole, 2-chloroethyl phenyl sulfide ([(2-chloroethyl)sulfanyl]benzene), and 1-chloro-4-[(2-chloroethyl)sulfanyl]benzene, which are available from Aldrich Chemical Company, St. Louis, Missouri.

Lee Y. et al., J. Am. Chem. Soc. (2002) 124, 12135-12143 describes a general chemical synthesis which can be adapted to prepare compounds of formula III (see Scheme 5 of Lee et al.). Wu, Y. et al., J. Med. Chem. (1962) 5:752-769 describes a general chemical synthesis for precursors to certain of the nitrogen heterocycle portion of the compounds of formula III.

Compounds of formula III where R₁₄ is CH₂ can also be prepared by the following procedures.

For n3b = 1, pyrrole is used as the starting material. The pyrrole nitrogen is protected, for example with the phenylsulfonyl group as follows.

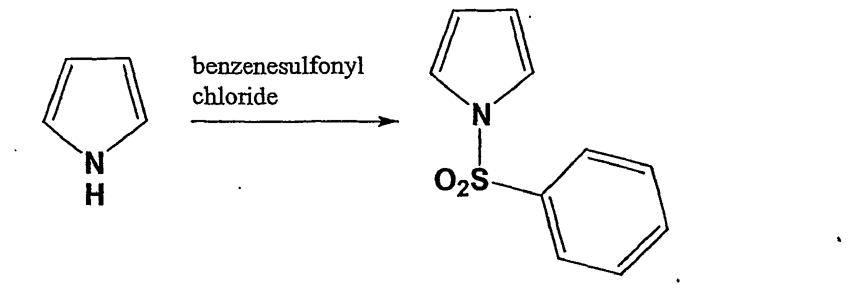

The 1-phenylsulfonylpyrrole is then reacted with substituted or unsubstituted phenylacetic acid (n3a = 1) or substituted or unsubstituted phenylpropionic acid (n3a = 2) which has been previously reacted with thionyl chloride, then aluminum chloride:

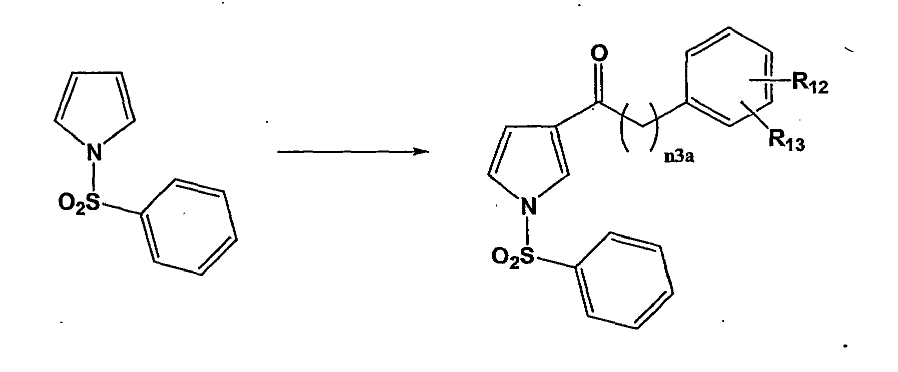

The carbonyl group is reduced and the pyrrole group is converted to a pyrroline group, e.g. by reaction with sodium cyanoborohydride in TFA:

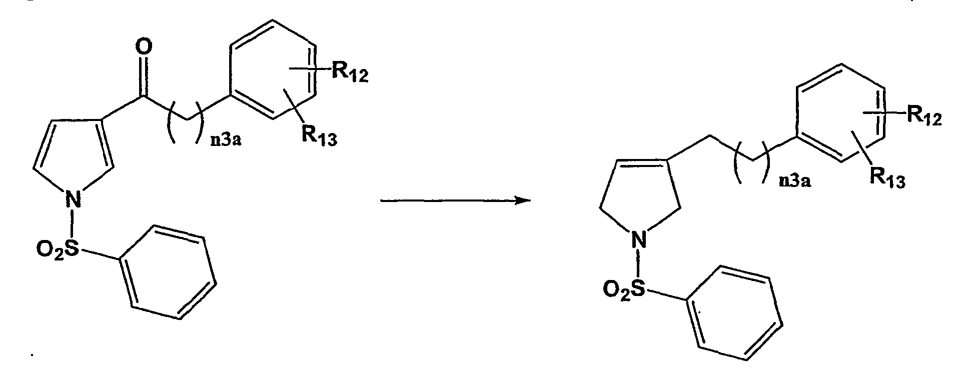

Finally, the phenylsulfonyl group is removed (for example, by reaction with sodium and anthracene in THF) to yield compounds of formula III where R₁₄ is CH₂:

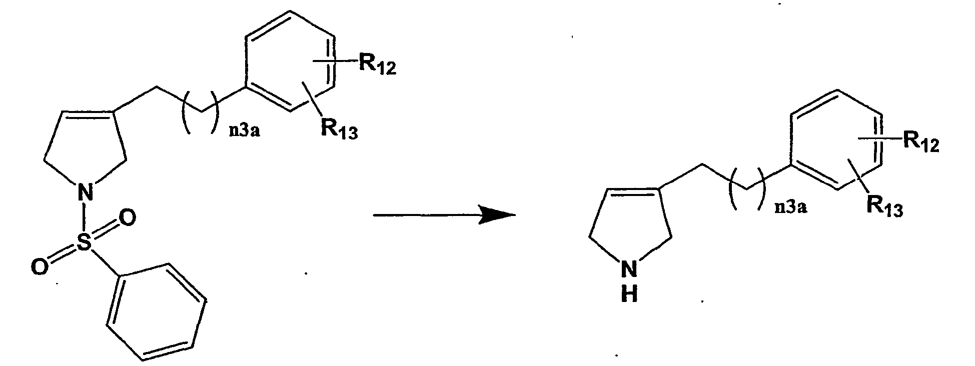

Examples of preparation of compounds of formula III by this method are given in Example 3I below.

For n3b = 2, the following procedure can be used when R₁₄ = O. The N-protected (e.g. carbobenzyloxy, Boc, etc.) ethyl ester of beta-alanine is reacted with the ethyl ester of acrylic acid to yield N-protected 4-oxo-piperidine carboxylic acid ethyl ester.

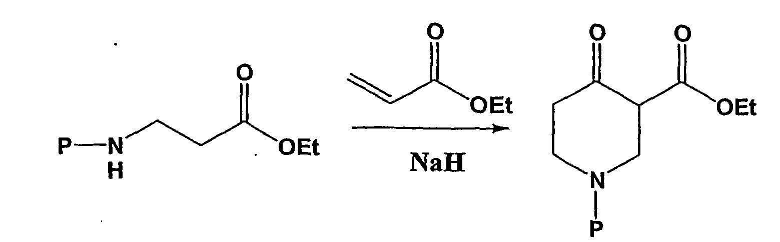

The 4-oxo group is then reduced, e.g., with sodium borohydride, to give the N-protected 4-hydroxypiperidine carboxylic acid ethyl ester.

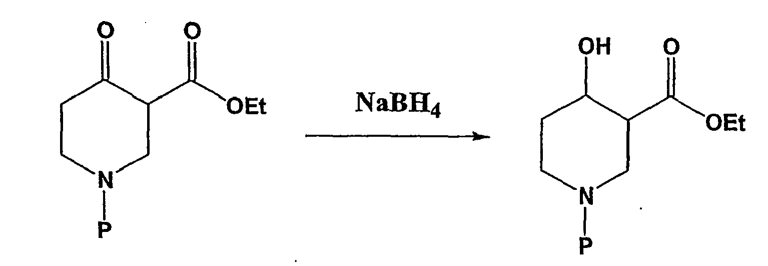

The alcohol is then activated and elimination occurs to give the 1,2,3,6-tetrahydropyridine-3-carboxylic acid ethyl ester derivative. Mesyl chloride/pyridine or tosyl chloride/pyridine, followed by basic treatment, can be used for this step.

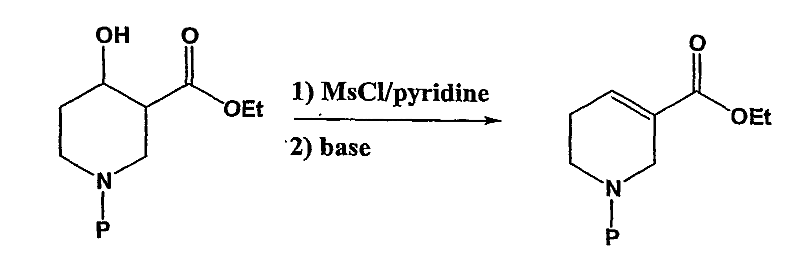

The ethyl ester is then reduced, for example, with lithium aluminum hydride:

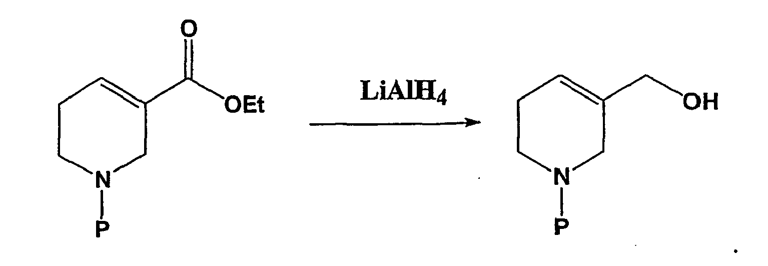

A phenolic compound of the general form: 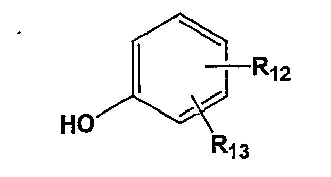 can then be coupled with the N-protected 3-hydroxymethyl-1,2,5,6-tetrahydropyridine, e.g. by using diisopropyl diazodicarboxylate and triphenylphosphine as follows:

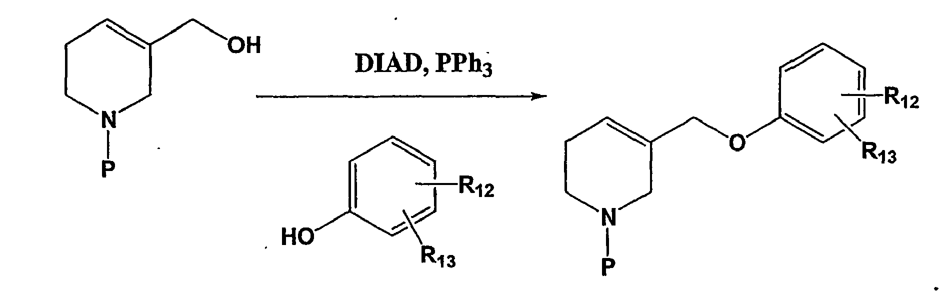

Finally, the N-protecting group is removed to yield the compounds of formula III where n3b = 2 and R₁₄ = O.

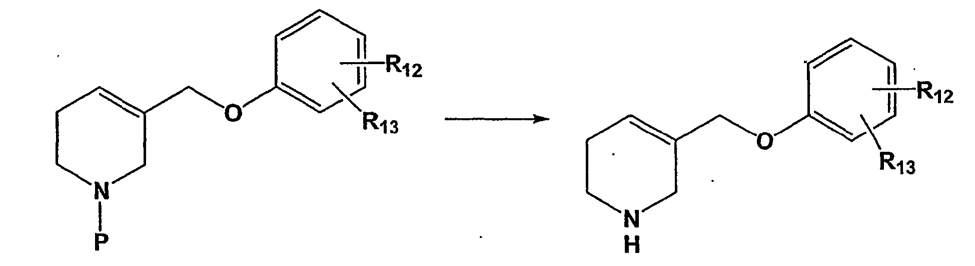

### General procedures for the preparation of compounds of formula IV

Phenyl trifluoromethyl ketone (2,2,2-trifluoroacetophenone, CF₃(C=O)C₆H₅) and benzyl trifluoromethyl ketone (1,1,1-trifluoro-3-phenyl-2-propanone, CF₃(C=O)CH₂C₆H₅) are commercially available (e.g., Aldrich Chemical Co., St. Louis, Missouri). The preparation of 1,1,1-trifluoro-4-phenyl-2-butanone (CF₃(C=O)CH₂CH₂C₆H₅) is described in US 5,238,598 and EP 282391. Other phenyl trifluoromethyl ketones, benzyl trifluoromethyl ketones, and related compounds can be prepared using the methods disclosed in EP 298478 and in the publications Kawase et al., International Journal of Antimicrobial Agents (2001), 18(2), 161-165, Kesavan et al., Tetrahedron Letters (2000), 41(18), 3327-3330, Linderman et al., Tetrahedron Letters (1987), 28(37), 4259-62, Creary, X., Journal of Organic Chemistry (1987), 52(22), 5026-30, and Herkes et al., Journal of Organic Chemistry (1967), 32(5), 1311-18. A Wittig reaction is used to transform the ketone into an acrylate ester, such as 3-phenyl-4,4,4-trifluoro-but-2-enoic acid ethyl ester or 3-benzyl-4,4,4-trifluoro-but-2-enoic acid ethyl ester. DIBAL or another suitable reducing agent is used to convert the ester into an alcohol, and then a Mitsunobu reaction is used to convert the alcohol into an amine. The amine is deprotected with hydrazine to yield the desired product.

A synthesis of a specific compound (3-benzyl-4,4,4-trifluoro-but-2-enylamine) embraced by formula IV is provided in Example 3.

### General procedures for the preparation of compounds of formula V

Compounds of formula V are synthesized by the following general procedure. Example 3A illustrates the synthesis of a specific compound (α-difluoromethylphenylalanine) embraced by general formula V.

The first two steps are adapted from the procedure for the synthesis of p-benzoyl-L-phenylalanine described in Kauer et al., J. Biol. Chem. 261(23):10695-700 (1986). The appropriate α-bromo-ω-phenyl alkyl starting material, of the form 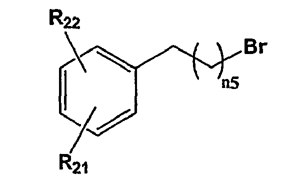 is reacted with the compound ethyl acetamidocyanoacetate, CH₃CONHCH(CN)CO₂C₂H₅ (Aldrich) under basic conditions to yield the protected intermediate

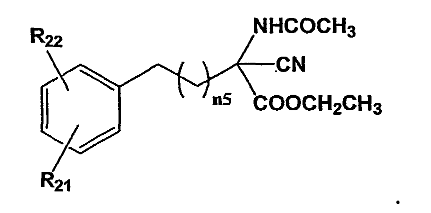

The protected intermediate is refluxed in aqueous HCl (mixtures of water, dioxane, and HCl can be used if necessary to solubilize the compound) to give the racemic mixture of

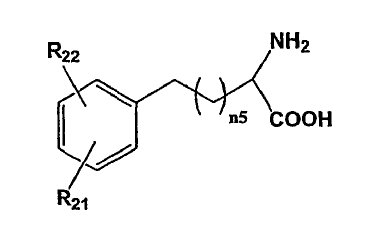

If desired, resolution of the enantiomers is readily achieved by acetylation of the racemic mixture, followed by enzymatic cleavage of the acetyl group from the L-amino acids by acylase enzyme. Separation of the acetylated D-amino acid from the unacetylated L-amino acid can be done using acid extraction of the L-amino acid; the acetyl group can then be removed from the D-amino acid by reflux in aqueous HCl or water/dioxane/HCl.

The amino acid is then esterified with an alcohol of the formula R₂₃OH by methods well-known in the art (see, e.g., Bodanszky and Bodanszky, The Practice of Peptide Synthesis, New York: Springer Verlag, 1984, at page 35, and other esterification procedures described therein), to give the compound

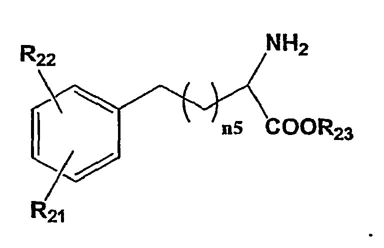

The N-formyl derivative is then formed by refluxing the amino acid ester with formamide.

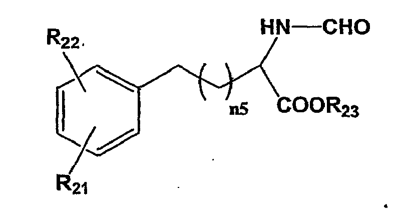

The N-formyl derivative is then reacted with phosphorus oxychloride in the presence of triethylamine in dichloromethane to form the isocyano intermediate

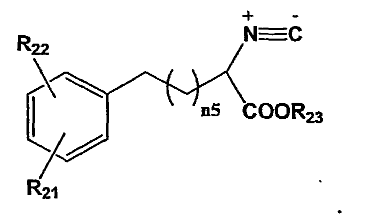

The isocyano intermediate is added to sodium hydride, followed by the addition of chlorodifluoromethane gas, to give a compound of the form

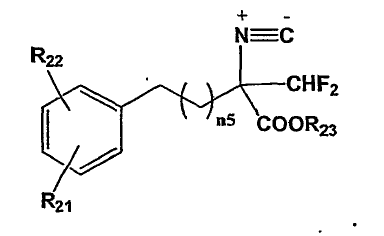

Finally, reflux in dry HCl (e.g., 2N HCl in ether) gives the α-difluoromethyl amino acid derivative

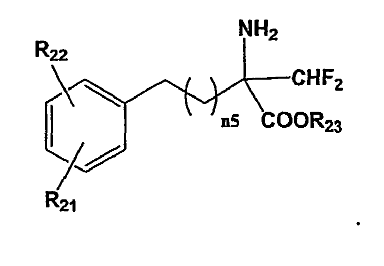

### General procedures for the preparation of compounds of fonnula VI

Unsubstituted, monosubstituted or disubstituted styrene oxide is reacted with a diamine compound as follows: 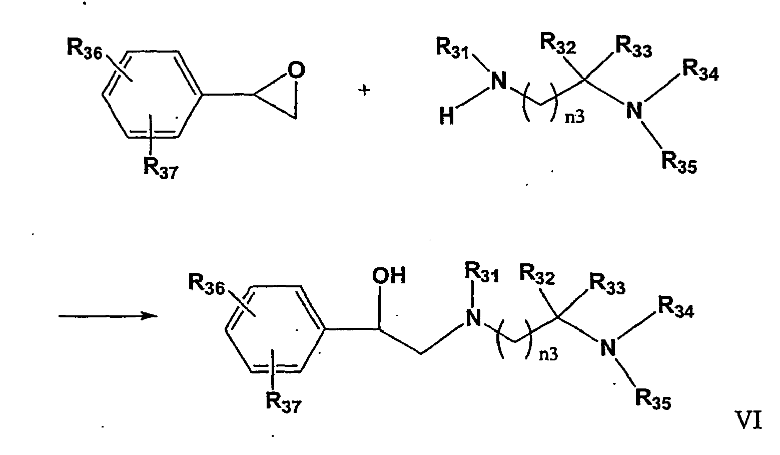 to yield compounds of formula VI.

When either R₃₄ and/or R₃₅ are hydrogen, the nitrogen bearing the R₃₄ and R₃₅ groups in the diamine reactant will often be sterically hindered by the R₃₂ and R₃₃ groups, and the nitrogen bearing the R₃₁ group will react with the styrene oxide to yield the desired product. If the nitrogen bearing the R₃₄ and R₃₅ groups is not sufficiently sterically hindered to ensure that only or primarily the nitrogen bearing the R₃₁ group reacts with the styrene oxide, then the nitrogen bearing the R₃₄ and R₃₅ groups can be protected with a suitable protecting group (e.g., t-Boc, carbobenzoxy, or other nitrogen protecting groups) to prevent reaction of the nitrogen bearing the R₃₄ and R₃₅ groups with styrene oxide. For example, in the diamine reactant, R₃₄ can be the t-Boc protecting group and R₃₅ can be a hydrogen atom. The Boc group can be removed in a later step to yield R₃₄ = H in the product.

Both S- and R-enantiomers of styrene oxide are commercially available, and a variety of substituted styrene oxides (phenyl-substituted 2-phenyloxiranes) are also commercially available. Substituted styrene oxides can also be synthesized by various methods; see, e.g., U.S. Patent Nos. 5,756,862 and 5,981,807. One convenient route to substituted styrene oxide is by epoxidation of substituted styrenes with a percarboxylic acid (e.g., peroxyacetic acid) as described in U.S. Patent No. 3,930,835. Substituted styrenes are widely available commercially.

The second reactant, an α-amino-di-ω-substituted-ω-amino alkane derivative, is also readily available commercially. Unsubstituted 1,2-diaminoethane, 1,3-diaminopropane,, 1,4-diaminobutane, etc., are well-known diamines. Substituted compounds, such as N,N-diethyl-1,3-propanediamine, are available from suppliers such as Aldrich Chemical Company. α-amino-di-ω-substituted-ω-amino alkane derivatives can also be prepared by procedures such as those described in U.S. Patent No. 4,902,831 or Japanese patent publication JP 8-27072 (published January 30, 1996).

A specific example of a synthesis of a compound (2-(2-Amino-2-methyl-propylamino)-1-phenyl-ethanol) falling within general formula VI is given below in Example 3B.

### General procedures for the preparation of compounds of formula VII

Compounds of formula VII can be prepared by the following procedures. Specific examples of syntheses of compounds falling within general formula VII are given below in Examples 3C, 3D, and 3E.

Typical syntheses use an ω-phenyl alkyl acid as the starting material. 3-phenyl propionic acid (hydrocinnamic acid) and 4-phenyl butyric acid are commercially available from Aldrich Chemical Company (St. Louis, Missouri). A variety of ω-phenyl alkanoic acids are available from other sources. An electrochemical synthesis of Ar(CH₂COOH) and Ar(CH₂CH₂COOH), where Ar is a substituted or unsubstituted aromatic group, is disclosed in U.S. Patent No. 4,517,061.

When R₇₃ is O or S, the compounds can be prepared from the reaction of a substituted phenol (R₇₃ = O) or thiophenol (phenyl mercaptan) (R₇₃ = S) and an ω-bromoaliphatic acid in the presence of NaOH or other base. The products, ω-phenoxyaliphatic acid or ω-phenylsulfanyl acid, are converted to their corresponding methyl esters. (Phenylthio)acetic acid (X=S) and phenoxylacetic acid (X=O) are also available from Aldrich. Additional compounds can be synthesized by reacting thiophenol (phenyl mercaptan) with an ω-bromo alkanoic acid (X=S) or by reacting phenol with an ω-bromo alkanoic acid (X=O) (a base can be used to drive the reaction of the thiolphenol or phenol with the ω-bromo alkanoic acid).

The ω-phenyl alkyl acid (or ω-phenoxyalkyl acid or ω-phenylsulfanylalkyl acid) is first converted to its corresponding methyl ester. The methyl ester can be reduced at -78°C with DIBAL to provide the corresponding aldehyde. The aldehyde is reacted with a Grignard reagent to give an alcohol. Mitsunobu conditions are used to convert the alcohol to a phthalimide derivative. The final compound is then obtained after removing the N protecting group.

### General procedures for the preparation of compounds of formula VIII

The following procedure, adapted from International Patent Application WO 02/38153, is used. A solution of histamine dihydrochloride (100 mmol), NaOH (250 mmol), and aldehyde R₈₀-CHO (250 mmol) in water (100 ml)/MeOH (450 ml) is refluxed for 24h, then cooled to room temperature. The reaction mixture is then cooled in an ice bath. To this cooled solution is added concentrated HCl solution to make the pH<1. The mixture is concentrated in vacuo. The residue is then dried in vacuo. The resulting oil is triturated with MeOH (3 x 50 ml) and filtered. The filtrate is concentrated in vacuo and dried under high vacuum. The residue is recrystallized from *i*-PrOH to give 4-substituted-4,5,6,7-tetrahydro-1H-imidazo[4,5-c]pyridine dihydrochloride. 2-substituted histamines can be used in order to introduce the functional group X as indicated in the general structure of formula VIII.

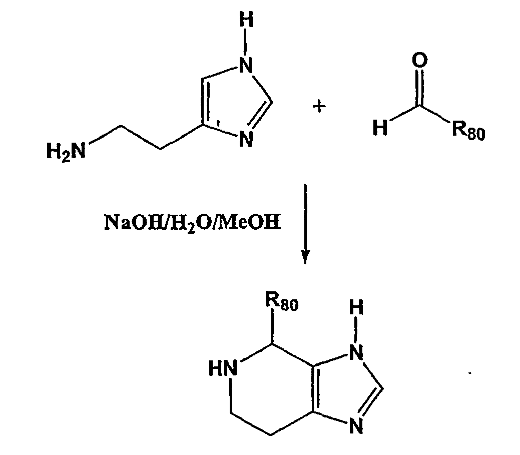

To a cooled solution of tetrahydroimidazopyridine dihydrochloride and K₂CO₃ in CH₂Cl₂/H₂O at 0°C is added dropwise a solution of acyl chloride in CH₂Cl₂. The resulting mixture is stirred for 24 h while warming up to room temperature. The mixture is transferred into a separation funnel. The layers are separated. The organic layer is dried (MgSO₄) and filtered. The filtrate is concentrated in vacuo. The residue is dissolved or suspended in MeOH (3ml/mmol of starting material) and aq. 1N NaOH (2 ml/mmol of starting material) is added. After 1h the mixture is acidified with aq. HCl. The final compounds are purified by either column chromatography (silica gel, 2-10% MeOH/CH₂Cl₂) or recrystallization from Et₂O.

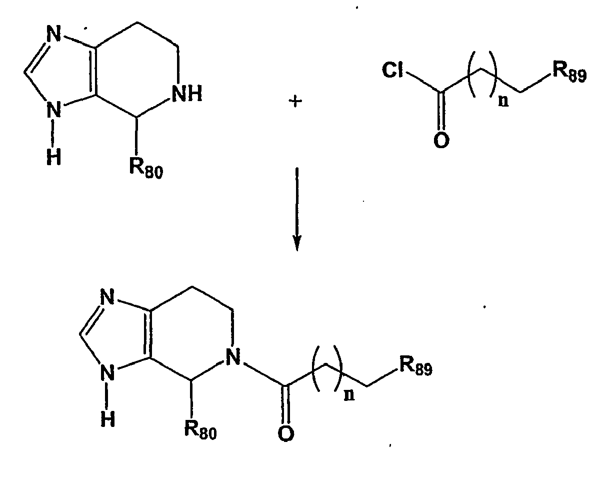

### General procedures for the preparation of compounds of formula IX

An aminomethyl-bearing derivative of the R₉₁ group, that is, of the form R₉₁-CH₂-NH₂, can be utilized as a starting material. Compounds such as 3-aminomethylpyridine (3-picolylamine), benzylamine, 3-methylbenzylamine, 3-methoxybenzylamine, (4-isopropylphenyl)methanamine, (2,4-dimethylphenyl)methanamine, 4-fluorobenzylamine, and 1-naphthylmethylamine are commercially available from suppliers such as Sigma-Aldrich. The R₉₂ group can also be present on the starting material (e.g., when R₉₁ = phenyl and n9 = 1, N-benzylmethylamine and N-benzyl-N-ethylamine can be used for R₉₂ = methyl and R₉₂ = ethyl, respectively). Alternatively, the R₉₂ group can be introduced easily by using a compound of the form R₉₂-Br or R₉₂-Cl for nucleophilic displacement by the NH₂ group of the R₉₁ fragment when R₉₂ is an alkyl, or by addition-elimination or elimination-addition reactions when R₉₂ is an aryl.

Once the appropriate compound of the form R₉₁-(CH₂)ₙ₉-N(R₉₂)H has been formed, the -C(=O)-CH(R₉₃)-N(R₉₄)(R₉₅) portion can be added easily by amide bond formation. Compounds of the form HO-C(=O)-CH(R₉₃)-N(R₉₄)(R₉₅) are alpha-amino acids, and an extraordinary variety of such compounds exist and are available from commercial suppliers (e.g, Bachem, Peninsula Laboratories, Sigma-Aldrich, SynPep (Dublin, CA), Calbiochem-Novabiochem, or are easily synthesized. The R₉₃ portion is the side chain of the amino acid, and both naturally-occurring and non-naturally-occurring side chains can be used as R₉₃.

Syntheses of compounds embraced by formula IX are given in Examples 3F and 3G below.

In one embodiment, the compounds of formula IX are chosen from the subset designated IX-a in the following scheme: 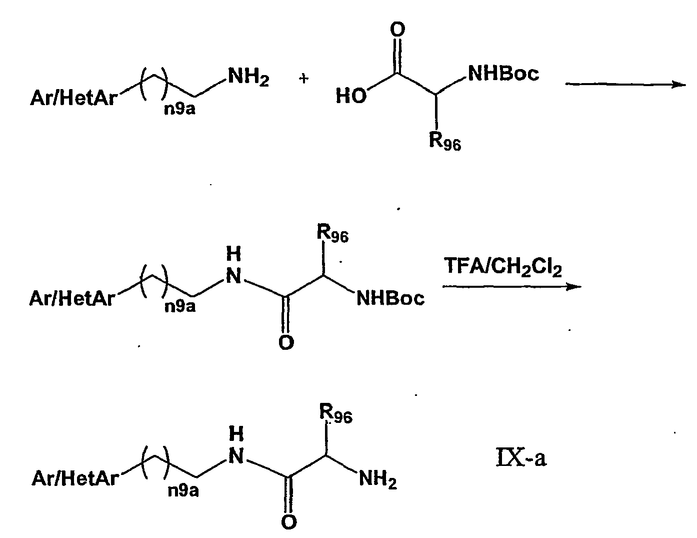 where Ar/HetAr is independently selected from aryl and heteroaryl; n9a is independently 0 or 1 (note that n9a = n9 - 1); and R₉₆ is independently selected from H and C₁-C₈ alkyl. The compounds of formula IX-a embrace the trifluoroacetate salt (which is produced using the scheme above), other salts, including pharmaceutically acceptable salts, and the free amine.

The synthesis of a compound embraced by formula IX-a is given in Example 3H below.

### Methods of Use

The compounds discussed herein can be used in a variety of manners. One such use is in treatment of inflammation, inflammatory diseases, inflammatory responses, and certain other diseases, as described in more detail below under "Treatment of Diseases." Other uses include inhibiting SSAO enzyme activity and/or VAP-1 binding activity or VAP-1 amine oxidase activity, both *in vivo* and *in vitro.* An example of *in vitro* use of the compounds is use in assays, such as conventional assays or high-throughput screening assays.

### Treatment of Diseases

Compounds discussed herein are useful for treating inflammation and inflammatory conditions, and for treating immune and autoimmune disorders. The compounds are also useful for treating one or more of a variety of diseases caused by or characterized by inflammation or immune disorders. Thus the compounds can be used to treat diseases caused by inflammation, and can also be used to treat diseases which cause inflammation. The compounds are used to treat mammals, preferably humans. "Treating" a disease with the compounds discussed herein is defined as administering one or more of the compounds discussed herein, with or without additional therapeutic agents, in order to prevent, reduce, or eliminate either the disease or one or more symptoms of the disease, or to retard the progression of the disease or of one or more symptoms of the disease, or to reduce the severity of the disease or of one or more symptoms of the disease. "Therapeutic use" of the compounds discussed herein is defined as using one or more of the compounds discussed herein to treat a disease, as defined above. A "therapeutically effective amount" of a compound is an amount of the compound, which, when administered to a subject, is sufficient to prevent, reduce, or eliminate either the disease or one or more symptoms of the disease, or to retard the progression of the disease or of one or more symptoms of the disease, or to reduce the severity of the disease or of one or more symptoms of the disease. A "therapeutically effective amount" can be given in one or more administrations.

The subjects which can be treated with the compounds and methods of the invention include vertebrates, preferably mammals, more preferably humans.

Diseases which can be treated with the compound and methods of the invention include inflammation, inflammatory responses, inflammatory diseases and immune disorders. It should be noted that inflammatory diseases can be caused by immune disorders, and that immune disorders are often accompanied by inflammation, and therefore both inflammation and immune disorders may be treated simultaneously by the compounds and methods of the invention. Diseases which can be treated with the compounds and methods of the invention include, but are not limited to, multiple sclerosis (including chronic multiple sclerosis); synovitis; systemic inflammatory sepsis; inflammatory bowel diseases; Crohn's disease; ulcerative colitis; Alzheimer's disease; atherosclerosis; rheumatoid arthritis; juvenile rheumatoid arthritis; pulmonary inflammatory conditions; asthma; skin inflammatory conditions and diseases; contact dermatitis; liver inflammatory and autoimmune conditions; autoimmune hepatitis; primary biliary cirrhosis; sclerosing cholangitis; autoimmune cholangitis; alcoholic liver disease; Type I diabetes and/or complications thereof; Type II diabetes and/or complications thereof; atherosclerosis; ischemic diseases such as stroke and/or complications thereof; and myocardial infarction. In another embodiment, the inflammatory disease or immune disorder to be treated by the present invention is multiple sclerosis. In another embodiment, the inflammatory disease or immune disorder to be treated by the present invention is chronic multiple sclerosis. In another embodiment, the inflammatory disease or immune disorder to be treated by the present invention is the inflammatory complications resulting from stroke.

### Modes of administration

The compounds described for use in the present invention can be administered to a mammalian, preferably human, subject via any route known in the art, including, but not limited to, those disclosed herein. Methods of administration include but are not limited to, intravenous, oral, intraarterial, intramuscular, topical, via inhalation (e.g. as mists or sprays), via nasal mucosa, subcutaneous, transdermal, intraperitoneal, gastrointestinal, rectal, and directly to a specific or affected organ. Oral administration is a preferred route of administration. The compounds described for use herein can be administered in the form of tablets, pills, powder mixtures, capsules, granules, injectables, creams, solutions, suppositories, enemas, colonic irrigation, emulsions, dispersions, food premixes, and in other suitable forms. The compounds can also be administered in liposome formulations. The compounds can also be administered as prodrugs, where the prodrug undergoes transformation in the treated subject to a form which is therapeutically effective. Additional methods of administration are known in the art.

The compounds of the present invention may be administered in an effective amount within the dosage range of about 0.1 µg/kg to about 300 mg/kg, or within about 1.0 µg/kg to about 40 mg/kg body weight, or within about 1.0 µg/kg to about 20 mg/kg body weight, preferably between about 1.0 µg/kg to about 10 mg/kg body weight. Other dosages which can be used are about 0.01 mg/kg body weight, about 0.1 mg/kg body weight, about 1 mg/kg body weight, about 10 mg/kg body weight, about 20 mg/kg body weight, about 30 mg/kg body weight, about 40 mg/kg body weight, or about 50 mg/kg body weight. Compounds of the present invention may be administered in a single daily dose, or the total daily dosage may be administered in divided dosage of two, three or four times daily.

The pharmaceutical dosage form which contains the compounds described herein is conveniently admixed with a non-toxic pharmaceutical organic carrier or a non-toxic pharmaceutical inorganic carrier. Typical pharmaceutically-acceptable carriers include, for example, mannitol, urea, dextrans, lactose, potato and maize starches, magnesium stearate, talc, vegetable oils, polyalkylene glycols, ethyl cellulose, poly(vinylpyrrolidone), calcium carbonate, ethyl oleate, isopropyl myristate, benzyl benzoate, sodium carbonate, gelatin, potassium carbonate, silicic acid, and other conventionally employed acceptable carriers. The pharmaceutical dosage form can also contain non-toxic auxiliary substances such as emulsifying, preserving, or wetting agents, and the like. A suitable carrier is one which does not cause an intolerable side effect, but which allows the compound(s) to retain its pharmacological activity in the body. Formulations for parenteral and nonparenteral drug delivery are known in the art and are set forth in Remington: The Science and Practice of Pharmacy, 20th Edition, Lippincott, Williams & Wilkins (2000). Solid forms, such as tablets, capsules and powders, can be fabricated using conventional tableting and capsule-filling machinery, which is well known in the art. Solid dosage forms, including tablets and capsules for oral administration in unit dose presentation form, can contain any number of additional non-active ingredients known to the art, including such conventional additives as excipients; desiccants; colorants; binding agents, for example syrup, acacia, gelatin, sorbitol, tragacanth, or polyvinylpyrollidone; fillers, for example lactose, sugar, maize-starch, calcium phosphate, sorbitol or glycine; tableting lubricants, for example magnesium stearate, talc, polyethylene glycol or silica; disintegrants, for example potato starch; or acceptable wetting agents such as sodium lauryl sulfate. The tablets can be coated according to methods well known in standard pharmaceutical practice. Liquid forms for ingestion can be formulated using known liquid carriers, including aqueous and nonaqueous carriers such as sterile water, sterile saline, suspensions, oil-in-water and/or water-in-oil emulsions, and the like. Liquid formulations can also contain any number of additional non-active ingredients, including colorants, fragrance, flavorings, viscosity modifiers, preservatives, stabilizers, and the like. For parenteral administration, the compounds for use in the invention can be administered as injectable dosages of a solution or suspension of the compound in a physiologically acceptable diluent or sterile liquid carrier such as water, saline, or oil, with or without additional surfactants or adjuvants. An illustrative list of carrier oils would include animal and vegetable oils (e.g., peanut oil, soy bean oil), petroleum-derived oils (e.g., mineral oil), and synthetic oils. In general, for injectable unit doses, sterile liquids such as water, saline, aqueous dextrose and related sugar solutions, and ethanol and glycol solutions such as propylene glycol or polyethylene glycol are preferred liquid carriers.

The pharmaceutical unit dosage chosen is preferably fabricated and administered to provide a defined final concentration of drug in the blood, tissues, organs, or other targeted region of the body. The optimal effective concentration of the compounds of the invention can be determined empirically and will depend on the type and severity of the disease, route of administration, disease progression and health, mass and body area of the patient. Such determinations are within the skill of one in the art. The compounds for use in the invention can be administered as the sole active ingredient, or can be administered in combination with another active ingredient.

### Kits

The invention also provides articles of manufacture and kits containing materials useful for treating diseases such as inflammatory diseases, autoimmune diseases, multiple sclerosis (including chronic multiple sclerosis); synovitis; systemic inflammatory sepsis; inflammatory bowel diseases; Crohn's disease; ulcerative colitis; Alzheimer's disease; atherosclerosis; rheumatoid arthritis; juvenile rheumatoid arthritis; pulmonary inflammatory conditions; asthma; skin inflammatory conditions and diseases; contact dermatitis; liver inflammatory and autoimmune conditions; autoimmune hepatitis; primary biliary cirrhosis; sclerosing cholangitis; autoimmune cholangitis; alcoholic liver disease; Type I diabetes and/or complications thereof; Type II diabetes and/or complications thereof; atherosclerosis; ischemic diseases such as stroke and/or complications thereof; and myocardial infarction; or for inhibiting SSAO enzyme activity (whether the enzyme activity is due either to soluble SSAO enzyme or membrane-bound VAP-1 protein, or due to both) and/or inhibiting binding to VAP-1 protein. The article of manufacture comprises a container with a label. Suitable containers include, for example, bottles, vials, and test tubes. The containers may be formed from a variety of materials such as glass or plastic. The container holds a composition having an active agent which is effective for treating diseases or for inhibiting SSAO or VAP-1 enzyme activity or binding to VAP-1 protein. The active agent in the composition is one or more of the compounds of formulas I, II, III, IV, V, VI, VII, VIII, IX, and/or X. The label on the container indicates that the composition is used for treating diseases such as inflammatory or autoimmune diseases, or for inhibiting SSAO or VAP-1 enzyme activity or binding to VAP-1 protein, and may also indicate directions for either *in vivo* or *in vitro* use, such as those described above.

The invention also provides kits comprising any one or more of the compounds of formulas I, II, III, IV, V, VI, VII, VIII, IX, and/or X. In some embodiments, the kit of the invention comprises the container described above. In other embodiments, the kit of the invention comprises the container described above and a second container comprising a buffer. It may further include other materials desirable from a commercial and user standpoint, including other buffers, diluents, filters, needles, syringes, and package inserts with instructions for performing any methods described herein (such as methods for treating autoimmune or inflammatory diseases, and methods for inhibiting SSAO or VAP-1 enzyme activity or binding to VAP-1 protein).

In other aspects, the kits may be used for any of the methods described herein, including, for example, to treat an individual with autoimmune or inflammatory disease, such as multiple sclerosis or ischemic disease (such as stroke) and the sequelae thereof.

The invention will be further understood by the following nonlimiting examples.

### EXAMPLES

### Example 1

### Synthesis of a precursor of the compounds of general formula I

A mixture of 3-bromo-2-methyl-propene (2) (1.86 ml, 17.9 mmol) and potassium phthalimide (1) (3.32 g, 17.9 mmol) in DMF (40 ml) was heated at 90°C for 4 hrs, then cooled and diluted with H₂O (40 ml). The resulting mixture was extracted with EtOAc (2 x 30 ml). The combined organic layers were washed with brine (30 ml), dried (MgSO4), and filtered. The filtrate was concentrated *in vacuo* to give 2-(2-methyl-allyl)-isoindole-1,3-dione (3) as a solid (2.6 g, 72%). ¹H NMR (CDCl₃, 300 MHz) δ 1.78 (s, 3H), 4.25 (s, 2H), 4.85 (s, 1H), 4.92 (s, 1H), 7.73-7.79 (m, 2H), 7.87-7.92 (m, 2H).

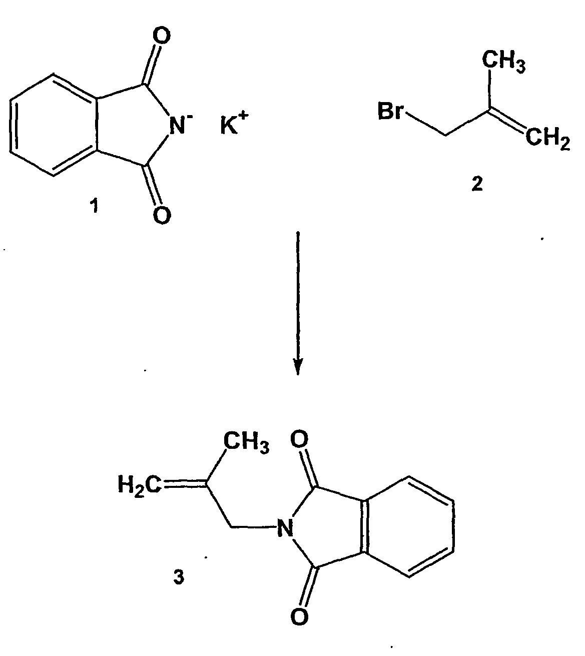

A mixture of 2-(2-methyl-allyl)-isoindole-1,3-dione (3) (1.0 g, 4.97 mmol) and N-bromosuccinimide (NBS) (1.13 g, 4.97 mmol) in CCl₄ (40 ml) is refluxed for 4 hrs. The mixture is filtered. The filtrate is concentrated *in vacuo.* The residue is purified on column (silica gel, 2-10 % EtOAc/hexanes) to obtain 2-(2-bromomethyl-allyl)-isoindole-1,3-dione (4).

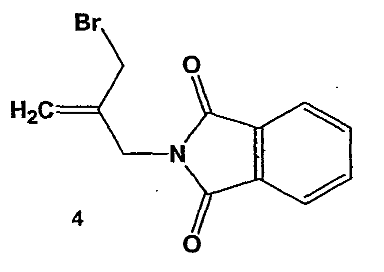

### Example 1A

### General procedure for use of 2-(2-bromomethyl-allyl)-isoindole-1,3-dione (4) for the synthesis of compounds of formula I

To a suspension of NaH (1.1 eq) in DMF (30 ml) is added a solution of an amide corresponding to the precursor to the groups of form Ia or Ib (1 eq) in DMF (5.0 ml) or an indole, benzimidazole, benzpyrazole, or benzotriazole corresponding to the group of form Ic. (A different procedure, which does not proceed via 2-(2-bromomethyl-allyl)-isoindole-1,3-dione, is used to synthesize compounds with a group corresponding to formula Id; see the section above entitled "General procedures for the preparation of compounds of formula I"). The resulting mixture is stirred at room temperature for 30 min. To this solution is added a solution of 2-(2-Bromomethyl-allyl)-isoindole-1,3-dione (1.2 eq) in DMF (5.0 ml). The reaction mixture is stirred at room temperature under N₂ for overnight, and then concentrated *in vacuo.* The residue is purified on column (silica gel, 20-40% EtOAc/hexanes) to give the corresponding alkylated amide.
This reaction is illustrated for a compound with a group of the form Ia: 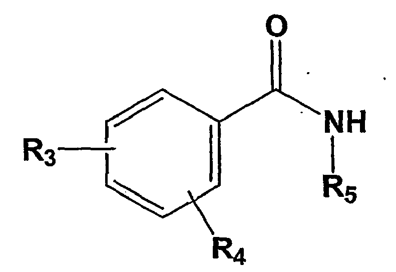 which reacts with 4 to yield 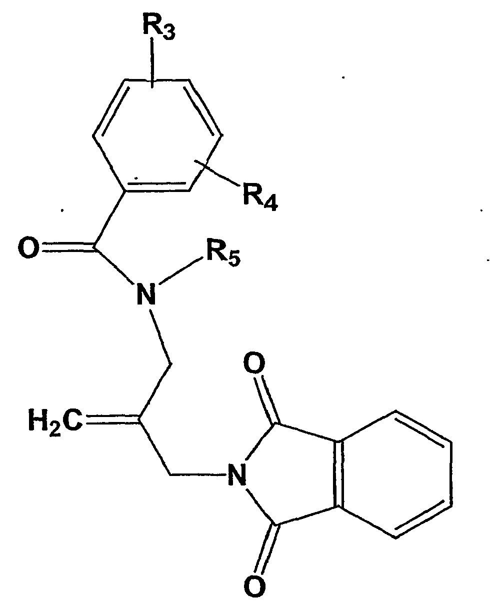 The phthalimido group is then removed by published procedures, for example by exposure to hydrazine hydrate (e.g., 1 hour in 1M hydrazine hydrate in absolute ethanol). The resulting compound is of the following form.

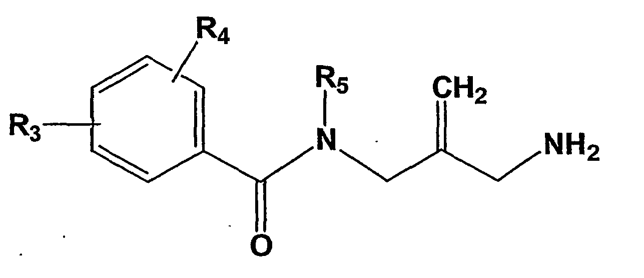

### Example 1B

### Synthesis of compounds of formula I-f

### Synthesis of 1,1-dichloro-2-phenylcyclopropane (Ex1b-2):

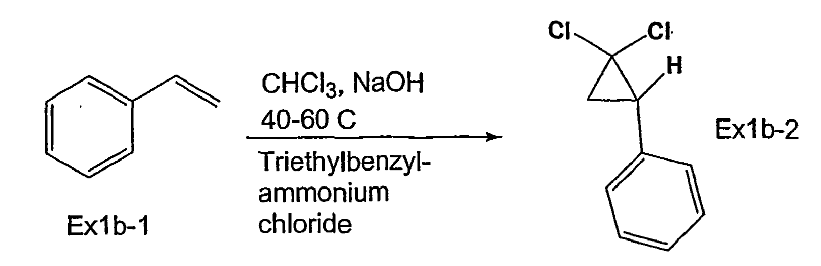

In a three-necked, round-bottomed flask equipped with a magnetic stirrer, a thermometer, and a reflux condenser was placed styrene (Ex1b-1) (14.3 mL, 125 mmol), chloroform (12.5 mL), triethylbenzylammonium chloride (0.5 g, 2.20 mmol), methylene chloride (6.5 mL), and a solution of sodium hydroxide (19.2 g) in water (19.2 mL). The mixture was stirred vigorously. The reaction temperature was allowed to rise to 40°C, and then kept around 40-45°C. After an hour, the reaction mixture was heated in a 55-60°C oil bath for another hour. Then the reaction mixture was poured into H₂O (30 mL) and extracted with petroleum ether (2 x 30 mL). The combined organic layers were dried over sodium sulfate and concentrated in vacuo to give an oil. This oil was distilled through a 20-cm Vigreux column, The fraction at 105°C (17 mmHg) was collected (15.5 g, 66.5%). ¹H NMR (CDCl₃, 300 MHz) δ 1.87 (dd, *J* = 7.5, 8.7 Hz, 1H), 1.98 (dd, *J* = 7.5, 10.5 Hz, 1H), 2.94 (dd, *J*= 8.7, 10.5 Hz, 1H), 7.3-7.45 (m, 5H).

### Synthesis of atropaldehyde diethyl acetal (Ex1b-3):

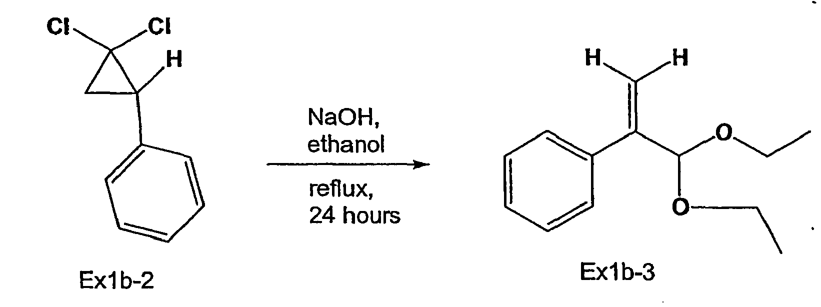

A mixture of 1,1-dichloro-2-phenylcyclopropane (15.5g, 82.8 mmol) and sodium hydroxide (13.2 g, 4 eq) in ethanol (130 mL) was refluxed for 24 hours. The reaction was poured into H₂O (30 mL) and extracted with petroleum ether (2 x 30 mL). The combined organic layers were dried (Na₂SO₄) and filtered. The filtrate was concentrated in vacuo to give an oil. The oil was distilled through a 20-cm Vigreux column to give the pure product (11.4 g, 67%). ¹H NMR (CDCl₃, 300 MHz) δ 1.21 (t, J = 6.9 Hz, 6H), 3.55 (m, 2H), 3.65 (m, 2H), 5.24 (s, 1H), 5.66 (m, 2H), 7.30 (m, 3H), 7.54 (m, 2H).

### Synthesis of atropaldehyde (Ex1b-4):

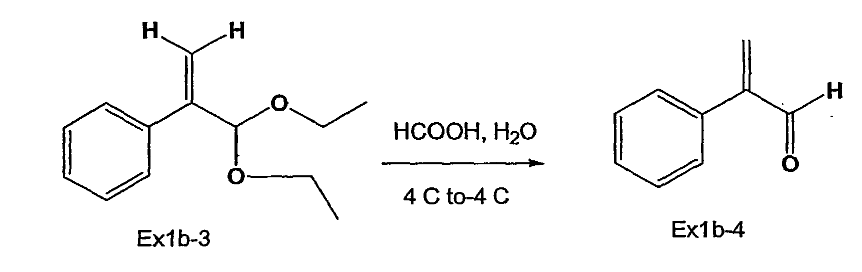

To cooled atropaldehyde diethyl acetal (9.98 g, 48.4 mmol) was added a cooled mixture of formic acid (12mL) and water (4mL) in one portion. The reaction was monitored by TLC after 10 min, and was quickly quenched by adding petroleum ether and water. The mixture was transferred to a separatory funnel and more petroleum ether and water were added as needed. The aqueous layer was washed with petroleum ether (2 x 30 mL). Then the combined organic layers were dried over sodium sulfate and concentrated in vacuo to give an oil. This oily product was dissolved in a mixture of diethyl ether (7 mL) and petroleum ether (7 mL). The resulting solution was cooled to -50°C. A crystalline solid was formed. The solid was filtered, dried under vacuum for a few minutes and then was stored at -20°C (4.63 g, 72.4%). ¹H NMR (CDCl₃, 300 MHz) δ 6.19 (d, *J*= 0.6 Hz, 1H), 6.64 (d, *J*= 0.6 Hz, 1H), 7.35-7.5 (m, 5H), 9.84 (s, 1H).

Note that this synthesis of compound Ex1b-4 is described in Organic Syntheses Collective Volume 7, page 12 and Annual Volume 60, page 6.

### Synthesis of compound Ex1b-5:

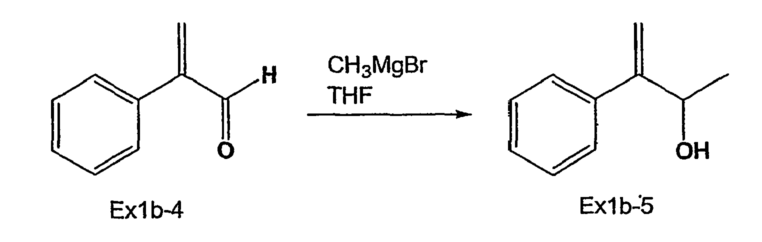

To a cooled solution of atropaldehyde (2.60 g, 19.7 mmol) in THF (16 mL) at 0°C was added dropwise methyl magnesium bromide (14.8 mL, 20.6 mmol). The reaction mixture was stirred at room temperature for 1.5 hrs, then quenched by adding H₂O (30 mL). The layers were separated. The aqueous layer was extracted with CH₂Cl₂ (2 x 30 mL). The combined organic layers were dried (Na₂SO₄) and filtered. The filtrated was concentrated in vacuo. The residue was purified on column chromatography (silica gel, 10-25% EtOAc/hexanes) to give an oil (2.98 g, 100%). ¹H NMR (CDCl₃, 300 MHz) δ 1.33 (d, *J* = 6.3 Hz, 3H), 1.66 (s, 1H), 4.84 (q, *J* = 5.7 Hz, 1H), 5.28 (d, *J*= 0.9 Hz, 1H), 5.37 (d, *J* = 1.2 Hz, 1H), 7.35-7.45 (m, 5H).

*Synthesis of compound Ex1b-6:* 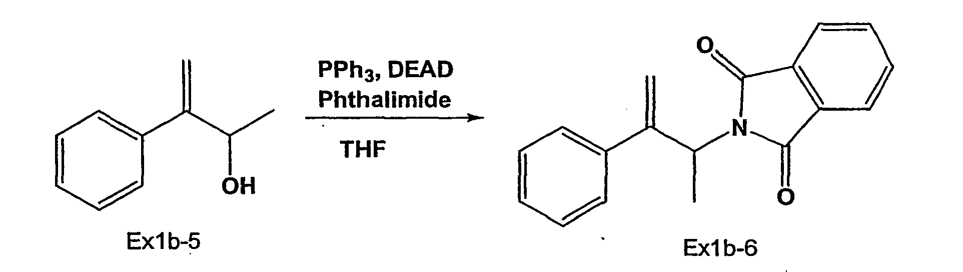 To a cooled solution of the alcohol (Exlb-5) (1.29 g, 8.7 mmol), phthalimide (1.34 g, 9.14 mmol), and triphenylphosphine (2.40 g, 9.14 mmol) in freshly distilled THF (28 mL) was added dropwise a solution of DEAD (1.59 g, 9.14 mmol) in THF (5 mL). Then the icebath was removed and the reaction was stirred under N₂ at room temperature for overnight. The solvent was removed in vacuo. The residue was purified on column chromatography (silica gel, 10-20% EtOAc/hexanes) to give an oil (1.23 g, 51%). ¹H NMR (CDCl₃, 300 MHz) δ 1.72 (d, *J*= 7.2 Hz, 3H), 4.12 (q, *J*= 7.2 Hz, 1H), 5.42, (d, *J* = 2.1 Hz, 1H), 5.45 (d, *J*= 2.1 Hz, 1H), 5.54 (m, 1H), 7.20-7.28 (m, 3H), 7.39 (m, 2H), 7.65 (m, 2H), 7.75 (m, 2H).

*Synthesis of compound Ex1b-7:* 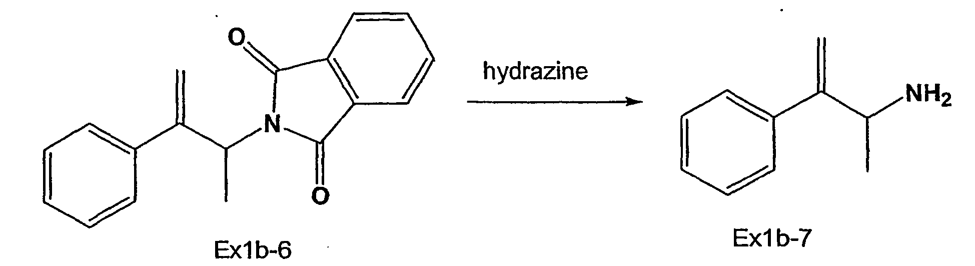 A mixture of compound Exlb-6 (0.402 g, 1.45 mmol) and hydrazine hydrate (0.127 mL, 2.17mmol) in ethanol (10 mL) was refluxed for 1.5 hrs. Then it was cooled and treated with 6N HCl (1.0 mL). The resulting reaction mixture was heated at 90°C for 45 min. The reaction mixture was concentrated in vacuo. The residue was dissolved in H₂O and filtered to remove insoluble solid. The filtrate was washed with ether (20 mL) and then basified to pH ∼9-10 by adding 1N NaOH solution. The basic solution was saturated with NaCl and extracted with ether (2 x 30 mL). The combined ether layers were then washed with H₂O (20 mL), brine (20 mL), dried (Na₂SO₄), and concentrated in vacuo. The amine residue was then dissolved in diethyl ether (3.0 mL), and 2N HCl in ether (3.62 mL, 7.24 mmol) was added. The hydrochloride salt was formed and precipitated immediately. It was filtered and dried in vacuo to give a solid (0.170 g, 63.8%). Mp: 175-178°C. ¹H NMR (D₂O, 300 MHz) δ 1.30 (d, *J*= 6.6 Hz, 3H), 4.45 (q, *J* = 6.6 Hz, 1H), 5.22 (s, 1H), 5.37 (s, 1H), 7.19-7.32 (m, 5H).

*Synthesis of compound Ex1b-8:* 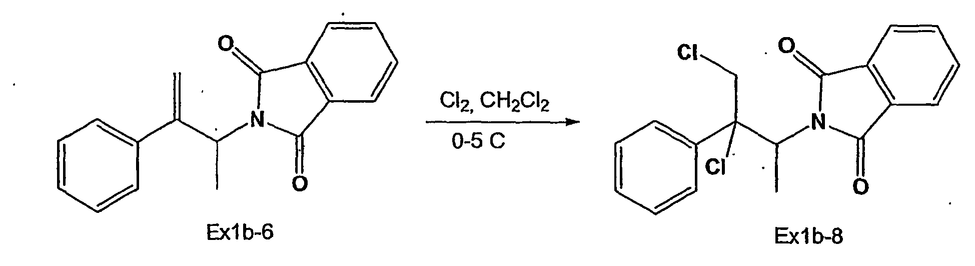 Cl₂ gas was bubbled through a cooled solution (well protected from light) of Exlb-6 (0.286 g, 1.03 mmol) in CH₂Cl₂ (12 mL) for 15 min. Then the Cl₂ gas was stopped and the reaction mixture was allowed to stir for 10 min when TLC showed that the reaction was completed. The reaction mixture was poured into brine and extracted with petroleum ether (2 x 30 mL). The combined organic layers were washed with 2% sodium bicarbonate, and then with water, dried over sodium sulfate, and concentrated in vacuo to give a crude oil. This oil was then purified on flash column chromatography (silica gel, 10% EtOAc/hexanes) to give the dichlorinated product (0.296 g, 82.4%). ¹H NMR (CDCl₃, 300 MHz) δ 1.66 (d, *J* = 7.2 Hz, 3H), 4.13 (d, *J* = 12.6 Hz, 1H), 4.65 (d, *J* = 12.3 Hz, 1H), 4.99 (q, *J* = 7.5 Hz, 1H), 7.19-7.34 (m, 5H), 7.50-7.61 (m, 2H), 7.69-7.81 (m, 2H).

*Synthesis of compounds Ex1b-10a and Ex1b-10b*
The final compounds Ex1b-10a and Ex1b-10b are obtained by using the procedures described by Ian McDonald in J. Med. Chem. 1985, 28, 186-193, proceeding through the intermediate compounds Exlb-9a and Exlb-9b. 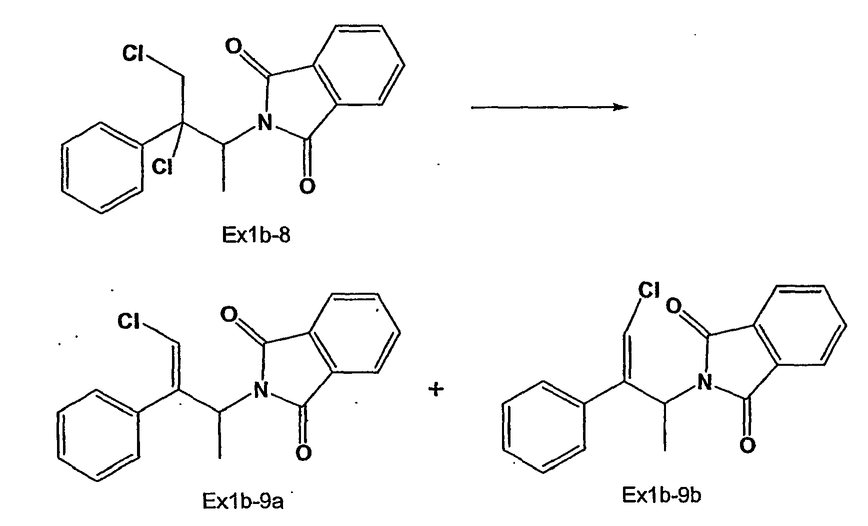 Following McDonald et al., the compound Exlb-8 and DBU are heated in dimethyl sulfoxide at about 95°C for about 4 hours. The mixture is cooled, diluted with cold water, and extracted with ether. The ether is concentrated and the product purified by column chromatography to give the mixture of Ex1b-9a and Exlb-9b.

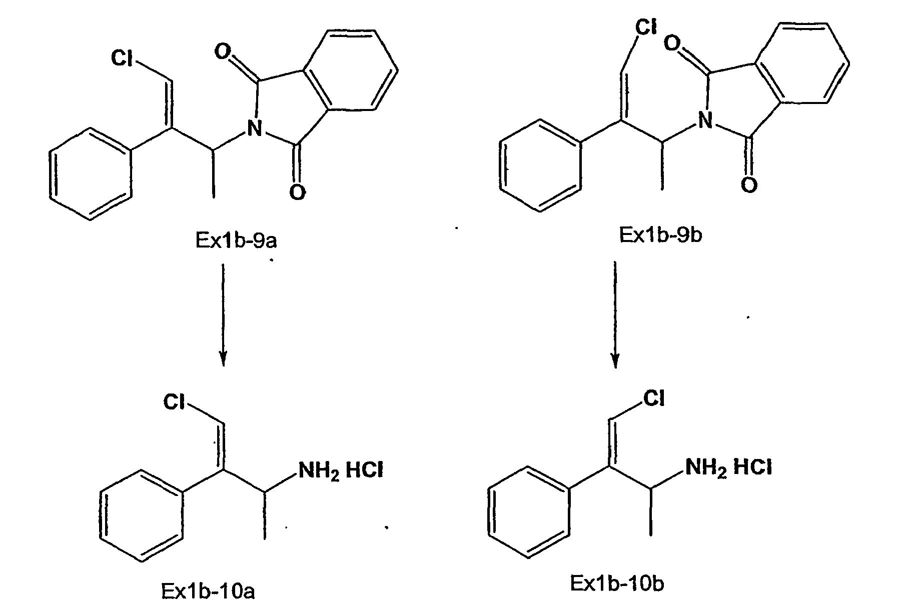

Compounds Exlb-9a and Exlb-9b are then deprotected using hydrazine hydrate in EtOH at reflux for about 90 minutes. The mixture is cooled, 18% aqueous HCl is added, and the mixture heated at about 90°C for about 45 minutes more. The mixture is cooled and filtered; the filtrate is then evaporated to dryness. The residue is extracted with ethanol, and the ethanol solution is evaporated to give Ex1b-10a and Ex1b-10b. The compounds can be recrystallized, e.g. from an ethanol/ether mixture. The E and Z isomers can be separated by methods known in the art, e.g. by HPLC.

### Example 2

### General synthetic procedures for the preparation of formula II

Compounds of formula II are prepared using compounds of the following structure as starting material: 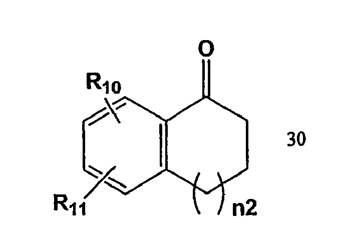 where n2 is 0, 1, or 2.

For example, to a cooled solution of indan-1-one 31 (3.0 g, 22.73 mmol; Aldrich) in THF (100 ml) is added dropwise a solution of LDA (1M, 23 ml, 23 mmol). After stirring at -78°C under N₂ for 30 min, a solution of N-(bromomethyl)phthalimide 32 (5.5 g, 22.91 mmol) in THF (30 ml) is added. The resulting mixture is stirred at room temperature overnight. The solvent is removed *in vacuo.* The residue is purified via column chromatography (silica gel, 20-40% EtOAc/haxenes) to give 2-(1-oxo-indan-2-ylmethyl)-isoindole-1,3-dione 33.

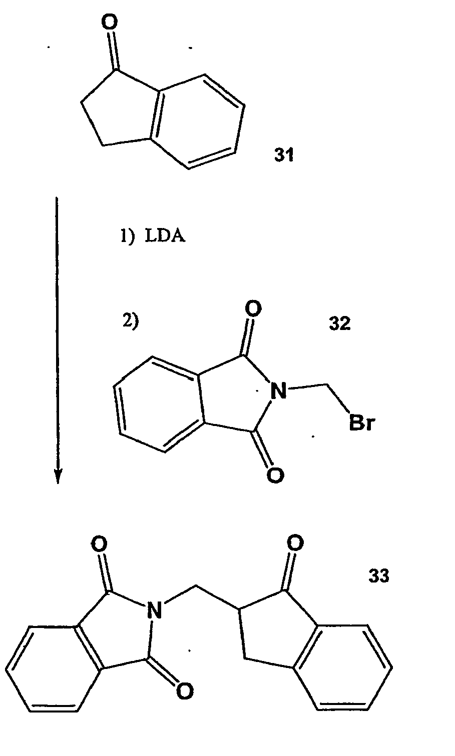

To a solution of 2-(1-oxo-indan-2-ylmethyl)-isoindole-1,3-dione 33 (2.7 g, 9.27 mmol) in MeOH (40 ml) is added NaBH₄ (0.35 g, 9.27 mmol). The resulting mixture is stirred at room temperature for 4 hrs, and then is quenched by adding 3% aqueous HCl solution. The mixture is extracted with EtOAc (3 x 20 ml). The combined organic layers are dried (MgSO₄), filtered. The filtrate is concentrated in vacuo to give a crude product (2.71 g, 100%). This crude product is used directly in the next step without any further purification.

To a cooled mixture of the crude product from above step (2.7 g, 9.22 mmol), Et₃N (1.93 ml, 13.82 mmol) in CH₂Cl₂ (75 ml) is added portionwise TsCl (1.76 g, 9.22 mmol). After the completion of addition of TsCl, the reaction mixture is stirred at room temperature for 3 hrs. The reaction mixture is washed with H₂O (2 x 20 ml), brine (20 ml), dried (MgSO₄), and filtered. The filtrate is concentrated in vacuo. The residue is purified via column chromatography (silica gel, 5-10% EtOAc/hexanes) to provide the tosylate 34.

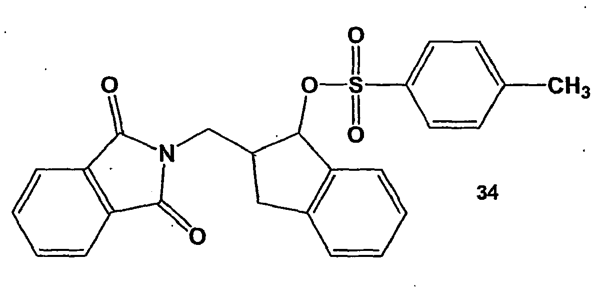

To a solution oftosylate in DMSO is added 1,8-diazabicyclo[5.4.0]undec-7-ene (DBU). The resulting mixture is heated to 60°C for 2 hrs. After cooling to room temperature, H₂O is added to the reaction mixture. The resulting mixture is extracted with EtOAc. The combined organic layers are dried (MgSO₄), and filtered. The filtrate is concentrated in vacuo. The residue is purified via column chromatography (silica gel, 5-10% EtOAc/hexanes) to give the corresponding 1H-indene derivative 35.

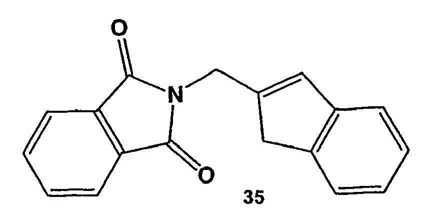

The target compound 36 is then be obtained by removing phthalido protecting group from the nitrogen using published procedures (e.g. hydrazinolysis).

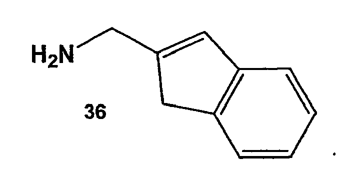

### Example 3

### Synthesis of 3-benzyl-4,4,4-trifluoro-but-2-enylamine, a compound embraced by General Formula IV

To a suspension of NaH (0.55 g, 21.77 mmol) in THF (40 ml) was added dropwise a solution of triethyl phosphonoacetate (5.0 g, 21.8 mmol) in THF (10 ml). The resulting mixture was stirred at room temperature for 15 min, then a solution of benzyl trifluoromethyl ketone (3.5 ml, 21.77 mmol) in THF (10 ml) was added. The reaction mixture was stirred at room temperature for 2 hrs, and then was quenched by addition of 10% aqueous HCl solution. The resulting mixture was extracted with EtOAc (3 x 20 ml). The combined organic layers were dried (MgSO₄) and filtered. The filtrate was concentrated *in vacuo.* The residue was purified via column (silica gel, 10% EtOAc/hexanes) to give 3-benzyl-4,4,4-trifluoro-but-2-enoic acid ethyl ester as an oil (4.0 g, 71%). ¹H NMR (CDCl₃, 300 MHz) δ 1.23-1.33 (m, 3H), 4.10 (s, 2H), 4.15-4.31 (m, 2H), 5.76, 6.49 (two br s, total 1H), 7.15-7.41 (m, 5H).

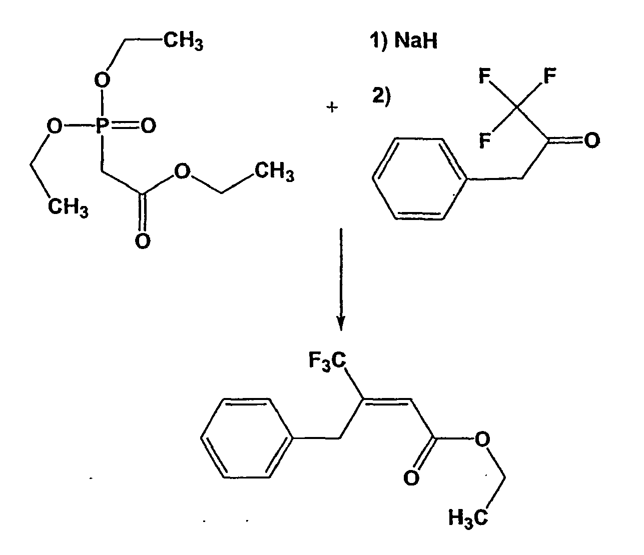

To a cooled solution of 3-benzyl-4,4,4-trifluoro-but-2-enoic acid ethyl ester (4.0 g, 15.5 mmol) in CH₂Cl₂/hexanes (7.0/23.0 mml) at -78°C under N₂ was added a solution of DIBAL in hexanes (1M, 36 ml, 36 mmol). The resulting mixture was stirred at -78°C under N₂ for 3 hrs, and then was quenched by adding MeOH (∼5.0 ml). The reaction mixture was concentrated *in vacuo.* The residue was dissolved in slightly acidic H₂O (20 ml). The aqueous solution was extracted with EtOAc (3 x 30 ml). The combined organic layers were dried (MgSO₄), filtered. The filtrate was concentrated in *vacuo* to give an oil (3.3 g, 100%), which was used directly in the next step without any further purification.

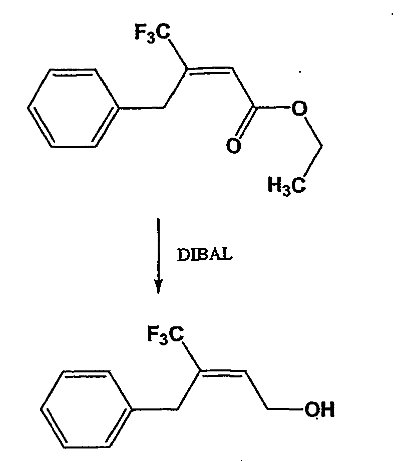

To a solution of the alcohol obtained from the previous step (3.3 g, 15.3 mmol), PPh₃ (4.28 g, 16.2 mmol), and phthalimide (2.4 g, 16.4 mmol) in THF (100 ml) was added a solution of diethylazo dicarboxylate (DEAD) (2.65 ml, 16.3 mmol) in THF (10 ml). The resulting mixture was stirred at room temperature under N₂ for overnight. The solvent was removed under reduced pressure. The residue was purified via column chromatography (silica gel, 20-30% EtOAc/hexanes) to provide 2-(3-benzyl-4,4,4-trifluoro-but-2-enyl)-isoindole-1,3-dione as a white solid (4.0 g, 76%). ¹H NMR (CDCl₃, 300 MHz) δ 3.48, 3.78 (two s, total 2H), 4.33, 4.57 (two br s, total 2H), 5.56, 6.30 (two t, *J* = 6 Hz, total 1H), 7.11-7.36 (m, 5H), 7.67-7.76 (m, 2H), 7.78-7.88 (m, 2H).

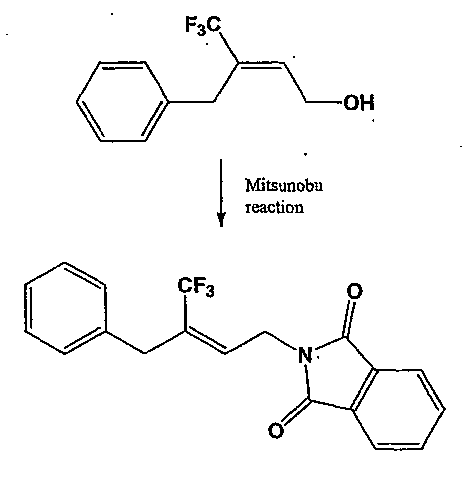

A mixture of 2-(3-benzyl-4,4,4-trifluoro-but-2-enyl)-isoindole-1,3-dione (1.75 g, 5.07 mmol), hydrazine hydrate (0.18 ml, 5.78 mmol) in MeOH (20 ml) was refluxed for 4 hrs. The mixture was concentrated in vacuo. The residue was re-dissolved in MeOH (20 ml).. To this solution was added 18% aqueous HCl solution. The resulting mixture was refluxed for 40 min, and then cooled to room temperature, and filtered. The filtrate was concentrated in vacuo. The residue was dissolved in H₂O (10 ml). The aqueous layer was washed with ether (2 x 20 ml). The aqueous layer was made basic by adding NaOH. The resulting basic solution was saturated with NaCl, extracted with ether (3 x 30 ml). The combined organic layers were dried (MgSO₄), filtered, and concentrated. The residue was dissolved in ether (5 ml). To this solution was added a solution of HCl in ether (2M, 5ml, 10 mmol). A white solid formed was filtered and washed with ether, then dried (0.5 g, 40%) to yield 3-benzyl-4,4,4-trifluoro-but-2-enylamine. mp 170°C (decom). ¹HNMR (D₂O, 300 MHz) δ 3.47, 3.56 (two s, total 2H), 3.64, 3.71 (two dt, J= 6, 3 Hz, total 2H), 5.68, 6.27 (two t, J = 6 Hz, total 1H), 7.06-7.33 (m, 5H).

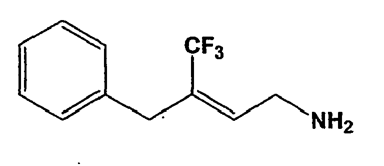

### Example 3A

### α-difluoromethylphenylalanine, a compound embraced by general formula V

A specific example of the synthesis of α-difluoromethylphenylalanine methyl ester hydrochloride is given below, starting from commercially available L-phenylalanine methyl ester (Aldrich).

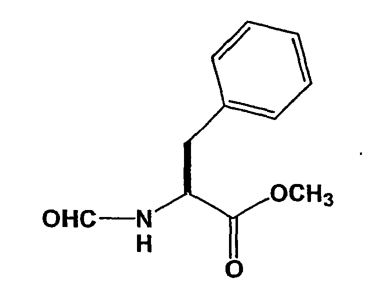

***N*-Formyl-L-phenylalanine methyl ester.** Formamide (276 µL, 6.95 mmol), L-phenylalanine methyl ester hydrochloride (1 g, 4.64 mmol) and toluene (50 mL) were combined. The flask was equipped with a Dean-Stark trap, and the system was heated to reflux for 4.5 h. The mixture was cooled to room temperature, and the solvent was removed in vacuo. The crude oil was purified by column chromatography (MeOH/CH₂Cl₂ 1%, 2%, 5%) to yield 0.79 g (82%) of an amber oil. ¹H NMR (300 MHz, CDCl₃) δ 3.10 (dd, *J* = 5.7, 14.1 Hz, 1H), 3.18 (dd, *J* = 5.7, 14.1 Hz, 1H), 3.74 (s, 3H), 4.96 (m, 1H), 6.22 (br s, 1H), 7.10 (m, 2H), 7.27 (m, 3H), 8.15 (s, 1H).

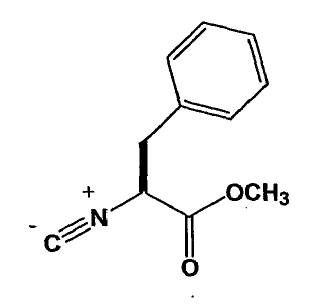

**2-Isocyano-3-phenyl-propionic acid methyl ester.** Phosphorous oxychloride (0.41 mL, 4.52 mmol) was slowly added to a mixture of *N*-formyl-L-phenlalanine methyl ester (0.78 g, 3.76 mmol), triethylamine (1.57 mL, 11.3 mmol) and CH₂Cl₂ (8 mL) while stirring at 0°C. The mixture was stirred under N₂ at decreased temperatures as the reaction was monitored by TLC. After 1 h, the reaction was quenched by the addition of chilled 5% NH₄OH. The layers were separated, and the aqueous layer was extracted with CH₂Cl₂. The organic layers were combined, washed with water and brine, and dried over Na₂SO₄. The crude product was purified by column chromatography (100% CH₂Cl₂, 1% MeOH/CH₂Cl₂) to yield 0.25 g (35%) of a clear oil. ¹H NMR (300 MHz, CDCl₃) δ 3.13 (dd, *J*= 8.2, 13.8 Hz, 1H), 3.26 (dd, *J*= 4.6, 14.1 Hz, 1H), 3.79 (s, 3H), 4.46 (dd, *J*= 5.0, 8.2 Hz, 1H), 7.26 (m, 2H), 7.33 (m, 3H).

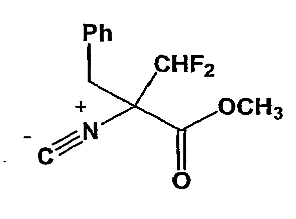

**2-Benzyl-3,3-difluoro-2-isocyano-propionic acid methyl ester.** A solution of 2-isocyano-3-phenylpropionic acid methyl ester in a minimum amount of THF (2.0 ml) was added to a suspension of NaH in THF (20 ml) stirring at 0°C. After 10 min, chlorodifluoromethane gas was bubbled through the mixture for 30 min. At this time, the flask was capped, and the mixture was gradually warmed to room temperature as it was stirred for another 3 h. The reaction was quenched upon the addition of 20% acetic acid (680 µL), and the mixture was concentrated in vacuo. The residual liquid was extracted with diethyl ether (2 x 20 ml). The ether layers were washed with water, saturated bicarbonate, and brine, before it was dried over Na₂SO₄ and concentrated. The crude product was purified by column chromatography (CH₂Cl₂/hexane 1:1) to yield 52 mg (16%) of a clear yellow liquid. ¹H NMR (300 MHz, CDCl₃) δ 3.20 (dd, *J* = 13.6, 36.6 Hz, 2H), 3.70 (s, 3H), 6.03 (t, *J*= 53.7 Hz, 1H), 7.19 (m, 2H), 7.31 (m, 3H).

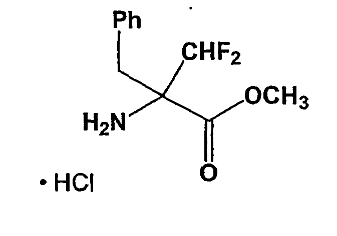

**α-Difluoromethylphenylalanine methyl ester hydrochloride.** A solution of 2 N HCl/ether (0.6 mL) was added to a solution of the nitrile in a minimum volume of methanol. The mixture was heated to 50 °C for 1 h then concentrated in vacuo. The product was precipitated upon addition of ethyl ether, which was removed in vacuo to yield a white foam. To remove impurities evident in analytical HPLC trace, the foam was dissolved in a mixture of water and ether. The aqueous layer was separated and the pH was adjusted to 10 with NaOH solution before it was extracted with ether. The organic layer was dried over Na₂SO₄ and concentrated. The product was converted to the hydrochloride salt upon treatment with 2 N HCl/ether. ¹H NMR was done in CDCl₃ on 300 MHz instrument. The spectrum was not well resolved. The peaks were very broad. HRMS (MALDI-FTMS) calcd for C₁₁H₁₄NF₂O₂ *m*/*z* (M+H⁺) 230.0987, found 230.0985; HPLC: Vycac C18, 10-35%B 20 min, 0.1% TFA in H₂O/CH₃CN, 210 nm, 1mL/min, t_{R} = 8.26 min.

### Example 3B

### Synthesis of 2-(2-Amino-2-methyl-propylamino)-1-phenyl-ethanol dihydrochloride (a compound embraced by general formula VI)

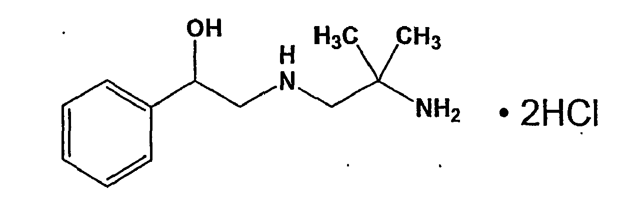

Styrene oxide (57 µL, 0.5 mmol) was added dropwise to a solution of 1,2-diamino-2-methylpropane (100 µL). The flask was equipped with a condenser and heated to 65 °C for approximately 90 min. The mixture was cooled to room temperature, and the sample was concentrated in vacuo. The residue was dissolved in water and washed with diethyl ether. The organic layer was washed with water. The aqueous layers were combined, acidified with glacial acetic acid, and filtered through a 0.45 µ filter before purification by HPLC.

The HPLC conditions used were as follows:
Column: Dynamax C18 60 A, 1 x 10 in.
Gradient: 7-14%B in 30 min.
Solvents: A) 0.1%TFA/H₂O; B) 0.1%TFA/CH₃CN
Detector: 254 nm
Flow rate: 10 mL/min

The fractions were checked by analytical HPLC (Vydac C18, 10-50%B in 20 min, 1 mL/min, 210/254 nm) and electrospray mass spectrometry. The fractions containing the product were combined and lyophilized to yield 9 mg (10%) of a white residue. Once dry, the residue was dissolved in a minimum volume of methanol (50-75 µL) and 1N HCl/ether. The solvent was removed after 5-10 min. More ethyl ether was added to the residue to precipitate the product, then the ether was removed in vacuo and the sample was dried under vacuum. ¹H NMR (300 MHz, MeOH-*d₄*) δ 1.55 (s, 6H), 3.36 (m, 2H), 3.47 (m, 2H), 5.16 (dd, *J* = 1.85, 6.50 Hz, 1H), 7.34 (m, 1H), 7.40 (m, 2H), 7.48 (m, 2H); MS (MALDI-FTMS) expected: 209.1648 (M+H); found: 209.1649 (M+H).

### Example 3C

### 1-[3-(4-Methoxy-phenyl)-propyl]-prop-2-ynylamine (a compound embraced by general formula VII)

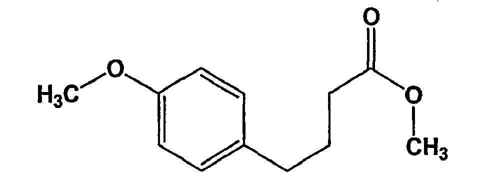

A mixture of 4-(4-methoxyphenyl) butyric acid (5.0 g, 25.8 mmol) and H₂SO₄ (concentrated, cat. amount) in MeOH (30 ml) was refluxed for 3 hr. The mixture was concentrated to remove excess MeOH. The residue was diluted with EtOAc (30 ml) and washed with H₂O (20 ml), Sat. NaHCO₃ (2 x 15 ml), H₂O (20 ml), and brine (30 ml) respectively. The organic layer was then dried (MgSO4), filtered. The filtrate was concentrated in vacuo to provide methyl 4-(4-methoxyphenyl) butyrate (5.3 g, 100%). ¹H NMR (CDCl₃, 300 MHz) δ 1.89 (p, J = 6.6 Hz, 2H), 2.30 (t, J = 7.2 Hz, 2H), 2.57 (t, J = 7.5 Hz, 2H), 3.64 (s, 3H), 3.76 (s, 3H), 6.80 (d, J = 8.1 Hz, 2H), 7.07 (d, J = 8.1 Hz, 2H).

To a cooled solution of methyl 4-(4-methoxyphenyl) butyrate (3.6 g, 17.3 mmol) in CH₂Cl₂ (40 ml) at -78°C under N₂ was added dropwise a solution of DIBAL in hexanes (1.0 M, 18.0 ml, 18 mmol). The resulting mixture was stirred at -78°C under N₂ for 3 hrs, and then quenched by adding MeOH (-5 ml). The resulting mixture was warmed gradually to room temperature and filtered. The filtrate was concentrated *in vacuo* to give 4-(4-Methoxy-phenyl)-butyraldehyde as an oil (3.06 g, 100%). ¹H NMR (CDCl₃, 300 MHz) δ 1.91 (p, J = 7.5 Hz, 2H), 2.41 (t, J = 7.5 Hz, 2H), 2.58 (t, J = 7.5 Hz, 2H), 3.76 (s, 3H), 6.81 (d, J = 8.4 Hz, 2H), 8.07 (d, J = 8.4 Hz, 2H), 9.73 (s, 1H). The residue was used directly in the next step without any further purification.

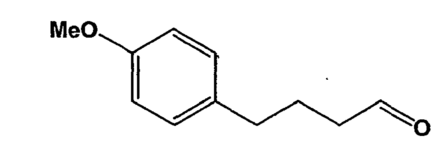

To a cooled solution of 4-(4-Methoxy-phenyl)-butyraldehyde (1.13 g, 6.35 mmol) in THF (30 ml) was added a solution of ethynylmagnesium bromide in THF (0.5 M, 14.0 ml, 7.0 mmol). The resulting mixture was stirred at room temperature for 3 hrs, and then was quenched by adding dilute HCl solution. The layers were separated. The aqueous layer was extracted with EtOAc (3 x 20 ml). The combined organic layers were dried (MgSO₄), filtered. The filtrate was concentrated in vacuo. The residue was purified via column chromatography (silica gel, 25-30% EtOAc/hexanes) to afford 6-(4-Methoxy-phenyl)-hex-1-yn-3-ol as an oil (0.61 g, 47%). 1H NMR (CDCl3, 300 MHz) δ 1.71-1.81 (m, 4H), 2.46 (s, 1H), 2.61 (t, J = 6.9 Hz, 2H), 3.79 (s, 3H), 4.38 (br s, 1H), 6.83 (d, J = 8.4 Hz, 2H), 7.11 (d, J = 8.4 Hz, 2H).

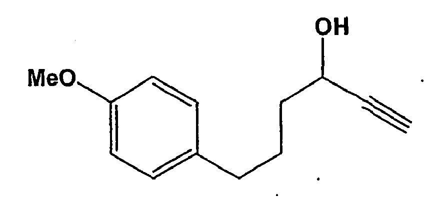

To a mixture of 6-(4-Methoxyphenyl)-hex-1-yn-3-ol (0.6 g, 2.94 mmol), PPh3 (0.77 g, 2.94 mmol), and phthalimide (0.43 g, 2.94 mmol) in THF (20 ml) was added a solution of DEAD (0.48 ml, 2.94 ml) in THF (5.0 ml). The resulting mixture was stirred at room temperature under N₂ for overnight. After removing solvent, the residue was purified via column chromatography (silica gel, 20-40% EtOAc/hexanes) to provide 2- {1-[3-(4-Methoxy-phenyl)-propyl]-prop-2-ynyl}-isoindole-1,3-dione (0.62 g, 63%). ¹H NMR (CDCl₃, 300MHz) δ 1.58-1.81 (m, 2H), 2.00-2.21 (m, 2H), 2.36 (br s, 1H), 2.55-2.65 (m, 2H), 3.77 (s, 3H), 5.06 (dt, J = 2.4, 8.1 Hz, 1H), 6.81 (d, J = 8.4 Hz, 2H), 7.08 (d, J = 8.4 Hz, 2H), 7.70-7.76 (m, 2H), 7.82-7.89 (m, 2H).

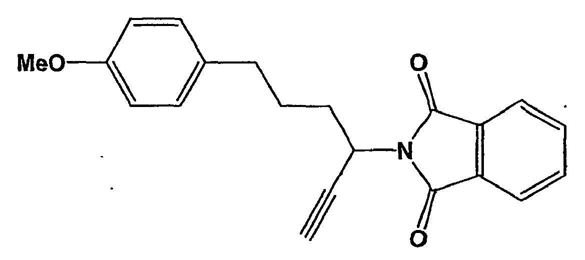

A mixture of 2-{1-[3-(4-Methoxyphenyl)-propyl]-prop-2-ynyl}-isoindole-1,3-dione (0.62 g, 1.86 mmol), H₂NNH₂*xH₂O (0.06 ml, 1.93 mmol) in MeOH (20 ml) was refluxed for 3 hrs. 6 N HCl (5.0 ml) was then added. The mixture was continued to reflux for 45 min, then cooled, and filtered. The filtrate was concentrated *in vacuo.* The residue was dissolved in H₂O (10 ml). The aqueous solution was washed with ether (2 x 20 ml). The aqueous solution was basified with NaOH to pH around 12, saturated with NaCl, and then extracted with ether (3 x 30 ml). The combined organic layers were dried (MgSO4), filtered. The filtrate was concentrated *in vacuo.* The residue was dissolved in ether (2 ml). To this solution was added a solution of HCl in ether (1.0 M, 5.0 ml, 5.0 mmol). The solid precipitated was filtered, washed with ether, and dried to give 1-[3-(4-Methoxy-phenyl)-propyl]-prop-2-ynylamine hydrochloride (0.31 g, 70%). mp: 175°C (decompose). ¹H NMR (D₂O, 300 MHz) δ 1.51-1.71 (m, 4H), 2.41-2.49 (m, 2H), 2.77-2.79 (m, 1H), 3.62 (s, 3H), 3.89-3.96 (m, 1H), 6.77 (d, J = 7.8 Hz, 2H), 7.05 (d, J = 7.8 Hz, 2H).

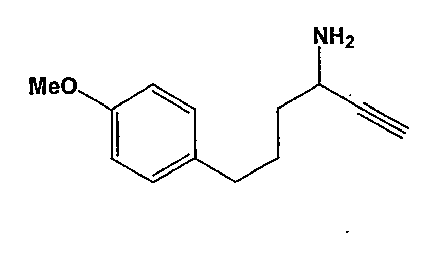

### Examples 3D

### 1-(3,4-difluorophenyl)-4-(4-methoxyphenyl)-butylamine (a compound embraced by general formula VII)

To a cooled solution of 4-(4-Methoxy-phenyl)-butyraldehyde (1.03 g, 5.78 mmol) in THF (30 ml) at 0°C under N₂ was added a solution of 3,4-difluorophenylmagnesium bromide in THF (0.5 M, 13 ml, 6.5 mmol). The resulting mixture was stirred at 0°C under N₂ for 3 hrs, and quenched by adding H2O. The layers were separated. The aqueous layer was extracted with EtOAc (3 x 20 ml). The combined organic layers were dried (MgSO4), filtered. The filtrate was concentrated in vacuo. The residue was purified via column chromatography (silica gel, 20-40% EtOAc/hexanes) to give 1-(3,4-Difluoro-phenyl)-4-(4-methoxy-phenyl)-butan-1-ol as a solid (0.25 g, 15%). ¹H NMR (CDCl₃, 300 MHz) δ 1.35-1.69 (m, 4H), 2,55 (t, J = 7.5 Hz, 2H), 3.76 (s, 3H), 4.49-4.66 (m, 1H), 6.80 (d, J = 8.4 Hz, 2H), 6.92-7.22 (m, 5H).

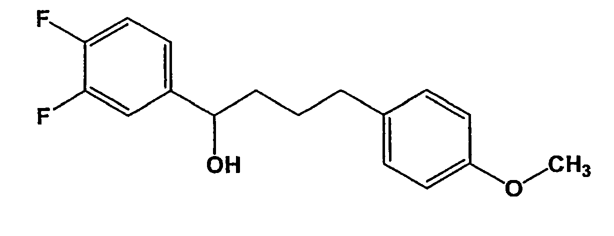

A mixture of 1-(3,4-Difluoro-phenyl)-4-(4-methoxy-phenyl)-butan-1-ol (0.25 g, 0.86 mmol), PPh₃ (0.23 g, 0.88 mmol), phthalimide (0.13 g, 0.88 mmol) in THE (30 ml) was treated with a solution of DEAD (0.14 ml, 0.88 mmol) in THF 95.0 ml). The resulting mixture was stirred at room temperature under N₂ for overnight, and then concentrated in vacuo. The residue was purified via column chromatography (silica gel, 20-30% EtOAc/hexanes) to give 2-[1-(3,4-Difluoro-phenyl)-4-(4-methoxy-phenyl)-butyl]-isoindole-1,3-dione as a solid (0.1 g, 27%). ¹H NMR (CDCl₃, 300MHz) δ 1.51-1.82 (m, 4H), 2.57 (t, J = 7.5 Hz, 2H), 3.78 (s, 3H), 4.59-4.69 (m, 1H), 6.81 (d, J = 8.7 Hz, 2H), 6.94-7.20 (m, 9H).

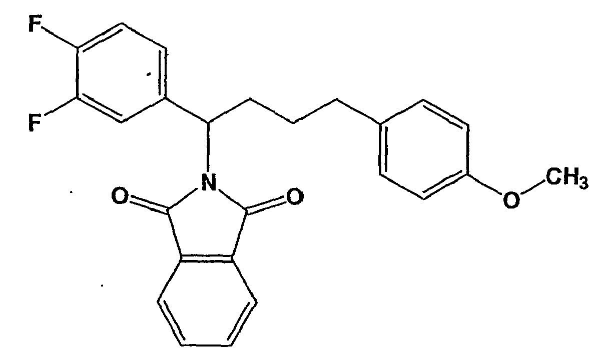

A mixture of 2-[1-(3,4-Difluoro-phenyl)-4-(4-methoxy-phenyl)-butyl]-isoindole-1,3-dione (0.1 g, 0.24 mmol), H₂NNH₂*xH₂O (10 µl, 0.32 mmol) in MeOH (5 ml) was refluxed for 3 hrs. A solution of 16N HCl (2.0 ml) was added. The mixture was continued to reflux for 45 min, then cooled to room temperature, and concentrated in vacuo. The residue was dissolved in H₂O (10 ml), washed with ether (2x10 ml). The aqueous layer was basified to pH 14 by adding NaOH, saturated with NaCl, and extracted with ether (3x20 ml). The combined organic layers were dried (MgSO4), filtered. The filtrate was concentrated in vacuo. The residue was dissolved in ether (-2 ml). To this solution was added a solution of HCl in ether (1.0 M, 2.0 ml). The solid precipitated was collected, washed with ether, and dried (10 mg, 13%) to yield the product 1-(3,4-difluorophenyl)-4-(4-methoxyphenyl)-butylamine. mp: 200°C (decompose). ¹HNMR D₂O, 300MHz) δ 1.13-1.47 (m, 2H), 1.62-1.87 (m, 2H), 2.22-2.47 (m, 2H), 3.62 (s, 3H), 4.07-4.15 (m, 1H), 6.73 (d, J = 8.7 Hz, 2H), 6.88-7.22 (m, 5H).

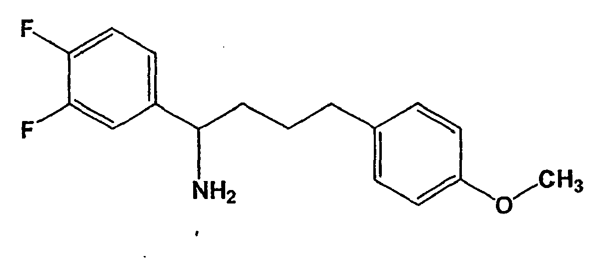

### Example 3E

### 1-(3-phenyl-propyl)-allylamine (a compound embraced by general formula VII)

A mixture of 4-phenyl butyric acid (3.64 g, 23.95 mmol), H₂SO₄ (several drops, catalytic amount) in MeOH (17 ml) was refluxed for 3 hr. TLC showed that no starting material was present. The mixture was concentrated *in vacuo.* The residue was dissolved in EtOAc (30 ml), and washed with sat. NaHCO₃ (2 x 20 ml), H₂O (2 x 20 ml), and brine (30 ml). The organic layer was dried (MgSO₄), and filtered. The filtrate was concentrated *in vacuo* to provide an oil (3.55 g, 90%). ¹H NMR (CDCl₃. 400 MHz) δ 1.93-2.03 (m, 2H), 2.46 (t, *J*= 5.88 Hz, 2H), 2.67 (t, *J*= 5.97 Hz, 2H), 3.67 (s, 3H), 7.12-7.22 (m, 3H), 7.24-7.33 (m, 2H).

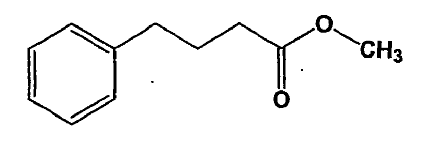

To a cooled mixture of methyl 4-phenyl butyric acid (3.55 g, 19.94 mmol) in CH₂Cl₂ (40 ml) at -78°C under N₂ was added dropwise a solution of DIBAL in hexanes (1 M, 22 ml, 22 mmol). The resulting mixture was stirred at -78°C under N₂ for 3hr, then quenched by adding MeOH (5.0 ml). The mixture was filtered. The filtrate was concentrated *in vacuo* to give an oil (2.84, 96%). ¹H NMR (CDCl₃, 400 MHz) δ 1.97 (t, *J*= 6.0 Hz, 2H), 2.47 (t, *J*= 6.0 Hz, 2H), 2.67 (t, *J*= 6.0 Hz, 2H), 7.11-7.22 (m, 3H), 7.24-7.35 (m, 2H), 9.76 (s, 1H).

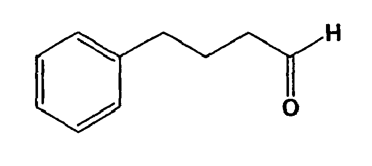

To an ice-cooled solution of 4-phenylbutyraldehyde (2.84 g, 19.16 mmol) in THF (40 ml) under N₂ was added a solution of vinylmagnesium bromide in THF (1 M, 19,2 ml, 19.2 mmol). The resulting mixture was stirred at room temperature under N₂ for 3 hr, then poured into ice-H₂O. The mixture was extracted with CH₂Cl₂ (2 x 30 ml). The combined organic layers were dried (MgSO₄) and filtered. The filtrate was concentrated *in vacuo.* The residue was purified on a column (silica gel, 10-20% EtOAc/hexanes) to give 6-phenyl-hex-1-en-3-ol as an oil (1.56 g, 46%). ¹H NMR (CDCl₃, 400 MHz) δ 1.50-1.82 (m, 5H), 2.65 (t, J = 6.04 Hz, 2H), 4.08-4.15 (m, 1H), 5.11 (dd, J = 8.28, 1.03 Hz, 1H), 5.22 (dt, J =13.8, 1.16 Hz, 1H), 5.81-5.91 (m, 1H), 7.15-7.21 (m, 3H), 7.25-7.32 (m, 2H).

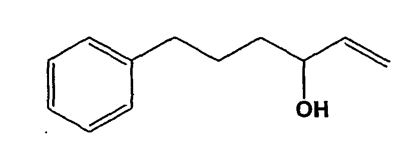

To a mixture of 6-phenyl-hex-1-en-3-ol (1.53 g, 8.68 mmol), phthalimide (1.28 g, 8.68 mmol), and PPh₃ (2.28 g, 8.68 mmol) in THF (30 ml) was added a solution of DEAD (1.41 ml, 8.68 mmol) in THF (5.0 ml). The resulting reaction mixture was stirred under N₂ at room temperature overnight. It was concentrated in vacuo. The residue was purified on a column (silica gel, 10-15% EtOAc/hexanes) to afford 2-[1-(3-phenyl-propyl)-allyl]-isoindole-1,3-dione as a yellow oil (1.47 g, 89%). 1H NMR (CDCl3, 400 MHz) δ 1.50-1.70 (m, 2H), 1.88-2.01 (m, 1H), 2.08-2.20 (m, 1H), 2.57-2.69 (m, 2H), 4.76 (q, J = 6.06 Hz, 1H), 5.15-5.25 (m, 2H), 6.14-6.26 (m, 1H), 7.08-7.19 (m, 3H), 7.22-7.28 (m, 2H), 7.66-7.74 (m, 2H), 7.78-7.83 (m, 2H).

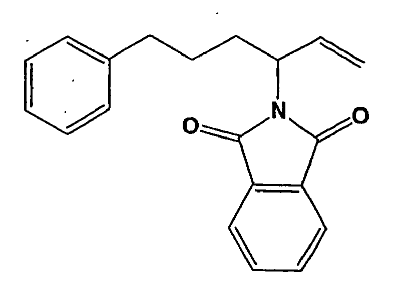

A mixture of 2-[1-(3-phenyl-propyl)-allyl]-isoindole-1,3-dione (1.4 g, 4.58 mmol) and NH₂NH₂·xH₂O (0.16 ml, 5.14 mmol) in MeOH (20 ml) was refluxed for 3 hr, then concentrated in vacuo. The residue was re-dissolved in MeOH (20 ml). To this solution was added 18% aqueous HCl (5.0 ml). The resulting mixture was refluxed for 40 min, cooled, and filtered. The filtrate was concentrated in vacuo. The residue was dissolved in H₂O (10 ml). The solution was washed with ether (2 x 20 ml). The aqueous layer was made basic by adding aq. NaOH. The solution was saturated with NaCl, then extracted with ether (3 x 20 ml). The combined ether layers were dried (MgSO₄) and filtered. The filtrate was concentrated *in vacuo.* The residue was dissolved in a small volume of ether (∼2.0 ml). To this solution was added a solution of HCl in ether (2 M, 3.0 ml, 6.0 mmol). A white precipitate was formed. The solid was filtered, washed with ether and EtOAc, and then dried (0.51 g, 91%) to yield the desired product, 1-(3-phenylpropyl)-allylamine. mp: 143-144°C. ¹HNMR (D₂O, 400 MHz) δ 1.58-1.75 (m, 4H), 2.58-2.72 (m, 2H), 3.68-3.82 (m, 1H), 5.32-5.41 (m, 2H), 5.75-5.82 (m, 1H), 7.18-7.28 (m, 3H), 7.31-7.41 (m, 2H).

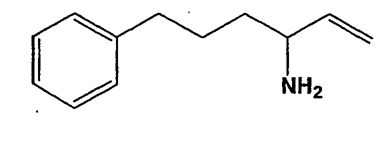

### Example 3F

### 2-amino-N-(4-fluorobenzyl)acetamide (a compound embraced by general fonnula IX)

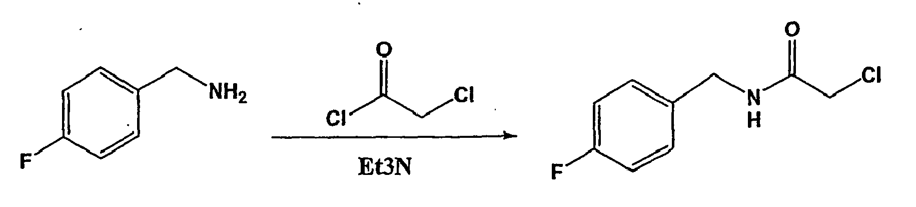

To a cooled solution of 4-fluorobenzylamine (2.3 g, 18.4 mmol) and Et₃N (3.85 ml, 27.6 mmol) in CH₂Cl₂ (45 ml) was added a solution of chloroacetyl chloride (2.49 g, 1.76 ml) in CH₂Cl₂ (5 ml). The resulting mixture was stirred at room temperature overnight, then was transferred into a separatory funnel, and washed with H₂O (2 x 50 ml), NaHCO₃ (100 ml), and brine (100 ml) respectively, and then dried over MgSO₄ and filtered. The filtrate was concentrated to give a yellowish solid. It was purified via column chromatography (silica gel, 33% EtOAc/hexanes) to provide the pure product (3.1g, 84.5%). ¹H NMR (CDCl₃, 300 MHz) δ 7.24-7.36 (m, 2H), 7.05-7.16 (m, 2H), 6.71-7.02(s, 1H), 4.38-4.51(d, 2H), 4.03-4.10(s, 1H).

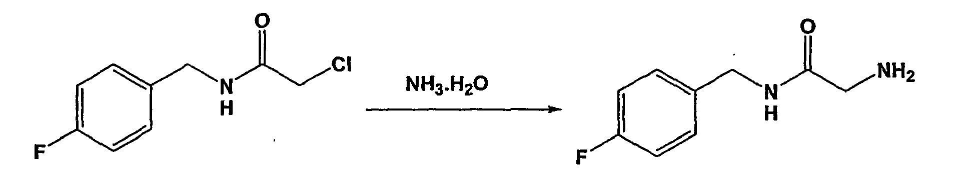

To a solution of 2-chloro-N-(4-fluoro-benzyl)-acetamide (0.2 g, 1 mmol) and KI (catalytic amount) in DMF (10 ml) was added concentrated NH₃·H₂O (0.4 ml). The resulting mixture was heated at 60°C for 1.5 hr. TLC showed the reaction was completed. The reaction mixture was concentrated. The residue was purified via column chromatography (silica gel, 5-10% MeOH/CH₂Cl₂) to give the product (140 mg, 77.7%). ¹H NMR (CDCl₃, 300 MHz) δ 7.22-7.38 (m, 2H), 6.95-7.10 (m, 2H), 4.45-4.50 (m, 2H), 3.32-3.45(m, 2H).

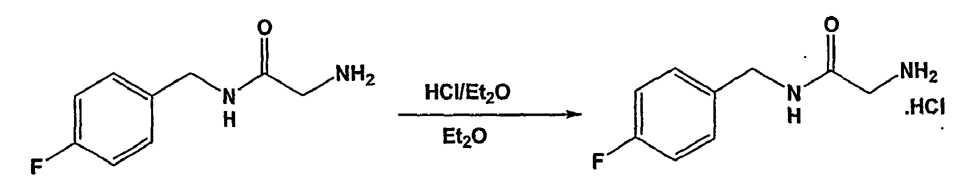

To a solution of 2-amino-N-(4-fluoro-benzyl)-acetamide (100 mg, 0.55 mmol) in Et₂O (20 ml) was added a solution of HCl in Et₂O (2.0 M, 0.5 ml, 1.0 mmol). The resulting mixture was stirred at room temperature. The solid precipitate was collected by filtration, washed with EtOAc, and dried in vacuo to give a solid (40 mg). mp 114-115 °C. ¹H NMR (D₂O, 300 MHz) δ 7.28-7.38 (m, 2H), 6.92-7.06(m, 2H), 4.35-4.48(m, 2H), 3.25-3.30 (s, 2H). HRMS Calcd for C₉H₁₂FN₂O [M+H]⁺ 183.0928. Found 183.0924.

### Example 3G

### 2-amino-N-pyridin-3-ylmethylacetamide (a compound embraced by general formula IX)

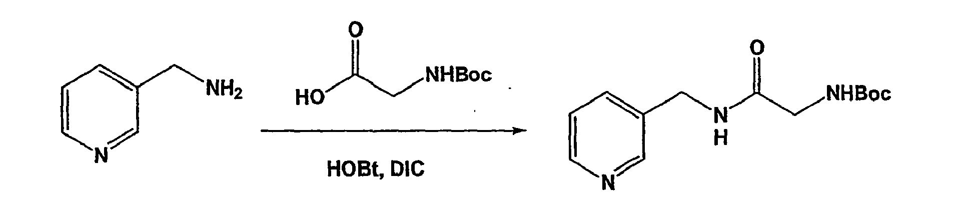

A solution of Boc-Gly-OH (1.73 g, 9.9 mmol) and 1,3-diisopropylcarbodiimide (2.0 ml, 12.8 mmol) in DMF (20 ml) was stirred at room temperature for 5 min. To this solution was added 1-hydroxybenzotriazole (HOBt; 1.74 g, 12.8 mmol), followed by 3-aminomethyl pyridine (1.0 ml, 9.9 mmol). The resulting mixture was stirred at room temperature overnight. The solid formed was filtered. The filtrate was washed with H₂O (2 x 50 ml), saturated NaHCO₃ (2 x 50 ml), and brine (100 ml), and then dried over Na₂SO₄. Ater the removal of solvent, the residue was purified via column chromatography (silica gel, CH₂Cl₂:EtOAc:MeOH = 3:2:0.03) to give the pure product (2.2g, 85%). ¹H NMR (CDCl₃, 300 MHz) δ 8.59-8.76 (m, 2H), 7.65-7.85 (m, 1H), 7.24-7.38 (m, 2H), 4.42-4.56 (m, 2H), 3.70-3.84 (m, 2H), 1.30-1.42 (s, 9H).

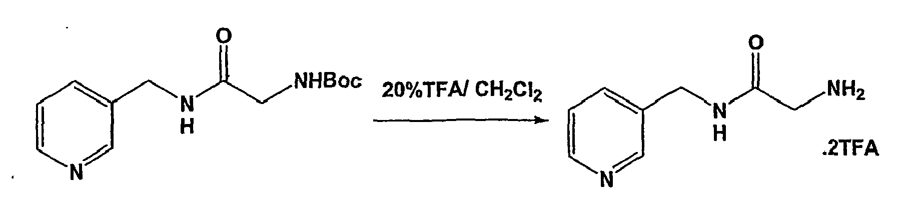

A mixture of [(pyridine-3-ylmethyl-carbamoyl)-methyl]-carbamic acid tert-butyl ester (1.5 g, 5.65 mmol) in 40 ml 20% TFA in CH₂Cl₂ was stirred for 30 min, and concentrated in vacuo. The residue was washed with ether twice, and lyophilized to give a white powder (1.58g, 80%). mp: 87-88°C. ¹H NMR (D₂O, 300 MHz) δ 8.58-8.76 (m, 2H), 8.35-8.42 (m, 1H), 7.88-8.02(m, 2H), 4.52-4.6 (s, 2H), 3.68-3.72(s, 2H). HRMS (ESI-TOF) Calcd for C₈H₁₂N₃O (MH⁺) 166.0975. Found 166.0971.

### Example 3H

### 2-amino-N-(3-fluoro-5-trifluoromethylbenzyl)acetamide (a compound embraced by formula IX-a)

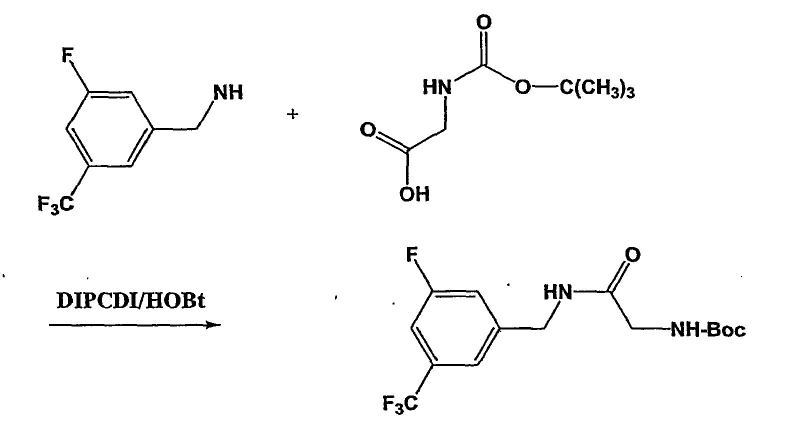

Synthesis of [(3-Fluoro-5-trifluoromethyl-benzylcarbamoyl)-methyl]-carbamic acid tert-butyl ester: A solution of Boc-Gly-OH (0.36 g, 2 mmol), 1,3-diisopropylcarbodiimide (0.42 mL, 2.6 mmol), and HOBt (0.36 g, 2.6 mmol) in DMF (15 mL) was stirred at room temperature for 5 mins, then was treated with 3-fluoro-5-trifluoromethyl-benzylamine (0.3 mL, 2 mmol). The resulting reaction mixture was stirred at room temperature overnight. The solid formed was filtered off, and the filtrate was washed with H₂O (2 x 50 mL), saturated NaHCO₃ (2 x 50 mL), and brine (100 mL). The organic layer was dried over Na₂SO₄, and filtered. The filtrate was concentrated *in vacuo.* The residue was purified on column chromatography (silica gel, 40% EtOAc/hexanes) to provide the pure product (0.69 g, 96%). ¹H NMR (CDCl₃, 300MHz) δ 1.41 (s, 9H), 3.83 (d, J = 6.3 Hz, 2H), 4.49 (d, J = 6.3 Hz, 2H), 5.07-5.24 (br s, 1H), 6.72-6.88 (br s, 1H), 7.16-7.41 (m, 3H). ESMS *m*/*z* 373 (M+Na)⁺.

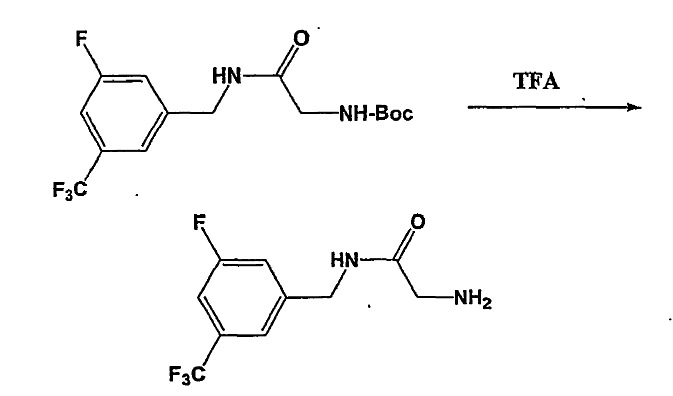

2-amino-N-(3-fluoro-5-trifluoromethylbenzyl)acetamide trifluoroacetate: A solution of [(3-fluoro-5- trifluoromethyl-benzylcarbamoyl)-methyl]-carbamic acid tert-butyl ester (0.68 g, 1.94 mmol) in 20% TFA in CH₂Cl₂ (40 mL) was stirred at room temperature for 30 mins, and concentrated *in vacuo.* The residue was washed with ether twice to give a white powder (0.69 g, 99%). mp: 189.5°C-190.5°C. ¹H NMR (D₂O, 300MHz) δ 3.71 (s, 2H), 4.34 (s, 2H), 7.11-7.36 (m, 3H). ESMS *m*/*z* 251 (M+H)⁺. Calcd. for C₁₂H₁₁F₇N₂O₃: C: 39.57; H: 3.04; N: 7.69. Found: C: 39.32; H: 3.36; N: 7.72.

### Example 3I

### 3-[2-(3-chlorophenyl)ethyl]-3-pyrroline, a compound embraced by formula III

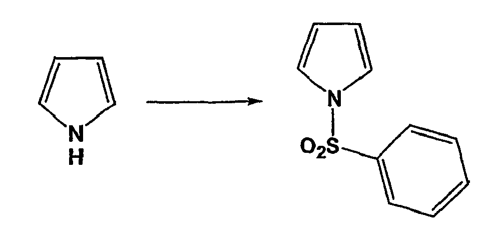

*1-Phenylsulfonylpyrrole.* A solution of benzenesulfonyl chloride (1.92 mL, 15.0 mmol) in toluene (15.0 mL) was added over a period of 15 min to a mixture of pyrrole (0.69 mL), 10.0 mmol), tetrabutylammonium bisulfate (0.34 g, 1.0 mmol), and 50% aqueous sodium hydroxide (10.0 mL) in toluene (30.0 mL). The mixture was stirred at room temperature, and the reaction was monitored by thin layer chromatography. After stirring at room temperature for 3 h, TLC showed that the reaction was completed. The layers were separated, and the organic layer was washed with water and brine, dried over Na₂SO₄, filtered, and concentrated. The crude product was purified by flash column chromatography (silica gel, 30% CH₂Cl₂/hexane) to yield 1.81 g (87%) of a white solid. M.p. 86-87 °C; ¹H NMR (300 MHz, CDCl₃) δ 6.30 (m, 2H), 7.17 (m, 2H), 7.50 (m, 2H), 7.57 (m, 1H), 7.84 (m, 1H), 7.87 (m, 1H).

*3-(3-Chlorophenyl)acetyl-1-phenylsulfonylpyrrole.* A solution of 3-chlorophenylacetic acid (0.85 g, 5.0 mmol) and thionyl chloride (3.0 mL) in CH₂Cl₂ (17.0 mL) was heated to reflux for 3 h, then cooled to room temperature and added to a suspension of aluminum chloride (1.12 g, 8.4 mmol) in 1,2-dichloroethane (3.0 mL) stirring at room temperature. After 15 min, a solution of 1-phenylsulfonylpyrrole (0.87 g, 4.2 mmol) in 1,2-dichloroethane (3.0 mL) was added, and the reaction mixture was stirred at room temperature. The reaction was monitored by thin layer chromatography. After 2 h, the mixture was poured over ice water and extracted with CH₂Cl₂ (3 x 20 mL). The combined organic layers were washed with water (20 mL) and brine (20 mL), dried over Na₂SO₄, filtered, and concentrated. The crude product was purified by flash column chromatography (silica gel, EtOAc/hexane 15-30%) to afford 1.33 g (88%) of a white solid. M.p. 106-108 °C; ¹H NMR (300 MHz, CDCl₃) δ 4.00 (s, 2H), 6.70 (dd, *J*=1.6, 3.3 Hz, 1H), 7.12 (m, 1H), 7.14 (dd, *J* = 2.2, 3.3 Hz, 1H), 7.24 (m, 3H), 7.57 (m, 2H), 7.65 (m, 1H), 7.77 (t, *J*=1.9 Hz, 1H), 7.91 (m, 2H); MS (ESI) 381.8 (M⁺ + Na).

*2-(3-Chlorophenyl)ethyl-2,5-dihydro-1-phenylsulfonylpyrroline.* Solid sodium cyanoborohydride (0.50 g, 8.35 mmol) was added very slowly to trifluoroacetic acid (10.0 mL) at room temperature. After 15 min, the acylated pyrrole was added to the mixture, which was stirred for 1 h at room temperature. At this time, additional sodium cyanoborohydride (0.50 g, 8.35 mmol) was added very slowly, and the reaction mixture was stirred overnight. The reaction was quenched with water, then extracted with CH₂Cl₂ (3 x 20 mL). The combined organic layers were washed with saturated NaHCO₃ (30 mL) and brine (30 mL), dried over Na₂SO₄, filtered, and concentrated. The crude product was purified by flash column chromatography (silica gel, EtOAc/hexane 10-20%) to afford 0.28 g (29%) as a clear oil. ¹H NMR (300 MHz, CDCl₃) δ 2.29 (m, 2H), 2.67 (m, 2H), 4.03 (m, 2H), 4.09 (m, 2H), 5.28 (quint, *J* = 1.6 Hz, 1H), 6.94 (m, 1H), 7.08 (br s, 1H), 7.15 (m, 2H), 7.51-7.61 (m, 3H), 7.83 (m, 2H); MS (ESI) 348.0 (M⁺ + H⁺), 370.0 (M⁺ + Na).

*3-[2-(3-chlorophenyl)ethyl]-3-pyrroline hydrochloride.* Sodium is stirred with a solution of anthracene in anhydrous THF (50.0 mL) for 1 h. The solution becomes dark blue and all the sodium is consumed. To a solution of 2-(3-chlorophenyl)ethyl-2,5-dihydro-1-phenylsulfonylpyrroline in THF (5.0 mL) at 0 °C is added drop wise the solution of sodium anthracene. The mixture remains blue for 1min then water is added. The reaction mixture is extracted with ethyl acetate, and the combined organic extracts are dried over anhydrous Na₂SO₄, filtered, and concentrated. The product in neutral form is isolated via column chromatography (alumna, 2-5% MeOH/CH₂Cl₂) and is converted into HCl salt by treating with HCl/ether and recrystallization from ethanol/ether.

### Additional compounds embraced by formula III

By using the protocol above, but substituting 3-fluorophenylacetic acid in place of 3-chlorophenylacetic acid, 3-[2-(3-fluorophenyl)ethyl]-3-pyrroline hydrochloride (compound III-1) was produced. ¹H NMR (D₂O, 300 MHz) δ 2.38 (t, *J*= 5.7 Hz, 2H), 2.73 (t, *J*= 5.7 Hz, 2H), 3.83 (s, 2H), 3.87 (s, 2H), 5.38 (s, 1H), 6.83-7.01 (m, 3H), 7.18-7.23 (m, 1H). LCMS: *m*/*e* 192.1 (M⁺+1). Calcd for C₁₂H₁₅ClFNO: C; 63.30; H; 6.64; N; 6.15. Found: C; 63.42; H; 6.58; N; 6.20.

By using the protocol above, but substituting 4-fluorophenylacetic acid in place of 3-chlorophenylacetic acid, 3-[2-(4-fluorophenyl)ethyl]-3-pyrroline hydrochloride (compound **III**-2)was produced. ¹H NMR (D₂O, 300 MHz) δ 2.36 (t, J = 5.4 Hz, 2H), 2.69 (t, J = 5.4 Hz, 2H), 3.82 (s, 2H), 3.87 (s, 2H), 5.36 (s, 1H), 6.94 (t, *J*= 6.3 Hz, 2H), 7.13 (t, *J* = 6.3 Hz. 2H). LCMS: *m*/*e* 192.1 (M⁺+1). Calcd for C₁₂H₁₅ClFNO: C; 63.30; H; 6.64; N; 6.15. Found: C; 63.24; H; 6.81; N; 6.22.

By using the protocol above, but substituting 3-methoxyphenylacetic acid in place of 3-chlorophenylacetic acid, 3-[2-(3-methoxyphenyl)ethyl]-3-pyrroline hydrochloride (compound **III**-3) was produced. ¹H NMR (D₂O, 300 MHz) δ 2.37 (t, J = 5.4 Hz, 2H), 2.70 (t, J = 5.4 Hz, 2H), 3.69 (s, 3H), 3.82 (s, 2H), 3.87 (s, 2H), 5.37 (s, 1H), 6.71-6.83 (m, 3H), 7.18 (t, *J*= 6 Hz, 1H). LCMS: *m*/*e* 204.0 (M⁺+1). Calcd for C₁₃H₁₈ClNO: C; 65.13; H; 7.57; N; 5.84. Found: C; 65.02; H; 7.42; N; 5.89.

By using the protocol above, but substituting 4-methoxyphenylacetic acid in place of 3-chlorophenylacetic acid, 3-[2-(4-methoxyphenyl)ethyl]-3-pyrroline hydrochloride (compound **III**-4) was produced. ¹H NMR (D₂O, 300 MHz) δ 2.34 (t, *J*= 5.4 Hz, 2H), 2.65 (t, *J=* 5.4 Hz, 2H), 3.68 (s, 3H), 3.81 (s, 2H), 3.87 (s, 2H), 5.36 (br s, 1H), 6.83 (d, J = 5.1 Hz, 2H), 7.10 (d, J = 6.3 Hz, 2H). LCMS: *m*/*e* 204.0 (M⁺+1). Calcd for C₁₃H₁₈ClNO: C; 65.13; H; 7.57; N; 5.84. Found: C; 64.90; H; 7.86; N; 5.87.

By using the protocol above, but substituting 3,4-dimethoxyphenylacetic acid in place of 3-chlorophenylacetic acid, 3-[2-(3,4-dimethoxyphenyl)ethyl]-3-pyrroline hydrochloride (compound **III-5)** was produced. ¹H NMR (D₂O, 300 MHz) δ 2.56 (t, *J*= 7.5 Hz, 2H), 2.86 (t, *J*= 7.5 Hz, 2H), 3.89 (s, 3H), 3.91 (s, 3H), 4.01 (s, 2H), 4.08 (s, 2H), 5.57 (br s, 1H), 6.93 (dd, J = 2.1, 8.4 Hz, 1H), 6.98-7.09 (m, 2H). LCMS: *m*/*e* 234.4
(M⁺+1). Calcd for C₁₄H₂₀ClNO₂*0.7 H₂O: C; 59.55; H; 7.64; N; 96. Found: C; 59.40; H; 7.65; N; 4.83.

The compounds above (III-1, III-2, III-3, III-4, III-5) were evaluated by the radiolabeled benzylamine procedure in Example 4 for their ability to inhibit SSAO. The results are shown in Example 22 and Table II.

### Example 3J

### Compounds of general formula III where n3b = 2 and R₁₄ = O

Using the general method described in the specification, the following compounds were prepared:

*3-[(3-fluorophenoxy)methyl]1,2,5,6-tetrahydropyridine hydrochloride* (compound **III-6)** (using 3-fluorophenol as the phenolic compound in the synthesis): ¹H NMR (D₂O, 300 MHz) δ 2.33 (br s 2H), 3.19 (t, *J*= 6.3 Hz, 2H), 3.65 (s, 2H), 4.50 (s, 2H), 6.01 (br s, 1H), 6.61-6.75 (m, 2H), 7.12-7.24 (m, 1H). LCMS: *m*/*e* 208.0 (M⁺+1). Calcd for C₁₂H₁₅ClFNO: C; 59.14; H; 6.20; N; 5.75. Found: C; 59.11; H; 6.20; N; 6.04.

*3-[(4-fluorophenoxy)methyl]1,2,5,6-tetrahydropyridine hydrochloride* ¹H (compound **III-7)** (using 4-fluorophenol as the phenolic compound in the synthesis): ¹H NMR (D₂O, 300 MHz) δ 2.11 (br s 2H), 3.19 (t, *J*= 6.0 Hz, 2H), 3.65 (s, 2H), 4.49 (s, 2H), 6.02 (br s, 1H), 6.86-6.96 (m, 2H), 6.97-7.03 (m, 2H). LCMS: *m*/*e* 208.0 (M⁺+1). Calcd for C₁₂H₁₅ClFNO: C; 59.14; H; 6.20; N; 5.75. Found: C; 58.92; H; 6.16; N; 5.96.

*3-[(3-methoxyphenoxy)methyl]1,2,5,6-tetrahydropyridine hydrochloride* (compound **III-8)** (using 3-methoxyphenol as the phenolic compound in the synthesis): ¹H NMR (D₂O, 300 MHz) δ 2.33 (br s 2H), 3.22 (t, *J*= 6.3 Hz, 2H), 3.65 (s, 2H), 3.70 (s, 3H), 4.49 (s, 2H), 6.02 (br s, 1H), 6.48-6.81 (m, 3H), 7.18 (t, *J*= 8.1 Hz, 1H). LCMS: *mle* 220.0 (M⁺+1). Calcd for C₁₃H₁₈ClNO₂: C; 61.05; H; 7.09; N; 5.48. Found: C; 60.54; H; 6.99; N; 5.67.

*3-[(4-methoxyphenoxy)methyl]1,2,5,6-tetrahydropyridine hydrochloride* (compound **III-9)** (using 4-methoxyphenol as the phenolic compound in the synthesis): ¹H NMR (D₂O, 300 MHz) δ 2.33 (br s 2H), 3.22 (t, *J=* 6.3 Hz, 2H), 3.64 (s, 2H), 3.67 (s, 3H), 4.45 (s, 2H), 5.99 (br s, 1H), 6.80-6.91 (m, 4H). LCMS: *mle* 220.0 (M⁺+1). Calcd for C₁₃H₁₈ClNO₂: C; 61.05; H; 7.09; N; 5.48. Found: C; 60.65; H; 7.09; N; 5.64.

*3-[(3,4-dimethoxyphenoxy)methyl]1,2,5,6-tetrahydropyridine hydrochloride(compound* **III-10)** (using 3,4-dimethoxyphenol as the phenolic compound in the synthesis): ¹H NMR (D₂O, 300 MHz) δ 2.31 (br s 2H), 3.19 (t, *J*= 6.3 Hz, 2H), 3.64 (s, 2H), 3.69 (s, 3H), 3.72 (s, 3H), 4.45 (s, 2H), 6.00 (br s, 1H), 6.48 (dd, *J*= 2.7, 8.7 Hz, 1H), 6.61 (d, *J*= 3.0 Hz, 1H), 6.86 )d, *J*= 8.7 Hz, 1H). LCMS: *m*/*e* 250.0 (M⁺+1). Calcd for C₁₃H₁₈ClNO₂: C; 58.84; H; 7.05; N; 4.90. Found: C; 58.76; H; 6.98; N; 5.14.

The compounds above (III-6, III-7, III-8, III-9, III-10) were evaluated by the radiolabeled benzylamine procedure in Example 4 for their ability to inhibit SSAO. The results are shown in Example 22 and Table II.

### Example 4

### In vitro inhibition of SSAO activity

SSAO activity was also measured as described (Lizcano JM. Et al. (1998) Biochem J. 331:69). Briefly, rat lung homogenates were prepared by chopping the freshly removed tissue into small pieces and washing them thoroughly in PBS. The tissue was then homogenized 1:10 (w/v) in 10 mM potassium phosphate buffer (pH 7.8) and centrifuged at 1000g at 4°C for 10 minutes; the supernatant was kept frozen until ready to use. SSAO activity in 100 ul of lung homogenate was determined radiochemically using 20 uM ¹⁴C-benzylamine as substrate. The reaction was carried out at 37°C in a final volume of 300 ul of 50 mM potassium phosphate buffer (pH 7.2) and stopped with 100 ul of 2 M citric acid. Radioactively labeled products were extracted into toluene/ethyl acetate (1:1, v/v) containing 0.6% (w/v) 2,5-diphenyloxdazole (PPO) before liquid scintillation counting.

SSAO activity can also be measured using the coupled colorimetric method essentially as described for monoamine oxidase and related enzymes (Holt A. et al. (1997) Anal. Biochem. 244:384). Bovine plasma amine oxidase (PAO) (Worthington Biochemical, Lakewood, NJ) is used as a source of SSAO for activity measurements. The SSAO assay is performed in 96 well microtitre plates as follows. A pre-determined amount of inhibitor diluted in 0.2 M potassium phosphate buffer, pH 7.6, is added to each well, if required. The amount of inhibitor varies in each assay but is generally at a final concentration of between 10 nM and 10 µM. Controls lack inhibitor. In order to study the effects of potential inhibitors, 50 µl of inhibitor solution are preincubated for 30 min at 37°C with 0.4 mU of PAO in a total volume of 130 µl of 0.2 M potassium phosphate buffer pH 7.6. Assays are then started by addition of 20 µl 10 mM benzylamine substrate and incubated for 20 min at 37°C. The following reagents are then added to a final reaction volume of 200 µl, 50 µl of freshly made chromogenic solution containing 750 nM vanillic acid (Sigma # V-2250), 400 nM 4-aminoantipyrine (Sigma # A-4328) and 12 U/ml horseradish peroxidase (Sigma # P-8250) in order to cause a change of 0.5 OD A490 per hour. This is within the linear response range of the assay. The plates are incubated for 1 hr at 37°C and the increase in absorbance, reflecting SSAO activity, is measured at 490 nm using a microplate spectrophotometer (Power Wave 40, Bio-Tek Inst.). Inhibition is determined as percent inhibition compared to control after correcting for background absorbance and IC₅₀ values, and is calculated using GraphPad Prism software.

### Example 5

### Comparison of inhibition of the SSAO activity of SSAO/VAP-1 versus MAO-A and MAO-B activities.

The specificity of the different SSAO inhibitors was tested by determining their ability to inhibit MAO-A and MAO-B activities *in vitro.* Recombinant human MAO-A and human MAO-B enzymes were obtained from BD Biosciences (MA, USA). MAO activities were measured in a similar way as for SSAO except that no preincubation with inhibitor or substrate was performed. A pre-determined amount of inhibitor diluted in 0.2 M potassium phosphate buffer, pH 7.6, was added to each well, if required. The amount of inhibitor varied in each assay but was generally at a final concentration of between 50 nM and 1 mM. Controls lacked inhibitor. The following agents were then added to a final reaction volume of 200 µl in 0.2 M potassium phosphate buffer, pH 7.6: 0.04 mg/ml of MAO-A or 0.07 mg/ml MAO-B enzyme, 15 µl of 10 mM tyramine substrate (for MAO-A), or 15 µl 100 mM benzylamine substrate (for MAO-B), and 50 µl of freshly made chromogenic solution (as above). The plates were incubated for 60 min at 37°C. The increase in absorbance, reflecting MAO activity, was measured at 490 nm using microplate spectrophotometer (Power Wave 40, Bio-Tek Inst.). Inhibition was determined as percent inhibition compared to control after correcting for background absorbance and IC₅₀ values, and was calculated using GraphPad Prism software. Clorgyline and pargyline (inhibitors of MAO-A and B, respectively) at 0.5 and 10 µM, respectively, were added to some wells as positive controls for MAO inhibition.

This procedure was used to screen for compounds which are specific inhibitors of SSAO activity. Example 23 provides data for several compounds of the invention.

### Example 6

### Acute Toxicity Studies

Oral (p.o.) and intravenous (i.v.) LD₅₀ values for the compounds of the invention, as well as mofegiline **(M1)** 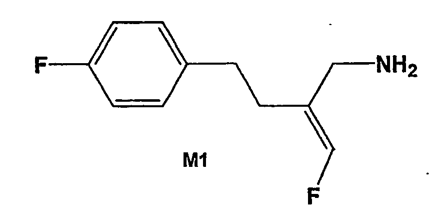 are determined in mice. Six-week old C57B1/6 female mice are divided in groups of five and administered a single i.v., p.o. or i.p. injection of compound dissolved in PBS (10-100 mg/kg in 100 µl i.v.; 30-1000 mg/kg p.o.; 30-500 mg/kg in 200 µl i.p.). Control groups are administered the same volume of PBS p.o. or i.v. Appearance and overt behavior are noted daily, and body weight is measured before compound administration (Day 1) and on Days, 3, 5 and 7. After seven days, animals are euthanized and their liver, spleen and kidneys are weighed.

### Example 7

### Inhibition of collagen-induced arthritis in mice

Collagen-induced arthritis (CIA) in mice is widely used as an experimental model for rheumatoid arthritis (RA) in humans. CIA is mediated by autoantibodies to a particular region of type II collagen and complement. The murine CIA model to be used in this study is called antibody-mediated CIA, and can be induced by i.v. injection of a combination of different anti-type II collagen monoclonal antibodies (Terato K., et al. (1995). Autoimmunity. 22:137). Several compounds have been used to successfully block inflammation in this model, including anti-α1β1 and anti-α2β2 integrins monoclonal antibodies (de Fougerolles A.R. (2000) J. Clin. Invest. 105: 721).

In this example, arthrogen-collagen-induced arthritis antibody kits are purchased from Chemicon International (Temecula, CA) and arthritis is induced using the manufacturer's protocol. Mice are injected i.v. with a cocktail of 4 anti-collagen Type II monoclonal antibodies (0.5 mg each) on day 0, followed by i.p. injection of 25 µg lipopolysaccharide (LPS) on day 3. Mice will develop swollen wrists, ankles, and digits 3-4 days after LPS injection, with disease incidence of 90% by day 7. Severity of arthritis in each limb is scored for 12 days as follows: 0 = normal; 1= mild redness, slight swelling of ankle or wrist; 2 = moderate redness and swelling of ankle or wrist; 3 = severe redness and swelling of some digits, ankle and paw; 4 = maximally inflamed limb. Animals are divided in 3 groups of 6 animals: vehicle, methotrexate (MTX)-treated, and compound-treated. Animals in the vehicle group are injected i.p. with phosphate buffer saline (PBS), twice daily for 12 days (starting on day 0). MTX (3 mg/kg) is administered i.p. starting on day 0 and continuing three times a week (Mon., Weds., Fri.) for the duration of the experiment.

### Example 8

### Inhibition of experimental autoimmune encephalomyelitis in mice by SSAO inhibitors-mofegiline (allylamine compound)

SSAO/VAP-1 is expressed on the endothelium of inflamed tissues/organs including brain and spinal cord. Its ability to support lymphocyte transendothelial migration may be an important systemic function of SSAO/VAP-1 in inflammatory diseases such as multiple sclerosis and Alzheimer's disease. An analysis of the use of SSAO inhibitors to treat inflammatory disease of the central nervous system (CNS) was performed through the use of an experimental autoimmune encephalomyelitis model (EAE) in C57BL/6 mice. EAE in rodents is a well-characterized and reproducible animal model of multiple sclerosis in human (Benson J.M. et al. (2000) J. Clin. Invest. 106:1031). Multiple sclerosis is a chronic immune-mediated disease of the CNS characterized by pachy perivenular inflammatory infiltrates in areas of demyelination and axonal loss. As an animal model, EAE can be induced in mice by immunization with encephalitogenic myelin antigens in the presence of adjuvant. The pathogenesis of EAE comprises presentation of myelin antigens to T cells, migration of activated T cells to the CNS, and development of inflammation and/or demyelination upon recognition of the same antigens.

To examine the role of SSAO/VAP-1 as a major regulator of the lymphocyte recruitment to the CNS, mofegiline (**M1**), an allylamine and SSAO inhibitor, was evaluated in an EAE model.

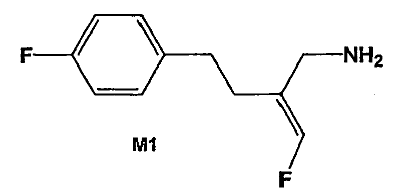

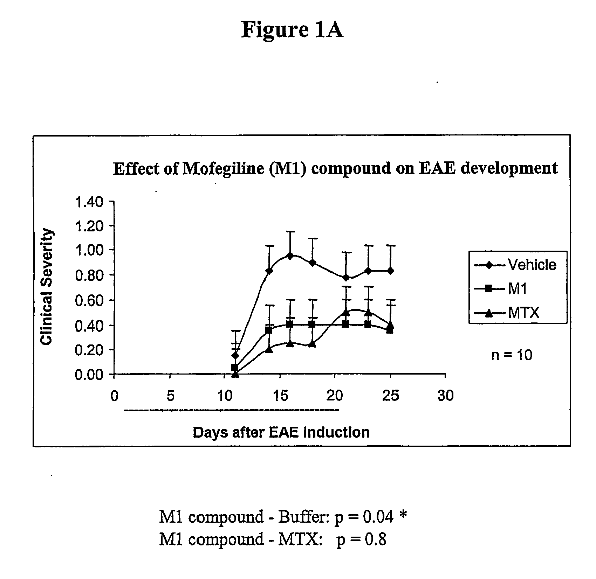

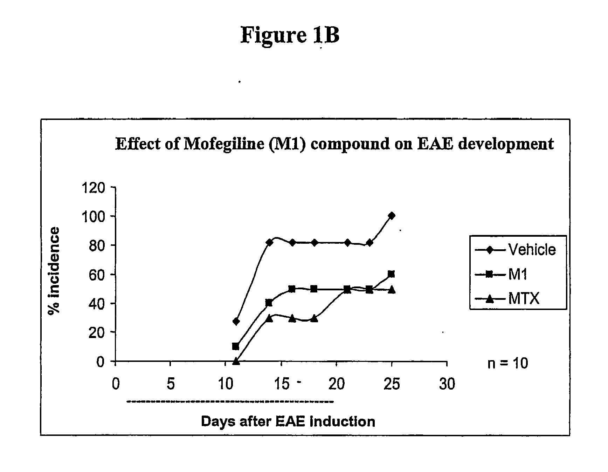

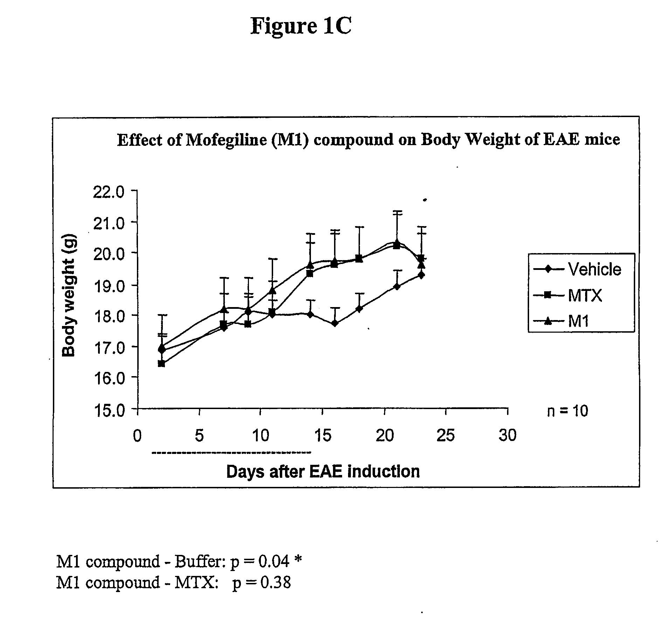

Thirty female C57BL/6 mice were immunized subcutaneously (s.c). with myelin oligodendrocyte glycoprotein 35-55 (MOG peptide 35-55) in Complete Freund Adjuvant (CFA) on day 0, followed by i.p. injections of pertussis toxin (one pertussis toxin injection on day 0, a second pertussis toxin injection on day 2). Groups of 10 mice received either the allylamine compound mofegiline (**M1**, 10 mg/kg/dose, twice daily for 18 consecutive days), methotrexate (MTX, 2.5 mg/kg/day, every other day (Mon., Weds., Fri.) till day 18) or vehicle control (twice/day for 18 consecutive days) all starting from one day after the immunization and all administered i.p. Then animals were monitored for body weight, signs of paralysis and death according to a 0-5 scale of scoring system as follows: 1 = limp tail or waddling gait with tail tonicity; 2 = waddling gait with limp tail (ataxia); 2.5 = ataxia with partial limb paralysis; 3 = full paralysis of one limb; 3.5 = full paralysis of one limb with partial paralysis of second limb; 4 = full paralysis of two limbs; 4.5 = moribund; 5 = death. Results are shown in Fig. 1A, Fig. 1B, and Fig. 1C. Compared with the vehicle-treated group during the dosing period (up to day 18), that showed an 80% disease incidence and moderate clinical severity; mofegiline-treated mice resulted in a statistically significant reduction of disease severity with 50% of mice affected. (p = 0.04 by repeated measure analysis to assess the treatment effect. Proper polynomial transformation, with the spacing corresponding to the collection days, was applied to test the time effect). Statistically significant differences in diseases severity between the Ml and vehicle-treated groups, continued even after stopping compound administration and were observed until the end of the study (d25).

As expected, the loss of body weight is correlated with the clinical severity in vehicle-control mice; and mofegiline treatment also prevented body weight loss in the mice during the dosing period (p = 0.04). In addition, the inhibitory effect of mofegiline on the EAE development was continuously observed for at least one more week after the last treatment (d19-25). MTX-treated mice exhibited a similar inhibitory effect during the treatment period (d0-18). However, a rise in disease incidence and severity was observed right after stopping the MTX treatment (Fig. 1A). There was no statistically significant difference (p = 0.8 and p = 0.38, for clinical severity and body weight, respectively) between the groups treated with MTX and mofegiline during or after the dosing period.

### Example 8B

### Inhibition of relapsing experimental autoimmune encephalomyelitis in mice by VAP-1/SSAO inhibitor (model of chronic multiple sclerosis).

An analysis of the use of VAP-1/SSAO inhibitors to treat inflammatory diseases of the CNS is performed through the use of a relapsing experimental autoimmune encephalomyelitis model (EAE) in SJL/J mice. Relapsing EAE in mice is a well-characterized and reproducible animal model of multiple sclerosis in humans (Brown & McFarlin 1981 Lab. Invest. 45:278-284; McRae et al 1992 J. Neuroimmunol. 38:229-240). Multiple sclerosis is a chronic immune-mediated disease of the CNS characterized by pachy perivenular inflammatory infiltrates in areas of demyelination and axonal loss. As an animal model, chronic relapsing EAE can be induced in mice by immunization with encephalitogenic myelin antigen in the presence of adjuvant. The pathogenesis of EAE comprises presentation of myelin antigens to T cells, migration of activated T cells to the CNS, and development of inflammation and/or demyelination upon recognition of the same antigens.

Vascular adhesion protein-1 (VAP-1) is an amine oxidase and adhesion receptor that is expressed on the endothelium of inflamed tissues/organs including brain and spinal cord. Its ability to support lymphocyte transendothelial migration may be an important systemic function of VAP-1 in inflammatory disorders such as multiple sclerosis and Alzheimer's disease.

To examine the role of VAP-1 as a major regulator of lymphocyte recruitment to the CNS, VAP-1/SSAO inhibitors are evaluated in a chronic relapsing EAE model. Twenty 7-8 week old female SJL/T mice are immunized s.c. with 50 µg of mouse PLP peptide 139-151 in Complete Freund Adjuvant (CFA), followed by two i.p. injections of 200 ng pertussis toxin. Groups of 10 mice are to receive i.p. either vehicle control (PBS, 0.1 ml) or a VAP-1/SSAO inhibitor, bid for 53 consecutive days, all starting from one day after the immunization.

Animals are monitored for signs of paralysis according to a 0-5 scale of scoring system as follows:

| | |
|---|---|
| 0.5 | partial tail weakness |
| 1 | limp tail or waddling gait with tail tonicity; |
| 1.5 | waddling gait with partial tail weakness |
| 2 | waddling gait with limp tail (ataxia); |
| 2.5 | ataxia with partial limb paralysis; |
| 3 | full paralysis of one limb; |
| 3.5 | full paralysis of one limb with partial paralysis of second limb; |
| 4 | full paralysis of two limbs; |
| 4.5 | moribund; |
| 5 | death. |

### Example 9

### Inhibition of carrageenan-induced rat paw edema

Carrageenan-induced paw edema has been extensively used in the evaluation of anti-inflammatory effects of various therapeutic agents and is a useful experimental system for assessing the efficacy of compounds to alleviate acute inflammation (Whiteley PE and Dalrymple SA, 1998. Models of inflammation: carrageenan-induced paw edema in the rat, in Current Protocols in Pharmacology. Enna SJ, Williams M, Ferkany JW, Kenaki T, Porsolt RE and Sullivan JP, eds., pp 5.4.1-5.4.3, John Wiley & Sons, New York). The full development of the edema is neutrophil-dependent (Salvemini D. et al. (1996) Br. J. Pharmacol. 118: 829).

Female Sprague Dawley rats were used and compounds of the invention were injected i.p. or dosed orally prior to carrageenan exposure. An equal volume of vehicle (PBS) was administered to the control group. Edema in the paws was induced as previously described by injecting 50 µl of a 0.5% solution of carrageenan (Type IV Lambda, Sigma) in saline with a 27-G needle s.c. in the right foot pat. (See Whiteley P.E. and Dalrymple S.A. (1998), Models of inflammation: carrageenan-induced paw edema in the rat, in Current Protocols in Pharmacology. Enna SJ, Williams M, Ferkany JW, Kenaki T, Porsolt RE and Sullivan JP, eds., pp 5.4.1-5.4.3, John Wiley & Sons, New York) The size of the tested foot of each animal was measured volumetrically, before induction of edema, and at 1, 2, 3, 4, and 6 hours after carrageenan induction, to screen for compounds which inhibit the development of edema as compared to control animals.

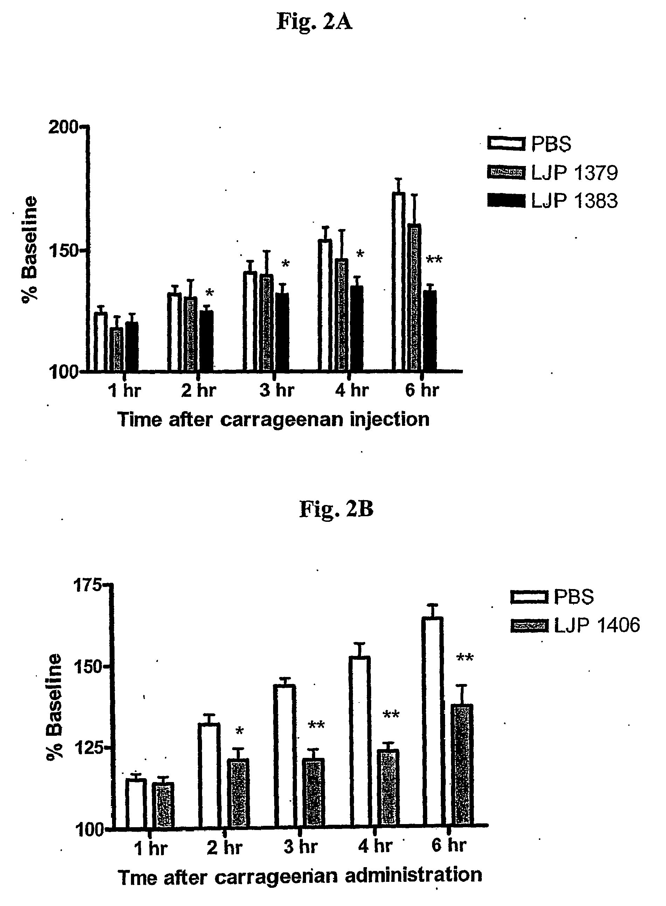

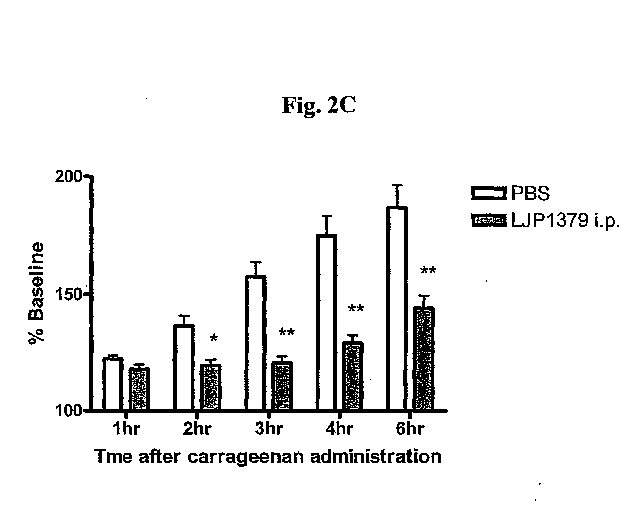

The results of an experiment where three compounds of Example 21, Table I (LJP 1379, LJP 1383, LJP 1406) were used are shown in Fig. 2A, Fig. 2B, and Fig. 2C. In Fig. 2A, animals were administered LJP 1379 (30 mg/kg, p.o.), LJP 1383 (30 mg/kg, p.o.), or PBS one hour before carrageenan injection. Paw volumes were recorded at the indicated times and expressed as percent of the volume before injection (100%). Data are shown as mean + SEM (n = 8). Statistical analysis was performed using one-way ANOVA followed by Dunnetts test, *p < 0.005, **p < 0.001. In Fig. 2B, animals were administered LJP 1406 (30 mg/kg, p.o.) or PBS one hour before carrageenan injection. Paw volumes were recorded at the indicated times and expressed as percent of the volume before injection (100%). Data are shown as mean + SEM (n = 8). Statistical analysis was performed using one-way ANOVA followed by Dunnetts test, *p < 0.005, **p < 0.001. In Fig. 2C, animals were administered LJP 1379 (30 mg/kg, i.p.) or PBS one hour before carrageenan injection. Paw volumes were recorded at the indicated times and expressed as percent of the volume before injection (100%). Data are shown as mean + SEM (n = 8). Statistical analysis was performed using one-way ANOVA followed by Dunnetts test, *p < 0.005, **p < 0.001. Data were analyzed with GraphPad Prism software (San Diego, Ca) by Dunnett's test following analysis of variance (p < 0.05).

Oral administration of LJP 1383 (Fig. 2A) and LJP 1406 (Fig. 2B) at 30 mg/kg significantly reduced the paw swelling at all time points tested. Administration of LJP 1379 was only effective when dosed i.p., (Fig. 2C; compare to Fig. 2A) suggesting that this compound may not be orally available in the manner used for oral administration in this experiment. These results indicate that these compounds of the invention inhibit the development of edema as compared to control animals, and can be further evaluated for use as anti-inflammatory therapeutics.

### Example 10

### Inhibition of chemically-induced colitis

2,4,6-trinitrobenzene sulfonic acid (TNBS)-induced colitis and dextran sodium sulfate (DSS)-induced colitis are TH1-mediated mouse models of colitis related to Crohn's disease. Compounds acting through various mechanisms have been demonstrated to be effective in these models, including prednisolone, anti IL-16, anti-ICAM, and anti-integrin, among many others (Strober W. et al (2002) Annu. Rev. Immunol. 20: 495). Oxazolone-induced colitis is a TH2-mediated process that closely resembles ulcerative colitis and is responsive to anti-IL4 therapy (Boirivant M. et al. (1998) J. Ex. Med 188: 1929).

TNBS colitis is induced as described (Fuss I.J. et al. (2002) J. Immunol. 168: 900). Briefly, 2.5 mg/ mouse of TNBS (pH 1.5-2, Sigma) in 50% ETOH is administered intrarectally in anesthetized SJL/J male mice through a 3.5 F catheter inserted 4 cm proximal to the anal verge. TNBS-injected mice are divided in three treatment groups and injected i.p. twice a day with: PBS; prednisolone (5 mg/kg) and a compound of the invention (at, e.g., 20 mg/kg). Injections are initiated at day 0 (day of TNBS injection) and are continued through day 7.

Oxazolone colitis is induced as described (Fuss I.J. et al. B (2002) J. Immunol. 168: 900). Briefly, mice are pre-sensitized by skin epicutaneous application of 3% oxazolone (4-ethoxymethylene-2-phenyl-2oxazolin-5-one, Sigma) in 100% EtOH (150 µl) on day 0, followed by intrarectal administration of 1% oxazolone in 50% EtOH (100 µl) to anesthetized SJL/J male mice on day 5 through a 3.5 F catheter inserted 4 cm proximal to the anal verge. Mice are divided in three treatment groups and injected i.p. twice a day with: PBS, prednisolone (5 mg/kg) and a compound of the invention (at, e.g., 20 mg/kg). Injections are initiated at day 0 and are continued through day 14.

Colitis is also induced by feeding Balb/c mice with 5% (wt/vol) DSS (ICN Biomedicals Inc., Ohio, USA) for 7 days as described (Okayasu I. et al. (1990) Gastroenterology 98: 694). Mice are divided in three treatment groups and injected i.p. twice a day with: PBS, prednisolone (5 mg/kg) and a compound of the invenvion (at, e.g., 20 mg/kg). Injections are initiated at day 0 (first day of DSS feeding) and are continued through day 7.

Disease progression is evaluated in all models by monitoring body weight, stool consistency, presence of blood in stool, histologic analysis of colon tissues sections, and monitoring levels of several cytokines.

This procedure is used to screen for compounds which inhibit the development of colitis as compared to control animals.

### Example 11

### Inhibition of concanavalin A-induced liver injury

Prevention of inflammation by administration of compounds of the invention is assessed in the concanavalin A (Con A) murine model of liver injury. Con A activates T lymphocytes and causes T cell-mediated hepatic injury in mice. Tumor necrosis factor alpha is a critical mediator in this experimental model. T-cell-mediated liver injury involves the migration of immune cells; notably CD4+ T lymphocytes, into liver tissue. Balb/c mice are inoculated with 10 mg/kg concanavalin A administered i.v. in 200 µl pyrogen-free saline as described (Willuweit A. et al. (2001) J Immunol. 167:3944). Previous to Con A administration, animals are separated into treatment groups and injected i.p with: PBS, and different concentrations of compound of the invention (e.g., 20 mg/kg). Liver damage is evaluated by determining serum levels of liver enzymes such as transaminase and alkaline phosphatase, hepatic histopathology, and levels of of different inflammatory cytokines in plasma and liver tissue.

This procedure is used to screen for compounds which inhibit the development of liver damage as compared to control animals.

### Example 12

### Effect of compounds of the invention in a mouse model of Alzheimer's disease

Alzheimer's disease (AD) is characterized clinically by a dementia of insidious onset and pathologically by the presence of numerous neuritic plaques and neurofibrillary tangles. The plaques are composed mainly of β-amyloid (Aβ) peptide fragments, derived from processing of the amyloid precursor protein (APP). Tangles consist of paired helical filaments composed of the microtubule-associated protein, tau. Transgenic mice carrying a pathogenic mutation in APP show marked elevation of Aβ-protein level and Aβ deposition in the cerebral cortex and hippocampus from approximately 1 year of age (Hsiao K. et al. (1996) Science 274:99). Mutant PS-1 transgenic mice do not show abnormal pathological changes, but do show subtly elevated levels of the Aβ42/43 peptide (Duff K, et al. (1996) Nature 383:710). Transgenic mice derived from a cross between these mice (PS/APP) show markedly accelerated accumulation of Aβ into visible deposits compared with APP singly transgenic mice (Holcomb L. et al. (1998) Nat Med 4:97). Further, a recent study indicates that in these mice, inflammatory responses may be involved in the Aβ depositions (Matsuoka Y. et al. (2001) Am J Pathol. 158(4):1345).

The PS/APP mouse, therefore, has considerable utility in the study of the amyloid phenotype of AD and is used in studies to assess efficacy of the compounds of the invention to treat Alzheimer's patients. Mice are injected with vehicle (e.g., PBS) or a compound of the invention (at, e.g., 10-20 mg/kg), and are evaluated by analysis of memory deficits, histological characteristics of sample tissues, and other indicators of disease progression.

### Alternate Alzheimer's model: Assessing efficacy in amyloid-B-induced autoimmune encephalitis

The abnormal processing and extracellular deposition of amyloid-B (Aβ) peptide, is a defining characteristic of Alzheimer's disease (AD). Recent evidence suggests that vaccination of transgenic mouse models of AD with Aβ causes a marked reduction in brain amyloid burden (e.g. Schenk D et al. (1999) Nature 400:173). Moreover, a recently published report suggests that vaccination with Aβ can, in certain circumstances, determine an aberrant autoimmune reaction to Aβ within the CNS, resulting in a perivenular inflammatory encephalomyelitis (Furlan R et al (2003) Brain 126:285).

Evaluation of the efficacy of compounds of the invention is carried out in the Aβ-induced autoimmune encephalomyelitis model. Thirty female C57BL/6 mice are immunized subcutaneously (s.c). with 100 µg of Aβ1-42 peptide in Complete Freund Adjuvant (CFA) on day 0, followed by i.p. injections of pertussis toxin (one pertussis toxin injection on day 0, a second pertussis toxin injection on day 2). Groups of 10 mice receive either a compound of the invention (10 mg/kg/dose, twice daily for 18 consecutive days), methotrexate (2.5 mg/kg/day, three times a week, till day 18) or vehicle control (twice/day for 18 consecutive days), all starting from one day after the immunization and all administered i.p. Then animals are monitored for body weight, signs of paralysis and death according to a 0-5 scale of scoring system as follows: 1 = limp tail or waddling gait with tail tonicity; 2 = waddling gait with limp tail (ataxia); 2.5 = ataxia with partial limb paralysis; 3 = full paralysis of one limb; 3.5 = full paralysis of one limb with partial paralysis of second limb; 4 = full paralysis of two limbs; 4.5 = moribund; 5 = death.

### Example 13

### Effect of compounds of the invention in murine models of Type I diabetes mellitus

It is widely accepted that proinflammatory cytokines play an important role in the development of type 1 diabetes. Thus, compounds of the invention can be used to treat patients suffering from this disease. A mouse with diabetes induced by multiple low doses of streptozotocin (STZ) can be used as an animal model for type 1 diabetes. STZ is used to induce diabetes in C57BL/6J mice. Briefly, STZ (40 mg/kg) or citrate buffer (vehicle) is given i.p. once daily for 5 consecutive days as described (Carlsson P.O. et al. (2000) Endocrinology. 141(8):2752). Compound administration (i.p. 10 mg/kg, twice a day) is started 5 days before STZ injections and continues for 2 weeks. Another widely used model is the NOD mouse model of autoimmune type 1 diabetes (Wong F.S. and Janeway C.A. Jr. (1999) Curr Opin Immunol. 11(6):643. Female NOD mice are treated with daily injections of a compound of the invention (20 mg/kg/day) from week 10 through week 25. The effect of the compounds of the invention in preventing the development of insulitis and diabetes in NOD-scid/scid females after adoptive transfer of splenocytes from diabetic NOD females is also assessed. For both the STZ and NOD models, the incidence of diabetes is monitored in several ways, including monitoring of blood glucose levels. Insulin secretion is assessed in pancreatic islets isolated from experimental mice. Cytokine production is measured in mouse sera. Islet apoptosis is assessed quantitatively.

This procedure is used to screen for compounds which inhibit development of diabetes as compared to control animals.

### Example 14

### Effect of compounds of the invention in models of airway inflammation.

Anti-inflammatory compounds such as SSAO inhibitors can have beneficial effects in airway inflammatory conditions such as asthma and chronic obstructive pulmonary disease. The rodent model here described has been extensively used in efficacy studies. Other murine models of acute lung inflammation can also be used to test the compounds of the invention.

For the evaluation of the effects of SSAO inhibitors in preventing airway inflammation, three groups of sensitized rats are studied. Animals are challenged with aerosolized OVA (ovalbumin) after intraperitoneal administration of the vehicle saline, a compound of the invention, or a positive control (e.g. prednisone) twice daily for a period of seven days. At the end of the week animals are anesthetized for measurements of allergen-induced airway responses as described (Martin J.G. et al. (2002) J Immunol. 169(7):3963). Animals are intubated endotracheally with polyethylene tubing and placed on a heating pad to maintain a rectal temperature of 36°C. Airflow is measured by placing the tip of the endotracheal tube inside a Plexiglas box (-250 ml). A pneumotachograph coupled to a differential transducer is connected to the other end of the box to measure airflow. Animals are challenged for 5 min with an aerosol of OVA ( 5% w/v). A disposable nebulizer will be used with an output of 0.15 ml/min. Airflow is measured every 5 min for 30 min after challenge and subsequently.at 15-min intervals for a total period of 8 h. Animals are then sacrificed for bronchoalveolar lavage (BAL). BAL is performed 8 h after challenge with five instillations of 5 ml of saline. The total cell count and cell viability is estimated using a hemacytometer and trypan blue stain. Slides are prepared using a Cytospin and the differential cell count is assessed with May-Grünwald-Giemsa staining, and eosinophil counts by immunocytochemistry.

### Alternate model of airway inflammation: assessing the effect of compounds of the invention

LPS-induced pulmonary inflammation in rats is a widely used model of airway inflammation (e.g. Billah M et al J. Pharmacol. Exp. Ther. (2002) 302.:127). Animals fasted overnight are orally dosed with either a compound of the invention (30 mg/kg), or vehicle 2 h before the LPS challenge. Using a Penn-Centry microspray needle, 0.1 ml of a 100-µg/ml LPS solution in saline is injected into the trachea of anesthetized male Sprague-Dawley rats (250-300 g). Animals not challenged with the LPS solution receive 0.1 ml of saline. Afterward, all animals are returned to their cages and allowed food and water ad libitum. At appropriate time points after intratracheal challenge with LPS, animals are surgically prepared with a tracheal cannula. Surgery is performed under anesthesia. The airways are flushed with 2 × 2 ml of 0.9% saline and the two washings pooled.

Lavage fluid is centrifuged (350g, 4°C, 7 min), the supernatant is aspirated, erythrocytes are lysed, and the white cell pellet is washed three times in phosphate-buffered saline containing 10% heat-inactivated fetal calf serum and 10 µg/ml DNase I. After the washes, the pellet is resuspended again in the same buffer. Total cell counts are performed using a hemacytometer. Differential cell counts are conducted on Cytospin-prepared slides stained with Fisher's Leukostat stain. At least 200 cells are assessed per slide using standard morphological criteria to define mononuclear, neutrophilic, and eosinophilic cells.

### Example 15

### Efficacy in model of systemic inflammation

Evaluation of the efficacy of compounds of the invention is carried out in a model of endotoxemia (Pawlinski R et al. (2003) Blood 103:1342). Sixteen female C57Bl/6 mice (eight to ten weeks old) are divided in two treatment groups: group A animals are administered 500 µl of PBS orally; group B animals are administered 100 mg/kg of LJP 1207 in 500 µl of PBS orally. Thirty minutes after oral administration of compound, inflammation is induced in all animals by administering i.p. 5 mg/kg of LPS (O111:B4, Sigma) in PBS. Blood samples (- 50 µl) are collected from the retro-orbital sinus at 0 (before oral administration of compound), 1, 2, 4, and 8 hrs after LPS injection. Each sample is immediately diluted ½ in PBS. Half of the diluted sample is used to prepare blood smear and the other 50 µl is centrifuged and serum is collected. Sera samples are used to determine IL1, IL6 and TNFa levels by ELISA. Animal survival rates are recorded for the next 3 days.

### Example 16

### Inhibition of cutaneous inflammation in the SCID mouse model of psoriasis

Recent establishment of the SCID-human skin chimeras with transplanted psoriasis plaques has opened new vistas to study the molecular complexities involved in psoriasis. This model also offers a unique opportunity to investigate various key biological events such as cell proliferation, homing in of T cells in target tissues, inflammation and cytokine/chemokine cascades involved in an inflammatory reaction. The SCID mouse model has been used to evaluate the efficacy of several compounds for psoriasis and other inflammatory diseases (Boehncke W.H. et al. (1999) Arch Dermatol Res. 291(2-3):104).

Transplantations are to be done as described previously (Boehncke, W.H. et al. (1994) Arch. Dermatol. Res. 286:325). Human full-thickness xenografts are transplanted onto the backs of 6- to 8-week-old C.B 17 SCID mice (Charles River). For the surgical procedure, mice are anesthetized by intraperitoneal injection of 100 mg/kg ketamine and 5 mg/kg xylazine. Spindle-shaped pieces of full-thickness skin measuring 1 cm in diameter are grafted onto corresponding excisional full-thickness defects of the shaved central dorsum of the mice and fixed by 6-0 atraumatic monofilament sutures. After applying a sterile petroleum jelly-impregnated gauze, the grafts are protected from injury by suturing a skin pouch over the transplanted area using the adjacent lateral skin. The sutures and over-tied pouches are left in place until they resolve spontaneously after 2-3 weeks. Grafts are allowed 2 weeks for acceptance and healing. Thereafter, daily intraperitoneal injections are performed between days 15 and 42 after transplantation. Mice are injected with either vehicle (PBS), dexamethasone (0.2 mg/kg body weight), or a compound of the invention (at, e.g., 20 mg/kg body weight) in a final volume of 200 µl. Mice are sacrificed at day 42, and after excision with surrounding mouse skin the grafts are formalin-embedded. Subsequently, routine hematoxylin-and-eosin staining is performed, and the grafts are analyzed with regard to their pathological changes both qualitatively (epidermal differentiation, inflammatory infiltrate) and quantitatively (epidermal thickness).

### Example 17

### Oral bioavailability studies in rodents

Oral bioavailability studies in mice and rats are to be performed using the following procedure. Briefly, C57B1/6 female mice and Sprague Dawley female rats are administered 50 mg/kg of different compounds of the invention by oral gavage. Animals are bled at different time intervals after compound administration and the levels of inhibitor in plasma are determined using the colorimetric assay described in Example 4.

### Example 18

### Dose-response effect from in vivo administration of SSAO/VAP-1 inhibitors

In vivo inhibition of SSAO is assessed in rat aorta and lungs, two of the tissues where SSAO activity is highest. Six week old female Sprague Dawley rats are to be administered 0, 0.1, 1, 10 and 50 mg/kg of a compound of the invention in 2.5 ml/kg PBS by oral gavage. Four hours after compound administration the animals are euthanized and their aortas and lungs are removed and frozen in liquid nitrogen. Tissues are homogenized in 0.1 M potassium phosphate pH 7.8 buffer (30 ml/g for aorta and 20 ml/g for lung) and centrifuged at 1000 x g for 15 min. Supernatants are collected and used in the radioactive assay following the protocol described by Lizcano J.M. et al. (1998) Biochem. J. 331:69. Enzymatic reactions are initiated by incubating a 200 µl aliquot of the tissue homogenate with 20 µl of 0.4 mM ¹⁴C-labeled benzylamine substrate (6 mCi/mmol specific activity, Pharmacia) for 30 min at RT. The assay is stopped by addition of 100 µl of 2 M citric acid, the assay volume is extracted with 5 ml toluene:ethyl acetate (1:1) containing 0.6% (w/v) 2,5-diphenyloxdazole (PPO), and an aliquot of the organic layer is counted by liquid scintillation. Because SSAO and MAO-B are both active towards benzylamine, control samples are run concomitantly so that MAO-B and SSAO activities can be identified. SSAO is inhibited with 0, 10, 50 and 500 µM of semicarbazide for MAO-B determinations, and MAO-B is inhibited with 0, 5, and 100 µM of pargyline for SSAO determinations. The inhibitors are added to the tissue supernatant prior to addition of benzylamine.

### Example 19

### Blocking of in vitro adhesion by SSAO/VAP-1 inhibitors.

These studies are carried out in order to determine whether SSAO/VAP-1 transfected into endothelial cells will retain the adhesion function and whether it plays any role in the adhesion of freshly isolated human PBMCs to these cells. Moreover, the studies are also designed to determine whether blocking of SSAO/VAP-1 will have an impact on the level of adhesion between these two cell types. Adhesion assays are performed using cells labeled with the fluorescent dye Calcein-AM (Molecular Probes, OR, USA) as per the manufacturer's instructions. Briefly, rat lymph node high endothelial cells (HEC; isolation and culture is described in Ager, A. (1987) J. Cell Sci. 87: 133) are plated overnight in 96-well plates (2,000 cells/well). PBMCs (peripheral blood mononuclear cells) (1x10⁷) are labeled with 1 ml of 10 µM Calcein-AM for 1 hr at 37°C, washed three times with RPMI, and added to the 96 well plates containing monolayers of HEC cells mock-transfected or transfected with full-length human SSAO/VAP-1 (60,000 PBMCs were plated per well containing 2,000 HEC cells). Adhesion is carried out for 3 hr at 37°C. Non-adherent cells are removed by washing three times with RPMI and fluorescence is measured in a fluorescence plate reader at an excitation wavelength of 485 nm and emission wavelength of 530 nm. Several controls are to be included, such as HEC cells and PBMCs (labeled and unlabeled) alone.

The next experiments are designed in order to investigate whether blocking the enzymatic catalytic site will have any effect on the adhesion function of SSAO/VAP-1, and whether or not inhibitors according to the invention will mediate an adhesion-inhibiting effect. Published results suggest that blocking SSAO enzymatic activity with semicarbazide inhibited lymphocyte rolling under laminar sheer on cardiac endothelial monolayers (Salmi et al. Immunity (2001) 14:265). These studies can thus be repeated using the adhesion assay as described above to evaluate the inhibitors of the invention. Adhesion blockers can include an anti-human VAP-1 monoclonal antibody (Serotec, Oxford,UK), neuramidase (a sialidase, because SSAO/VAP-1 is a sialoglycoprotein; Sigma), and several function-blocking antibodies to rat adhesion molecules (CD31- PECAM, CD54-ICAM-1, CD92P-P Selectin). Controls can include the SSAO inhibitor semicarbazide (Sigma), MAO-A and MAO-B inhibitors (clorgyline and pargyline, respectively; Sigma), and mouse IgG1 and IgG2 isotype controls (BD, USA). Antibodies (10 µg/ml) and neuramidase (5 mU) are incubated with the HECs for 30 min at 37°C; excess antibody is washed away prior to the addition of the labeled PBMCs. Small-molecule inhibitors are pre-incubated the same way at IC₁₀₀ concentrations, but the amounts present in the supernatant are not washed away to preserve the IC₁₀₀ concentration during the adhesion step.

### Example 20

### Inhibition of lipopolysaccharide (LPS)-induced endotoxemia

In sepsis exposure of endothelial cells of all organs to elevated levels of LPS and inflammatory cytokines leads to upregulation of adhesion molecules and chemokines, which results in an increase in the tethering, rolling and transmigration of leukocytes (Pawlinski R. et al. (2004) Blood 103:1342). LPS-induced endotoxemia is a well-characterized model of systemic inflammation and thus can be used to investigate the putative role of SSAO inhibition in these inflammatory mechanisms. Sepsis is to be induced in C57B1/6J female mice by i.p. administration of 5 mg/kg of LPS. Sixty minutes prior to LPS injections, 200 µl of vehicle (PBS) or 50 mg/kg of (2-phenylallyl)hydrazine are administered orally to the animals. Dexamethasone is administered i.p, at a concentration of 3mg/kg 1 hr prior to disease induction. Blood is drawn from the retroorbital plexus of anesthetized animals and sera is collected and frozen until time of cytokine measurements. IL-1β, TNF-α, and IL-6 concentrations are determined by ELISA using commercial kits (R&D Systems, Minneapolis, MN) according to the manufacturer's instructions.

### Example 21

### Inhibition of SSAO enzyme activity by compounds of formula X

Various compounds embraced by formula X were evaluated by the radiolabeled benzylamine procedure in Example 4. The results are shown below in Table I. Compounds LJP 1379 and LJP 1383 (both in Table I) are irreversible inhibitors of SSAO enzyme activity.

**Table I**

| In vitro biological data for compounds of formula X | | | | | | | | | | |
|---|---|---|---|---|---|---|---|---|---|---|
| 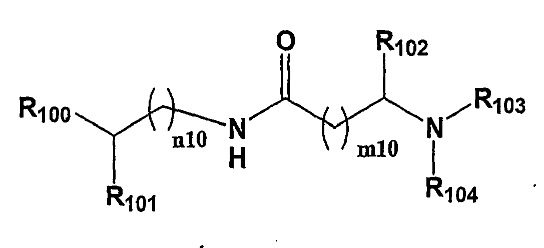 | | | | | | | | | | |

| **LJP** # | **n10** | **m10** | **R₁₀₀** | **R₁₀₁** | **R₁₀₂** | **R₁₀₃** | **R₁₀₄** | **mp (°C)** | **% inhibition** | **IC₅₀ (**µ**M)** |
|---|---|---|---|---|---|---|---|---|---|---|
| 1308 | 0 | 0 | 4-F-Ph | H | H | *i*-Pr | *i*-Pr | 220-221 | | inactive |
| 1311 | 0 | 0 | 4-F-Ph | H | H | Me | Me | 138-139 | | >100 |
| 1313 | 0 | 0 | 4-F-Ph | H | H | 4-F-Ph | H | 247-249 | | >100 |
| 1326 | 1 | 0 | Ph | OH | H | 4-Me-Ph | H | 218-219 | | inactive |
| 1327 | 1 | 0 | Ph | OH | H | Et | Et | | 2 (44 µM) | |
| 352 | 1 | 0 | Ph | OH | H | H | H | 148-149 | 32 (8 µM) | |
| 1319 | 1 | 0 | Ph | H | H | Me | Me | | 4 (50 µM) | |
| 1320 | 1 | 0 | Ph | H | H | Et | Et | 104-105 | | >100 |
| 1321 | 1 | 0 | Ph | H | H | benzyl | H | 248-249 | | inactive |
| 1351 | 1 | 0 | Ph | H | H | H | H | 141-142 | 28 (9 µM) | |
| 1334 | 0 | 0 | 4-F-Ph | H | H | H | H | 114-115 | | 2.4 |
| 1356 | 0 | 0 | 3-pyridyl | H | H | H | H | 87-88 | | 0.7 |
| 1357 | 0 | 0 | 2-pyridyl | H | H | H | H | | 28 (6 µM) | |
| 1358 | 0 | 0 | 4-pyridyl | H | H | H | H | | 44 (6 µM) | |
| 1369 | 0 | 0 | 3-pyridyl | H | Me | H | H | (D) | 65 (33 µM) | |
| 1374 | 0 | 0 | 3-pyridyl | H | Me (L) | H | H | | 25 (33 µM) | |
| 1370 | 0 | 0 | Ph | H | Me (L) | H | H | | 0 (9 µM) | |
| 1376 | 0 | 0 | 4-F-Ph | H | Me (L) | H | H | | 12 (8 µM) | |
| 1387 | 0 | 0 | 4-F-Ph | H | Me (D) | H | H | | 23 (8 µM) | |
| 1363 | 0 | 0 | Ph | H | H | H | H | 157-158 | 37 (6 µM) | |
| 1373 | 0 | 0 | 2-F-Ph | H | H | H | H | 133-134 | 90 (8 µM) | |
| 1377 | 0 | 0 | 4-F-2-CF₃-Ph | H | H | H | H | 178-178.5 | 59 (6 µM) | |
| 378 | 0 | 0 | 3-F-Ph | H | H | H | H | 135-136 | 90 (8 µM) | |
| 1379 | 0 | 0 | 4-F-3-CF₃-Ph | H | H | H | H | 182-182.5 | | 0.18 |
| 1383 | 0 | 0 | 3-F-5-CF₃-Ph | H | H | H | H | 189.5-190 | | 0.033 |
| 1384 | 0 | 0 | 3-F-4-CF₃-Ph | H | H | H | H | 205-206 | 77 (7 µM) | 0.49 |
| 1386 | 0 | 0 | 4-F-Ph | Me | H | H | H | 187-188 | 30 (8 µM) | |
| 1388 | 1 | 0 | 4-F-Ph | H | H | H | H | 128-129 | 29 (8 µM) | |
| 1412 | 1 | 0 | 2-F-Ph | H | H | H | H | 121-122 | 17% (16 µM) | |
| 1420 | 1 | 0 | 3-F-Ph | H | H | H | H | 131-132 | 6% (16 µM) | |
| 1392 | 0 | 1 | 4-F-Ph | H | H | H | H | | 54 (8 µM) | |
| 1393 | 0 | 0 | 6-Cl-3-pyridyl | H | H | H | H | 190-191 | 88 (9 µM) | 0.52 |
| 1406 | 0 | 0 | 2-F-3-CF₃-Ph | H | H | H | H | 149-149.5 | | 0.015 |
| 1407 | 0 | 0 | 2-F-5-CF₃-Ph | H | H | H | H | 159-160 | | 0.01 |
| 1413 | 0 | 0 | 3,5-di-CF₃-Ph | H | H | H | H | 201-202 | 3% (12 µM) | |
| 1414 | 0 | 0 | 3-CF₃-Ph | H | H | H | H | 157-158 | 96% (14 µM) | |
| 1421 | 0 | 0 | 2-F-4-CF₃-Ph | H | H | H | H | 141-142 | 97% (14 µM) | |
| 1422 | 0 | 0 | 4-CF₃-Ph | H | H | H | H | 179-180 | 86% (14 µM) | |
| 1423 | 0 | 0 | 4-Me-Ph | H | H | H | H | 136-137 | | 0.1 |

### Example 22

### Inhibition of SSAO enzyme activity by compounds of formula III

Various compounds embraced by formula III (see Example 3I and Example 3J for the identities of these compounds) were evaluated by the radiolabeled benzylamine procedure in Example 4. The results are shown below in.Table II. (Note that for this particular subset of compounds of formula III, the positions of R₁₂ and R₁₃ are para and meta, respectively, to the site of attachment of the remainder of the molecule.) Compound LJP 1368 (Table II) is an irreversible inhibitor of SSAO activity.

**Table II**

| In vitro biological data for compounds of formula III | | | | | | | |
|---|---|---|---|---|---|---|---|
| 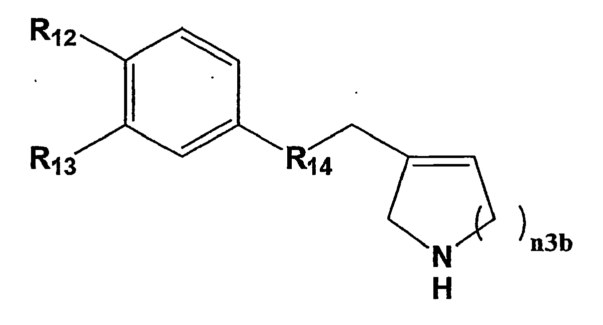 | | | | | | | |

| **LJP #** | **Cmpd # (Example 3I or Example 3J)** | **n3b** | **R₁₄** | **R₁₂** | **R₁₃** | **% inhibition** | **IC₅₀ (**µ**M)** |
|---|---|---|---|---|---|---|---|
| 1367 | III-1 | 1 | CH₂ | H | F | | 2.4 |
| 1402 | III-2 | 1 | CH₂ | F | H | | 1.83 |
| 1368 | III-3 | 1 | CH₂ | H | OMe | | 0.5 |
| 1396 | III-4 | 1 | CH₂ | OMe | H | | 2.1 |
| 1427 | III-5 | 1 | CH2 | OMe | OMe | 50 (9 µM) | |
| 1398 | III-6 | 2 | O | H | F | 61 (10 µM) | |
| 1395 | III-7 | 2 | O | F | H | 56 (10 µM) | |
| 1401 | III-8 | 2 | O | H | OMe | 74 (10 µM) | |
| 1404 | III-9 | 2 | O | OMe | H | 63 (10 µM) | |
| 1397 | III-10 | 2 | O | OMe | OMe | 54 (9 µM) | |

### Example 23

### Selectivity of SSAO inhibitors for SSAO over MAO-A and MAO-B

Using the protocol as outlined in Example 5, data for the inhibition of MAO-A and MAO-B (IC₅₀ values, in micromolar concentration) was generated. The results are shown in Table III. (See Table I for the identities of compounds LJP 1379, LJP 1383, LJP 1406, and LJP 1407; see Table II and Examples 3I and 3J for the identity of compound LJP 1368, which corresponds to compound III-3 in Example 3I).

**Table III**

| Selectivity of LJP Compounds | | |
|---|---|---|
| **LJP#** | **MAO-A (IC₅₀,** µ**M)** | **MAO-B (IC₅₀,** µ**M)** |
| 1368 | >1000 | >1000 |
| 1379 | >1200 | >1200 |
| 1383 | >1300 | >1300 |
| 1406 | >1000 | >1000 |
| 1407 | >1000 | >1000 |

The disclosures of all publications, patents, patent applications and published patent applications referred to herein by an identifying citation are hereby incorporated herein by reference in their entirety.

Although the foregoing invention has been described in some detail by way of illustration and example for purposes of clarity of understanding, it is apparent to those skilled in the art that certain minor changes and modifications will be practiced. Therefore, the description and examples should not be construed as limiting the scope of the invention.

## Claims

1. A compound which is of the following formula: 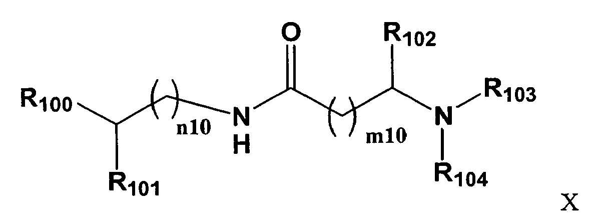 wherein R₁₀₀ is independently chosen from C₆-C₁₀ unsubstituted aryl, C₆-C₁₇ substituted aryl, C₆-C₁₄ aralkyl, C₄-C₉ unsubstituted heteroaryl, and C₄-C₁₅ substituted heteroaryl;
R₁₀₁ is independently chosen from H, -OH, C₁-C₄ alkyl, -O-C₁-C₄ alkyl, C₃-C₈ cycloalkyl, C₇-C₁₄ aralkyl, C₆-C₁₀ aryl, C₆-C₁₇ substituted aryl;
R₁₀₂ is independently chosen from H, F, C₁-C₄ alkyl, C₃-C₈ cycloalkyl, C₇-C₁₄ aralkyl, C₆-C₁₀ aryl, C₆-C₁₇ substituted aryl;
R₁₀₃ and R₁₀₄ are independently chosen from H, C₁-C₄ alkyl, C₃-C₈ cycloalkyl, C₇-C₁₄ aralkyl, C₆-C₁₀ aryl, C₆-C₁₇ substituted aryl;
n10 is independently chosen from 0 and 1; and
m10 is independently chosen from 0 and 1;
or is a stereoisomer, E/Z isomer, solvate, hydrate or salt thereof.

2. The compound of claim 1, wherein R₁₀₀ is independently phenyl, 4-Me-phenyl, 2-F-phenyl, 3-F-phenyl, 4-F-phenyl, 3-CF₃-phenyl, 4-CF₃-phenyl, 2-F-3-CF₃-phenyl, 2-F-4-CF₃-phenyl, 2-F-5-CF₃-phenyl, 3,5-di-CF₃-phenyl, 3-F-4-CF₃-phenyl, 3-F-5-CF₃-phenyl, 4-F-2-CF₃-phenyl, 4-F-3-CF₃-phenyl, 2-pyridyl, 3-pyridyl, 4-pyridyl, or 6-Cl-3-pyridyl.

3. The compound of claim 1 or 2, wherein R₁₀₁ is independently H, -OH, or C₁-C₄ alkyl.

4. The compound of any one of claims 1 to 3, wherein R₁₀₂ is independently H, F, or C₁-C₄ alkyl.

5. The compound of any one of the preceding claims, wherein R₁₀₃ is independently H, methyl, ethyl, n-propyl, or isopropyl, benzyl, unsubstituted phenyl, 4-fluorophenyl, or 4-methylphenyl.

6. The compound of any one of the preceding claims, wherein R₁₀₄ is independently H, methyl, ethyl, n-propyl, or isopropyl, benzyl, unsubstituted phenyl, 4-fluorophenyl, or 4-methylphenyl.

7. The compound of claim 1, which is a compound as identified in Table 1.

8. A composition comprising a compound as claimed in any one of the preceding claims and a pharmaceutically acceptable carrier.

9. A compound as claimed in any one of claims 1 to 7 or composition as claimed in claim 8 for use in a method of treatment of the human or animal body.

10. A compound as claimed in any one of claims 1 to 7 or composition as claimed in claim 8 for use in a method of treatment of inflammation, a disease caused by inflammation, or a disease which causes inflammation.

11. A compound as claimed in any one of claims 1 to 7 or composition as claimed in claim 8 for use in treating an immune or autoimmune disorder.

12. A compound as claimed in any one of claims 1 to 7 or composition as claimed in claim 8 for use in treating multiple sclerosis.

13. A compound as claimed in any one of claims 1 to 7 or composition as claimed in claim 8, for use in a method of treatment of an ischaemic disease or the sequelae of an ischaemic disease.

14. An *in vitro* method of inhibiting semicarbazide-sensitive amine oxidase (SSAO) activity or inhibiting binding to vascular adhesion protein-1 (VAP-1) by supplying a compound as claimed in any one of claims 1 to 7 to an *in vitro* environment in an amount sufficient to inhibit SSAO activity or inhibit binding to VAP-1.

15. Use of a compound as claimed in any one of claims 1 to 7 or a composition as claimed in claim 8 in the manufacture of a medicament for use in the treatment of inflammation, a disease caused by inflammation or a disease which causes inflammation, an immune or autoimmune disorder, multiple sclerosis, an ischaemic disease or the sequalae of an ischaemic disease.
